# EUROPEAN PATENT APPLICATION

(11) **EP 4 775 578 A1**
(43) Date of publication of application: **15.07.2026**
(21) Application number: 24861977.7
(22) Date of filing: 04.09.2024
(51) Int. Cl.: C07D 455/03, A61K 31/4375, A61P 25/28, A61P 25/30, A61P 25/24, A61P 25/20

(54) **TETRAHYDROISOQUINOLINE DERIVATIVE AND USE THEREOF**

(30) Priority: 04.09.2023 CN 202311129751; 28.08.2024 CN 202411195283
(71) Applicant: Beijing Tide Pharmaceutical Co., Ltd., Beijing 100176 (CN)
(72) Inventor: LI, Wenming, Beijing 100176 (CN); LI, Xiaobo, Beijing 100176 (CN); GUO, Guangzhu, Beijing 100176 (CN); YU, Xiaolei, Beijing 100176 (CN); PENG, Lijiao, Beijing 100176 (CN); LI, Xinchun, Beijing 100176 (CN); LI, Gege, Beijing 100176 (CN); PEI, Xiaolin, Beijing 100176 (CN); ZHOU, Liying, Beijing 100176 (CN); GAN, Leling, Beijing 100176 (CN); LI, Eryao, Beijing 100176 (CN); WANG, Jingying, Beijing 100176 (CN)
(74) Representative: Novitas Patent AB
(86) International application number: PCT/CN2024/116714
(87) International publication number: WO 2025/051134

(57) **Abstract**

The present invention provides a tetrahydroisoquinoline derivative and a use thereof. Specifically, the present invention relates to a compound represented by formula (I), or an isotopic variant, tautomer, stereoisomer, prodrug, polymorph, hydrate or solvate thereof, or a pharmaceutically acceptable salt thereof. The present invention also provides a pharmaceutical composition comprising the compound, and an effect of the compound in prevention and treatment of diseases related to the central nervous system.

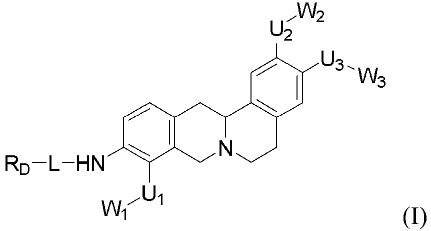

## Description

The present application claims priority to Chinese Application 202311129751.9 filed on September 04, 2023, and Chinese application 202411195283.X filed on August 28, 2024, which are incorporated herein by reference in their entirety.

### FIELD OF THE INVENTION

The present disclosure relates to a class of tetrahydroisoquinoline derivatives, or isotopic variants, tautomers, stereoisomers, prodrugs, polymorphs, hydrates or solvates thereof, or pharmaceutically acceptable salts thereof, and uses thereof. The present disclosure also relates to pharmaceutical compositions comprising said tetrahydroisoquinoline derivatives, and the use of said tetrahydroisoquinoline derivatives in the prevention and treatment of central nervous system related diseases such as pain, depression and addiction.

### BACKGROUND OF THE INVENTION

1-orydalmine (1-DL) is an isoquinoline alkaloid with tetracyclic structure, which has analgesic, detoxification, and anti-drug addiction effects. Its chemical name is (S)-2,3,9-trimethoxy-5,8,13,13a-tetrahydro-6H-isoquinolino[3,2-a]isoquinolin-10-ol, and it has the following structure:

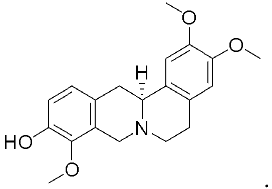

Corydalmine, as one of the active alkaloid components in the traditional Chinese medicine *Corydalis yanhusuo,* has an extremely low natural content, which limits its application in the pharmaceutical field. Therefore, there is an urgent need to research other active compounds based on the structure of corydalmine.

### SUMMARY OF THE INVENTION

Based on the structure of corydalmine, the present disclosure has developed a series of active compounds that exhibit excellent analgesic effects.

In one aspect, the present disclosure provides a compound of formula (I), or an isotopic variant, a tautomer, a stereoisomer, a prodrug, a polymorph, a hydrate or a solvate thereof, or a pharmaceutically acceptable salt thereof: 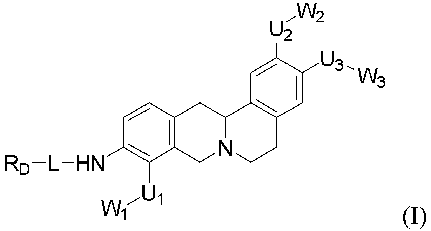 wherein the variables are as defined herein.

In another aspect, the present disclosure provides a pharmaceutical composition comprising a compound of the present disclosure, and a pharmaceutically acceptable carrier, adjuvant or vehicle, optionally other therapeutic agent(s).

In another aspect, the present disclosure provides the use of a compound of the present disclosure in the manufacture of a medicament for use in the treatment or prevention of a central nervous system related disease.

In another aspect, the present disclosure provides a method of treating or preventing a central nervous system related disease in a subject, comprising administering to the subject a compound of the present disclosure or a pharmaceutical composition of the present disclosure.

In another aspect, the present disclosure provides a compound of the present disclosure or a pharmaceutical composition of the present disclosure, for use in the treatment or prevention of a central nervous system related disease.

In a specific embodiment, the disease is selected from the group consisting of pain, depression, and addiction.

In another specific embodiment, the pain is selected from the group consisting of neuralgia, perioperative pain (including preoperative, intraoperative or post-operative pain, such as somatic or visceral pain caused by postoperative trauma or surgical incisions, pain caused by visceral injury, and other comprehensive types of pain), and cancer pain (such as pain caused by cancer).

In another specific embodiment, the pain is acute pain or chronic pain (including acute/chronic pain before, during, or after surgery, acute neuralgia, or chronic neuralgia).

In another specific embodiment, the neuralgia is central pain, such as spinal pain, thalamic pain, pontine pain, medullary pain, or cerebral cortical pain.

In another specific embodiment, the postoperative pain is pain caused by surgery, such as pain resulting from abdominal surgery, orthopedic surgery, cesarean section, or brain surgery.

In another specific embodiment, the compound or pharmaceutical composition is used for sedation or sleep aid.

### Definitions

### Chemical definitions

Definitions of specific functional groups and chemical terms are described in detail below.

When a range of values is given, it is intended to include each value and every subrange within the range. For example, "C₁₋₆ alkyl" includes C₁, C₂, C₃, C₄, C₅, C₆, C₁₋₆, C₁₋₅, C₁₋₄, C_{1-3,} C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₁₈ alkyl" refers to a linear or branched saturated hydrocarbon group having 1 to 18 carbon atoms. In some embodiments, C₁₋₁₀ alkyl is alternative. In some embodiments, C₁₋₆ alkyl is alternative. In some embodiments, C₁₋₄ alkyl, C₁₋₃ alkyl and C₁₋₂ alkyl are alternative. Examples of C₁₋₆ alkyl include: methyl (C₁), ethyl (C₂), *n*-propyl (C₃), isopropyl (C₃), *n*-butyl (C₄), *tert*-butyl (C₄), *sec*-butyl (C₄), isobutyl (C₄), *n*-pentyl (C₅), 3-pentyl (C₅), pentyl (C₅), neopentyl (C₅), 3-methyl-2-butyl (C₅), *tert*-pentyl (C₅), and *n*-hexyl (C₆). The term "C₁₋₆ alkyl" also includes heteroalkyl in which one or more (e.g., 1, 2, 3, or 4) carbon atoms are replaced by a heteroatom (e.g., oxygen, sulfur, nitrogen, boron, silicon, or phosphorus). The alkyl group may be optionally substituted with one or more substituents, e.g., 1 to 5 substituents, 1 to 3 substituents, or 1 substituent. Conventional abbreviations of alkyl include: Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂), or t-Bu(-C(CH₃)₃).

"C₂₋₆ alkenyl" refers to a linear or branched hydrocarbon group having 2 to 6 carbon atoms and at least one carbon-carbon double bond. In some embodiments, C₂₋₄ alkenyl is alternative. Examples of C₂₋₆ alkenyl include: vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), and the like. The term "C₂₋₆ alkenyl" also includes heteroalkenyl in which one or more (e.g., 1, 2, 3, or 4) carbon atoms are replaced by a heteroatom (e.g., oxygen, sulfur, nitrogen, boron, silicon, or phosphorus). The alkenyl group may be optionally substituted with one or more substituents, e.g., 1 to 5 substituents, 1 to 3 substituents, or 1 substituent.

"C₂₋₆ alkynyl" refers to a linear or branched hydrocarbon group having 2 to 6 carbon atoms, at least one carbon-carbon triple bond, and optionally one or more carbon-carbon double bonds. In some embodiments, C₂₋₄ alkynyl is alternative. Examples of C₂₋₆ alkynyl include, but are not limited to: ethynyl (C₂), 1-propynyl (C₃), 2-propynyl (C₃), 1-butynyl (C₄), 2-butynyl (C₄), pentynyl (C₅), hexynyl (C₆), and the like. The term "C₂₋₆ alkynyl" also includes heteroalkynyl in which one or more (e.g., 1, 2, 3, or 4) carbon atoms are replaced by a heteroatom (e.g., oxygen, sulfur, nitrogen, boron, silicon, or phosphorus). The alkynyl group may be optionally substituted with one or more substituents, e.g., 1 to 5 substituents, 1 to 3 substituents, or 1 substituent.

"C₁₋₁₀ alkylene" refers to a divalent group formed by removing another hydrogen from a C₁₋₁₀ alkyl group, which may be substituted or unsubstituted. In some embodiments, C₁₋₆ alkylene is alternative. In some embodiments, C₁₋₄ alkylene, C₂₋₄ alkylene, C₁₋₃ alkylene, and C₁₋₂ alkylene are alternative. Unsubstituted alkylene includes, but is not limited to: methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Examples of substituted alkylene, for example, an alkylene substituted with one or more alkyl (methyl) groups, include, but are not limited to: substituted methylene (-CH(CH₃)- and - C(CH₃)₂-), substituted ethylene (-CH(CH₃)CH₂-, -CH₂CH(CH₃)-, -C(CH₃)₂CH₂-, and - CH₂C(CH₃)₂-), substituted propylene (-CH(CH₃)CH₂CH₂-, -CH₂CH(CH₃)CH₂-, - CH₂CH₂CH(CH₃)-, -C(CH₃)₂CH₂CH₂-, -CH₂C(CH₃)₂CH₂-, and -CH₂CH₂C(CH₃)₂-), and the like.

"C₂₋₆ alkenylene" refers to a divalent group formed by removing another hydrogen from a C₂₋₁₃ alkenyl group, which may be substituted or unsubstituted. In some embodiments, C₂₋₄ alkenylene is particularly alternative. Examples of unsubstituted alkenylene include, but are not limited to: vinylene (-CH=CH-) and propenylene (e.g., -CH=CHCH₂- and -CH₂-CH=CH-). Examples of substituted alkenylene, for example, an alkenylene substituted with one or more alkyl (methyl) groups, include, but are not limited to: substituted vinylene (-C(CH₃)=CH- and -CH=C(CH₃)-), substituted propenylene (-C(CH₃)=CHCH₂-, - CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, and -CH₂-CH=C(CH₃)-), and the like.

"C₂₋₆ alkynylene" refers to a divalent group formed by removing another hydrogen from a C₂₋₆ alkynyl group, which may be substituted or unsubstituted. In some embodiments, C₂₋₄ alkynylene is particularly alternative. Examples of alkynylene include, but are not limited to: ethynylene (-C≡C-), substituted or unsubstituted propynylene (-C≡CCH₂-), and the like.

"C₀₋₁₀ alkylene" refers to a chemical bond and the above-mentioned "C₁₋₁₀ alkylene". In some embodiments, "C₀₋₆ alkylene" is alternative. In some embodiments, C₀₋₄ alkylene is alternative. In some embodiments, C₀₋₃ alkylene is alternative. In some embodiments, C₀₋₂ alkyleneis alternative.

"C₁₋₁₀ deuterated alkyl" refers to the aforementioned "C₁₋₁₀ alkyl" group, which is substituted with one or more D (deuterium) groups. In some embodiments, C₁₋₆ deuterated alkyl is particularly alternative, yet alternatively C₁₋₄ deuterated alkyl, even yet alternatively C₁₋₃ deuterated alkyl, and most alternatively C₁₋₂ deuterated alkyl. Exemplary said deuterated alkyl groups include, but are not limited to: -CD₃, -CH₂D, -CHD₂, -CHDCH₂D, -CH₂CHD₂, - CD₂CD₃, -CH₂CH₂CD₃, -C(CH₃)₂CD₃, and the like. A deuterated alkyl group may be substituted at any available connection site with, for example, 1 to 5 substituents, 1 to 3 substituents, or 1 substituent.

"Halo" or "halogen" refers to fluorine (F), chlorine (Cl), bromine (Br), and iodine (I).

Thus, "C₁₋₁₈ haloalkyl" refers to the "C₁₋₁₈ alkyl" described above substituted with one or more halogen groups. In some embodiments, C₁₋₁₀ haloalkyl is particularly alternative, yet alternatively C₁₋₆ haloalkyl, yet alternatively C₁₋₄ haloalkyl, yet alternatively C₁₋₃ haloalkyl, yet alternatively C₁₋₂ haloalkyl. Examples of haloalkyl include, but are not limited to: -CF₃, -CH₂F, -CHF₂, -CHFCH₂F, -CH₂CHF₂, -CF₂CF₃, -CH₂CH₂CF₃, -C(CH₃)₂CF₃, -CCl₃, -CH₂Cl, -CHCl₂, 2,2,2-trifluoro-1,1-dimethyl-ethyl, and the like. The haloalkyl group may be substituted at any available connection site with, for example, 1 to 5 substituents, 1 to 3 substituents, or 1 substituent.

"C₃₋₁₄ cycloalkyl" refers to a non-aromatic cyclic hydrocarbon group having 3 to 14 ring carbon atoms and no heteroatoms and optionally containing 1, 2, or 3 double or triple bonds. In some embodiments, C₃₋₁₀ cycloalkyl, C₅₋₁₀ cycloalkyl, C₃₋₇ cycloalkyl and C₃₋₆ cycloalkyl are particularly alternative; C₅₋₇ cycloalkyl, C₄₋₆ cycloalkyl and C₅₋₆ cycloalkyl are more alternative. The cycloalkyl also includes ring systems in which the cycloalkyl ring described above is fused to one or more aryl or heteroaryl groups, where the connection site is on the cycloalkyl ring, and in such cases, the number of carbon still represents the number of carbon in the cycloalkyl system. The cycloalkyl also includes those in which substituents on any nonadjacent carbon atoms of the cycloalkyl ring described above are joined to form a bridged ring, together forming polycyclic alkane sharing two or more carbon atoms. The cycloalkyl also includes those in which substituents on the same carbon atom of the cycloalkyl ring described above are joined to form a ring, together forming polycyclic alkane sharing one carbon atom. Examples of cycloalkyl include, but are not limited to: cyclopropyl (C₃), **cyclopropenyl** (C₃), cyclobutyl (C₄), cyclobutenyl (C₄), cyclopentyl (C₅), cyclopentenyl (C₅), 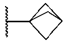 (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), and the like. The cycloalkyl group may be optionally substituted with one or more substituents, e.g., 1 to 5 substituents, 1 to 3 substituents, or 1 substituent.

"C₃₋₇ cycloalkylene" refers to a divalent group formed by removing another hydrogen from a C₃₋₇ cycloalkyl group, which may be substituted or unsubstituted. In some embodiments, C₃₋₆ cycloalkylene, C₃₋₅ cycloalkylene, and C₃₋₄ cycloalkylene are particularly alternative, alternatively cyclopentylene, yet alternatively, cyclopropylene.

"3- to 14-membered heterocyclyl" refers to a saturated or unsaturated group of a 3- to 14-membered non-aromatic ring system having ring carbon atoms and 1 to 5 ring heteroatoms, wherein each heteroatom is independently selected from nitrogen, oxygen, sulfur, boron, phosphorus, and silicon, and the group optionally contains 1, 2, or 3 double or triple bonds. In heterocyclyl containing one or more nitrogen atoms, the connection site may be a carbon or nitrogen atom, as long as the valency permits. In some embodiments, 3- to 10-membered heterocyclyl, which is a 3- to 10-membered non-aromatic ring system having ring carbon atoms and 1 to 5 ring heteroatoms, is alternative; in some embodiments, 5- to 10-membered heterocyclyl, which is a 5- to 10-membered non-aromatic ring system having ring carbon atoms and 1 to 5 ring heteroatoms, is alternative; in some embodiments, 3- to 7-membered heterocyclyl, which is a 3- to 7-membered non-aromatic ring system having ring carbon atoms and 1 to 4 ring heteroatoms, is alternative; 5- to 7-membered heterocyclyl, which is a 5- to 7-membered non-aromatic ring system having ring carbon atoms and 1 to 3 ring heteroatoms, is alternative; 3- to 6-membered heterocyclyl, which is a 3- to 6-membered non-aromatic ring system having ring carbon atoms and 1 to 3 ring heteroatoms, is alternative; 4- to 6-membered heterocyclyl, which is a 4- to 6-membered non-aromatic ring system having ring carbon atoms and 1 to 3 ring heteroatoms, is alternative; 5- to 6-membered heterocyclyl, which is a 5- to 6-membered non-aromatic ring system having ring carbon atoms and 1 to 3 ring heteroatoms, is more alternative; 5-membered heterocyclyl is more alternative. The heterocyclyl also includes ring systems in which the heterocyclyl ring described above is fused to one or more cycloalkyl groups, where the connection site is on the heterocyclyl ring, or ring systems in which the heterocyclyl ring described above is fused to one or more aryl or heteroaryl groups, where the connection site is on the heterocyclyl ring; in such cases, the number of ring members still represents the number of ring members in the heterocyclyl ring system. The heterocyclyl also includes those in which substituents on any nonadjacent carbon or nitrogen atoms of the heterocyclyl ring described above are joined to form a bridged ring, together forming polycyclic heteroalkane sharing two or more carbon or nitrogen atoms. The heterocyclyl also includes those in which substituents on the same carbon atom of the heterocyclyl ring described above are joined to form a ring, together forming polycyclic heteroalkane sharing one carbon atom. Examples of 3-membered heterocyclyl containing one heteroatom include, but are not limited to: aziridinyl, oxiranyl, and thiorenyl. Examples of 4-membered heterocyclyl containing one heteroatom include, but are not limited to: azetidinyl, oxetanyl, and thietanyl. Examples of 5-membered heterocyclyl containing one heteroatom include, but are not limited to: tetrahydrofuranyl, dihydrofuranyl, tetrahydrothienyl, dihydrothienyl, pyrrolidinyl, dihydropyrrolyl, and pyrrolyl-2,5-dione. Examples of 5-membered heterocyclyl containing two heteroatoms include, but are not limited to: pyrazolidinyl, dioxolanyl, oxasulfuranyl, disulfuranyl, and oxazolidin-2-one. Examples of 5-membered heterocyclyl containing three heteroatoms include, but are not limited to: triazolinyl, oxadiazolinyl, and thiadiazolinyl. Examples of 6-membered heterocyclyl containing one heteroatom include, but are not limited to: piperidinyl, dihydropyranyl, tetrahydropyranyl, dihydropyridinyl, and thianyl. Examples of 6-membered heterocyclyl containing two heteroatoms include, but are not limited to: piperazinyl, morpholinyl, dithianyl, and dioxanyl. Examples of 6-membered heterocyclyl containing three heteroatoms include, but are not limited to: triazinanyl. Examples of 7-membered heterocyclyl containing one heteroatom include, but are not limited to: azepanyl, oxepanyl, and thiepanyl. Examples of 5-membered heterocyclyl fused to a C₆ aryl ring (also referred to herein as 5,6-bicyclic heterocyclyl) include, but are not limited to: indolinyl, isoindolinyl, dihydrobenzofuranyl, dihydrobenzothienyl, benzoxazolinonyl, and the like. Examples of 6-membered heterocyclyl fused to a C₆ aryl ring (also referred to herein as 6,6-bicyclic heterocyclyl) include, but are not limited to: tetrahydroquinolinyl, tetrahydroisoquinolinyl, and the like. The heterocyclyl also includes those in which the heterocyclyl described above shares one or two atoms with a cycloalkyl, heterocyclyl, aryl, or heteroaryl group to form bridged or spiro rings, where the shared atoms may be carbon or nitrogen atoms, as long as the valency permits. The heterocyclyl also includes those in which the heterocyclyl and heterocyclyl groups described above may be optionally substituted with one or more substituents, e.g., 1 to 5 substituents, 1 to 3 substituents, or 1 substituent.

"C₆₋₁₄ aryl" refers to a group of a monocyclic or polycyclic (e.g., tricyclic) 4n+2 aromatic ring system (e.g., having 6 or 14 π electrons shared in a cyclic arrangement) having 6-14 ring carbon atoms and no heteroatoms. In some embodiments, C₆₋₁₀ aryl is alternative. In some embodiments, the aryl has six ring carbon atoms ("C₆ aryl"; e.g., phenyl). In some embodiments, the aryl has ten ring carbon atoms ("C₁₀ aryl"; such as naphthyl, e.g., 1-naphthyl and 2-naphthyl). The aryl also includes ring systems in which the aryl ring described above is fused to one or more cycloalkyl or heterocyclyl groups, and the connection site is on the aryl ring; in such cases, the number of carbon atoms still represents the number of carbon atoms in the aryl ring system. The aryl group may be optionally substituted with one or more substituents, e.g., 1 to 5 substituents, 1 to 3 substituents, or 1 substituent.

"5- to 14-membered heteroaryl" refers to a group of a 5- to 14-membered monocyclic or bicyclic 4n+2 aromatic ring system (e.g., having 6, 10, or 14 π electrons shared in a cyclic arrangement) having ring carbon atoms and 1-4 ring heteroatoms, wherein each heteroatom is independently selected from nitrogen, oxygen, and sulfur. In heteroaryl containing one or more nitrogen atoms, the connection site may be a carbon or nitrogen atom, as long as the valency permits. The heteroaryl bicyclic system may contain one or more heteroatoms in one or both rings. The heteroaryl also includes ring systems in which the heteroaryl ring described above is fused to one or more cycloalkyl or heterocyclyl groups, and the connection site is on the heteroaryl ring; in such cases, the number of carbon atoms still represents the number of carbon atoms in the heteroaryl ring system. In some embodiments, 5- to 10-membered heteroaryl, which is a 5- to 10-membered monocyclic or bicyclic 4n+2 aromatic ring system having ring carbon atoms and 1-4 ring heteroatoms, is alternative. In some other embodiments, 5- to 6-membered heteroaryl, which is a 5- to 6-membered monocyclic or bicyclic 4n+2 aromatic ring system having ring carbon atoms and 1-4 ring heteroatoms, is particularly alternative. In some other embodiments, 5-membered heteroaryl is particularly alternative. In some other embodiments, 6-membered heteroaryl is particularly alternative. Examples of 5-membered heteroaryl containing one heteroatom include, but are not limited to: pyrrolyl, furanyl, and thienyl. Examples of 5-membered heteroaryl containing two heteroatoms include, but are not limited to: imidazolyl, pyrazolyl, oxazolyl, isoxazolyl, thiazolyl, and isothiazolyl. Examples of 5-membered heteroaryl containing three heteroatoms include, but are not limited to: triazolyl, oxadiazolyl (e.g., 1,2,4-oxadiazolyl), and thiadiazolyl. Examples of 5-membered heteroaryl containing four heteroatoms include, but are not limited to: tetrazolyl. Examples of 6-membered heteroaryl containing one heteroatom include, but are not limited to: pyridinyl or pyridonyl. Examples of 6-membered heteroaryl containing two heteroatoms include, but are not limited to: pyridazinyl, pyrimidinyl, and pyrazinyl. Examples of 6-membered heteroaryl containing three or four heteroatoms include, but are not limited to: triazinyl and tetrazinyl. Examples of 7-membered heteroaryl containing one heteroatom include, but are not limited to: azepinyl, oxepinyl, and thiepinyl. Examples of 5,6-bicyclic heteroaryl include, but are not limited to: indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indolizinyl, and purinyl. Examples of 6,6-bicyclic heteroaryl include, but are not limited to: naphthyridinyl, pteridinyl, quinolinyl, isoquinolinyl, cinnolinyl, quinoxalinyl, phthalazinyl, and quinazolinyl. The heteroaryl group may be optionally substituted with one or more substituents, e.g., 1 to 5 substituents, 1 to 3 substituents, or 1 substituent.

"C₆₋₁₀ arylene" refers to a divalent group formed by removing another hydrogen atom from a C₆₋₁₀ aryl group, which may be substituted or unsubstituted. In some embodiments, phenylene is particularly alternative, alternatively 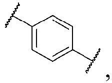 alternatively

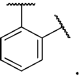

"5- to 10-membered heteroarylene" refers to a divalent group formed by removing another hydrogen atom from a 5- to 10-membered heteroaryl, which may be substituted or unsubstituted. In some embodiments, 9- to 10-membered heteroarylene is particularly alternative, alternatively, indolylene, alternatively, 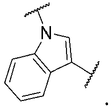 In some embodiments, 5- to 6-membered heteroarylene is particularly alternative.

Divalent groups formed by removing another hydrogen from the above-defined alkyl, alkenyl, alkynyl, cycloalkyl, heterocyclyl, aryl, and heteroaryl groups are collectively referred to as "-ylene". Cyclic groups such as cycloalkyl, heterocyclyl, aryl, and heteroaryl are collectively referred to as "cyclyl".

The alkyl, alkenyl, alkynyl, cycloalkyl, heterocyclyl, aryl, heteroaryl, and the like as defined herein are optionally substituted groups.

Examples of substituents on carbon atoms include, but are not limited to: halogen, - CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, - SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, - C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, - C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, - OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, - SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, - P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, - NR^{bb}P(=O)(NR^{bb})₂, -P(R^{cc})₂, -P(R^{cc})₃, -OP(R^{cc})₂, -OP(R^{cc})_{3,} -B(R^{aa})₂, -B(OR^{cc})₂, -BR^{aa}(OR^{cc}), alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl, and heteroaryl is independently substituted with 0, 1, 2, 3, 4, or 5 R^{dd} groups;
or two geminal hydrogen atoms on a carbon atom are substituted with group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each R^{aa} is independently selected from alkyl, haloalkyl, alkenyl, alkynyl, cycloalkyl, heterocyclyl, aryl, and heteroaryl, or two R^{aa} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{bb} is independently selected from: hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, - P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{bb} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{cc} is independently selected from hydrogen, alkyl, haloalkyl, alkenyl, alkynyl, cycloalkyl, heterocyclyl, aryl, and heteroaryl, or two R^{cc} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{dd} is independently selected from: halogen, -CN, -NO₂, -N₃, -SO₂H, -SO₃H, - OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, - CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, - NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, - C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, - SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl, and heteroaryl is independently substituted with 0, 1, 2, 3, 4, or 5 R^{gg} groups, or two geminal R^{dd} substituents may be joined to form =O or =S;
each R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl, and heteroaryl is independently substituted with 0, 1, 2, 3, 4, or 5 R^{gg} groups;
each R^{ff} is independently selected from hydrogen, alkyl, haloalkyl, alkenyl, alkynyl, cycloalkyl, heterocyclyl, aryl, and heteroaryl, or two R^{ff} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{gg} groups;
each R^{gg} is independently: halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, - NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, - SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, - C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, - OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, 3- to 7-membered heterocyclyl, or 5- to 10-membered heteroaryl; or two geminal R^{gg} substituents may be joined to form =O or =S; wherein X⁻ is a counter ion.

Examples of substituents on nitrogen atoms include, but are not limited to: hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, - C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{cc} groups connected to a nitrogen atom are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as described above.

### Other definitions

The term "anti-inflammatory agent" refers to a pharmaceutical molecule that possesses activity in preventing or treating inflammation, capable of inhibiting the production or release of inflammatory factors, thereby alleviating inflammation and pain. Common anti-inflammatory agents fall into two main categories: one is steroidal anti-inflammatory agents, which can be further divided into sex hormones and corticosteroids, regulating glucose metabolism, water and salt metabolism, exerting anti-inflammatory and anti-allergic effects, and improving metabolic functions. Common steroidal anti-inflammatory agents include dexamethasone and methylprednisolone. The other category is non-steroidal anti-inflammatory agents (NSAIDs), such as loxoprofen, flurbiprofen, fenoprofen, ketoprofen, tolmetin, bromofenic acid, tiaprofenic acid, indomethacin, sulindac, ketorolac, nimesulide, mefenamic acid, clofenamic acid, diclofenac, aspirin, ibuprofen, naproxen, nabumetone, etodolac, rofecoxib, celecoxib, piroxicam, meloxicam, and oxyphenbutazone, among others.

The term "derivative" refers to a compound formed by substituting an atom or an atomic group in a molecule with another atom or atomic group.

The term "central nervous system related disease" includes, but is not limited to, pain, depression, and addiction.

The term "pain" includes, but is not limited to, acute pain and chronic pain.

In one embodiment, the term "pain" includes, but is not limited to the following pains: neuralgia, perioperative pain (including preoperative, intraoperative or post-operative pain, such as somatic or visceral pain caused by postoperative trauma or surgical incisions, pain caused by visceral injury, and other comprehensive types of pain), and cancer pain (such as pain caused by cancer).

In one embodiment, the term "pain" is acute pain or chronic pain (including acute/chronic pain before, during, or after surgery, acute neuralgia, or chronic neuralgia).

In one embodiment, the term "neuralgia" is central pain, such as spinal pain, thalamic pain, pontine pain, medullary pain, or cerebral cortical pain.
the term "postoperative pain" is pain caused by surgery, such as pain resulting from abdominal surgery, orthopedic surgery, cesarean section, or brain surgery or other surgery.

The term "treat", "treating", or "treatment" as used herein relates to reversing, alleviating, or inhibiting the progression of a disorder or condition to which the term applies, or one or more symptoms of such a disorder or condition, or preventing the disorder or condition. The noun form "treatment" as used herein relates to the action of the verb treat, as just defined above.

The term "pharmaceutically acceptable salt" as used herein refers to those carboxylates and amino acid addition salts of the compound of the present disclosure, which are, within the scope of sound medical judgment, suitable for use in contact with the tissues of patients without undue toxicity, irritation, allergic response, and the like, and are commensurate with a reasonable benefit/risk ratio and effective for their intended use, including the zwitterionic forms, where possible, of the compound of the present disclosure.

Pharmaceutically acceptable base addition salts are formed with metals or amines, such as alkali metal and alkaline earth metal hydroxides or organic amines. Examples of metals used as cations are sodium, potassium, magnesium, calcium, and the like. Examples of suitable amines are *N,N*'-dibenzylethylenediamine, chloroprocaine, choline, diethanolamine, ethylenediamine, N-methylglucamine, and procaine.

Base addition salts of acidic compounds may be prepared by contacting the free acid form with a sufficient amount of a desired base to form the salt in a conventional manner. The free acid may be regenerated by contacting the salt form with an acid and separating the free acid in a conventional manner. The free acid forms differ somewhat from their respective salt forms in certain physical properties, such as solubility in polar solvents, but the salts are equivalent to their respective free acids for the purposes of the present disclosure.

The salt may be sulfate, pyrosulfate, bisulfate, sulfite, bisulfite, nitrate, phosphate, monohydrogenphosphate, dihydrogenphosphate, metaphosphate, pyrophosphate, chloride, bromide, and iodide prepared from inorganic acids such as hydrochloric acid, nitric acid, sulfuric acid, hydrobromic acid, hydroiodic acid, phosphoric acid, and the like. Representative salts include: hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthoate, mesylate, glucoheptonate, lactobionate, laurylsulfonate, isethionate, and the like. The salts may also be prepared from organic acids such as aliphatic mono- and dicarboxylic acids, phenyl-substituted alkanoic acids, hydroxyalkanoic acids, alkanedioic acids, aromatic acids, aliphatic and aromatic sulfonic acids, and the like. Representative salts include acetate, propionate, octanoate, isobutyrate, oxalate, malonate, succinate, suberate, sebacate, fumarate, maleate, mandelate, benzoate, chlorobenzoate, methylbenzoate, dinitrobenzoate, naphthoate, benzenesulfonate, tosylate, phenylacetate, citrate, lactate, maleate, tartrate, mesylate, and the like. The pharmaceutically acceptable salts may include cations based on the alkali metals and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. Also encompassed are salts of amino acids such as arginate, gluconate, galacturonate, and the like (see, e.g., Berge S. M. et al., "Pharmaceutical Salts", J. Pharm. Sci., 1977; 66:1-19, which is incorporated herein by reference).

The "subject" to which the compound is administered includes, but is not limited to: a human (i.e., a male or female of any age group, e.g., a pediatric subject (e.g., an infant, a child, or an adolescent) or an adult subject (e.g., a young adult, a middle-aged adult, or an older adult)) and/or a non-human animal, e.g., a mammal, such as a primate (e.g., a cynomolgus monkey or a rhesus monkey), a cow, a pig, a horse, a sheep, a goat, a rodent, a cat, and/or a dog. In some embodiments, the subject is a human. In some embodiments, the subject is a non-human animal. The terms "human", "patient", and "subject" are used interchangeably herein.

The "disease", "disorder", and "condition" are used interchangeably herein.

Unless otherwise indicated, the term "treat" used herein includes effects that, when a subject suffers from a particular disease, disorder, or condition, reduce the severity of the disease, disorder, or condition, or delay or slow the progression of the disease, disorder, or condition ("therapeutic treatment"), and also includes effects that occur before the subject begins to suffer from a particular disease, disorder, or condition ("prophylactic treatment").

Generally, the "effective amount" of the compound refers to an amount sufficient to elicit a biological response of interest. It will be appreciated by those of ordinary skill in the art that, the effective amount of the compound of the present disclosure may vary depending on the following factors: for example, biological objectives, pharmacokinetics of the compound, the disease to be treated, the mode of administration, and the age, health, and symptom of the subject. The effective amount includes a therapeutically effective amount and a prophylactically effective amount. In some embodiments of the present disclosure, the effective dose, calculated based on the compound, is from 0.1 mg/day to 1000 mg/day, alternatively from 3 mg/day to 300 mg/day, and yet alternatively from 5 mg/day to 50 mg/day.

Unless otherwise indicated, the "therapeutically effective amount" of a compound used herein is an amount sufficient to provide a therapeutic benefit in the treatment of a disease, disorder, or condition, or an amount to delay or minimize one or more symptoms associated with a disease, disorder, or condition. The therapeutically effective amount of a compound refers to an amount of a therapeutic agent that, alone or in combination with other therapies, provides a therapeutic benefit in the treatment of a disease, disorder, or condition. The term "therapeutically effective amount" may include an amount that improves the overall treatment, reduces or avoids symptoms or causes of a disease or condition, or enhances the therapeutic effect of other therapeutic agents.

Unless otherwise indicated, the "prophylactically effective amount" of a compound used herein is an amount sufficient to prevent a disease, disorder, or condition, an amount sufficient to prevent one or more symptoms associated with a disease, disorder, or condition, or an amount to prevent the recurrence of a disease, disorder, or condition. The prophylactically effective amount of a compound refers to an amount of a therapeutic agent that, alone or in combination with other agents, provides a prophylactic benefit in the prevention of a disease, disorder, or condition. The term "prophylactically effective amount" may include an amount that improves the overall prophylaxis, or an amount to enhance the prophylactic effect of other prophylactic agents.

The "combination" and related terms refer to the simultaneous or sequential administration of the compound of the present disclosure and an additional therapeutic agent. For example, the compound of the present disclosure may be administered simultaneously or sequentially with the additional therapeutic agent in separate unit dosage forms, or simultaneously with the additional therapeutic agent in a single unit dosage form.

### BRIEF DESCRIPTION OF THE DRAWINGS

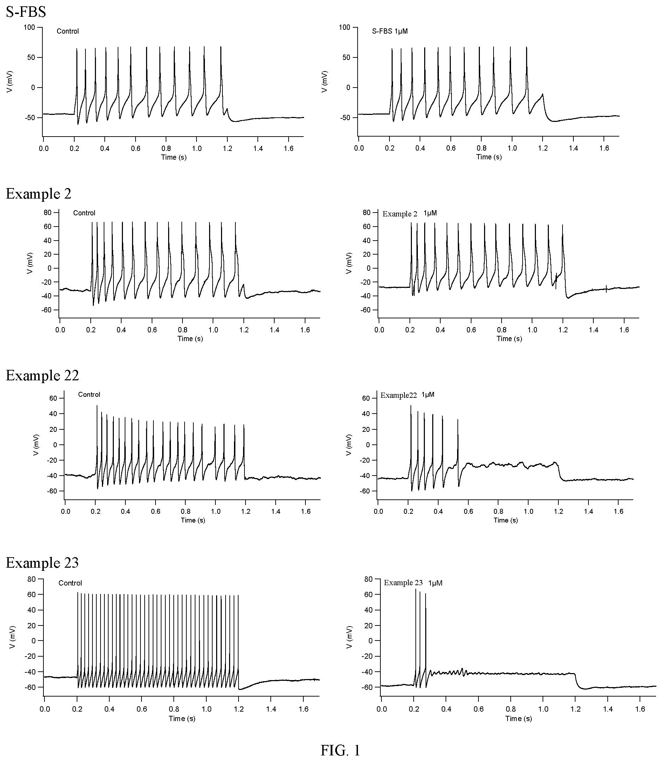

FIG. 1 shows the result of DRG assay.

### DETAILED DESCRIPTION OF THE INVENTION

As used herein, "the compound of the present disclosure" refers to the following compound of formula (I), formula (II), formula (III), formula (III-1) and the like, or an isotopic variant, a tautomer, a stereoisomer, a prodrug, a polymorph, a hydrate or a solvate thereof, or a pharmaceutically acceptable salt thereof.

As used herein, compounds are named using standard nomenclature. For compounds with asymmetric centers, it will be appreciated that all optical isomers and mixtures thereof are included, unless otherwise indicated. Furthermore, all isomeric compounds and carbon-carbon double bonds included in the present disclosure may be present in a Z or E form unless otherwise specified. A compound present in different tautomeric forms is not limited to any particular tautomer, but is intended to encompass all tautomeric forms.

In one embodiment, the present disclosure relates to a compound of formula (I), or an isotopic variant, a tautomer, a stereoisomer, a prodrug, a polymorph, a hydrate or a solvate thereof, or a pharmaceutically acceptable salt thereof:
wherein, 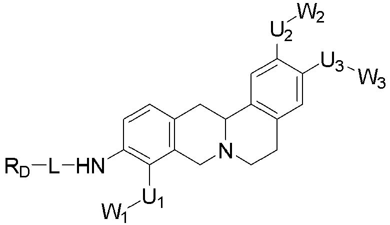
R_{D} is a residue formed by a drug molecule;
U₁, U₂ and U₃ are each independently selected from O, S, -NH-, -C(O)-, -O-C(O)-, - NH-C(O)- and -O-CH₂-O-;
W₁, W₂ and W₃ are each independently selected from H, C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl;
L is a chemical bond or 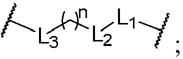 alternatively 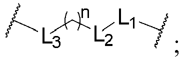
L₁ is selected from chemical bond, -C(O)-, -OC(O)-, -NR_{b}C(O)-, -S(O)-, -S(O)₂-, - OS(O)₁₋₂- and -NR_{b}S(O)₁₋₂-;
L₂ is selected from chemical bond, C₃₋₇ cycloalkylene, 3- to 7-membered heterocyclylene, phenylene and 5- to 6-membered heteroarylene;
L₃ is selected from O, S, NR', -C(O)-, -S(O)- and -S(O)₂-;
n is selected from 0, 1, 2, 3, 4, 5 and 6;
the methylene group in 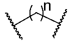 is optionally substituted with 1, 2, 3, 4, 5 or 6 independent R;
R is independently selected from H, D, halogen, CN, =O, -ORₐ, -SRₐ, -NR_{b}R_{c}, -C₀₋₁₀ alkylene-C(O)Rₐ, -C₀₋₁₀ alkylene-OC(O)Rₐ, -C₀₋₁₀ alkylene-C(O)ORₐ, -C₀₋₁₀ alkylene-NR_{b}C(O)Rₐ, -C₀₋₁₀ alkylene-C(O)NR_{b}R_{c}, C₁₋₁₀ alkyl, C₁₋₁₀ haloalkyl, C₃₋₆ cycloalkyl and 3- to 6-membered heterocyclyl;
R' is selected from H, C₁₋₁₈ alkyl, C₁₋₁₈ haloalkyl, C₃₋₁₀ cycloalkyl and 3- to 10-membered heterocyclyl, or R' and R are taken together with the atoms to which they are attached to form 3- to 10-membered heterocyclyl;
Rₐ, R_{b} and R_{c} are each independently selected from H, C₁₋₁₀ alkyl and C₁₋₁₀ haloalkyl, or R_{b} and R_{c} are taken together with the atoms to which they are attached to form 3- to 10-membered heterocyclyl;
wherein each of the above groups is optionally deuterated, up to full deuteration.

In another embodiment, the compound of the present disclosure is further represented by formula (II), formula (III) or formula (III-1): 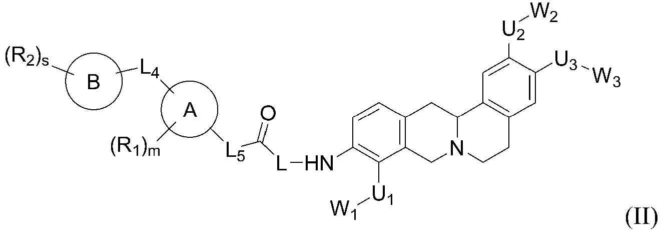 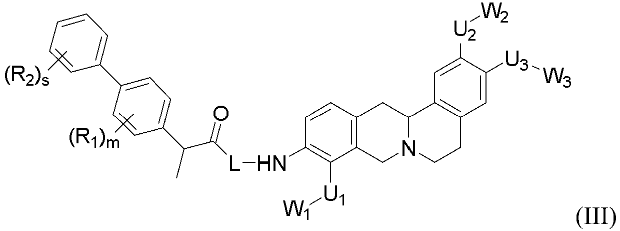 or 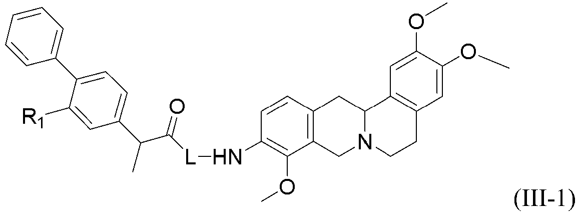 wherein the variables are as defined in the present disclosure.

In the compounds of the present disclosure, each group may be as defined below.

### R_{D}

In one embodiment, R_{D} is a residue formed by a drug molecule.

In one embodiment, the drug molecule is an anti-inflammatory agent and a derivative thereof; in another embodiment, the drug molecule is an anti-inflammatory agent; in another embodiment, the drug molecule is a derivative of an anti-inflammatory agent.

In one embodiment, the anti-inflammatory agent is a non-steroidal anti-inflammatory agent.

In one embodiment, the non-steroidal anti-inflammatory agent is loxoprofen; in another embodiment, the non-steroidal anti-inflammatory agent is flurbiprofen, such as S-flurbiprofen, such as R-flurbiprofen; in another embodiment, the non-steroidal anti-inflammatory agent is fenoprofen; in another embodiment, the non-steroidal anti-inflammatory agent is ketoprofen; in another embodiment, the non-steroidal anti-inflammatory agent is tolmetin; in another embodiment, the non-steroidal anti-inflammatory agent is bromofenic acid; in another embodiment, the non-steroidal anti-inflammatory agent is tiaprofenic acid; in another embodiment, the non-steroidal anti-inflammatory agent is indomethacin; in another embodiment, the non-steroidal anti-inflammatory agent is sulindac; in another embodiment, the non-steroidal anti-inflammatory agent is ketorolac; in another embodiment, the non-steroidal anti-inflammatory agent is nimesulide; in another embodiment, the non-steroidal anti-inflammatory agent is mefenamic acid; in another embodiment, the non-steroidal anti-inflammatory agent is clofenamic acid; in another embodiment, the non-steroidal anti-inflammatory agent is diclofenac; in another embodiment, the non-steroidal anti-inflammatory agent is aspirin; in another embodiment, the non-steroidal anti-inflammatory agent is ibuprofen; in another embodiment, the non-steroidal anti-inflammatory agent is naproxen; in another embodiment, the non-steroidal anti-inflammatory agent is nabumetone; in another embodiment, the non-steroidal anti-inflammatory agent is etodolac; in another embodiment, the non-steroidal anti-inflammatory agent is rofecoxib; in another embodiment, the non-steroidal anti-inflammatory agent is celecoxib; in another embodiment, the non-steroidal anti-inflammatory agent is piroxicam; in another embodiment, the non-steroidal anti-inflammatory agent is meloxicam; in another embodiment, the non-steroidal anti-inflammatory agent is oxyphenbutazone.

In a more specific embodiment, the drug molecule is selected from loxoprofen, flurbiprofen, fenoprofen, ketoprofen, tolmetin, bromofenic acid, tiaprofenic acid, indomethacin, sulindac, ketorolac, nimesulide, mefenamic acid, clofenamic acid, diclofenac, aspirin, ibuprofen, naproxen, nabumetone, etodolac, rofecoxib, celecoxib, piroxicam, meloxicam, and oxyphenbutazone, and derivatives thereof; in another more specific embodiment, the drug molecule is selected from loxoprofen, flurbiprofen, indomethacin, mefenamic acid, clofenamic acid, and aspirin, and derivatives thereof; in another more specific embodiment, the drug molecule is selected from flurbiprofen and clofenamic acid, and derivatives thereof; in another more specific embodiment, the drug molecule is selected from flurbiprofen, and derivatives thereof; in another more specific embodiment, the drug molecule is selected from S-flurbiprofen, and derivatives thereof; in another more specific embodiment, the drug molecule is selected from R-flurbiprofen, and derivatives thereof.

In one embodiment, R_{D} is 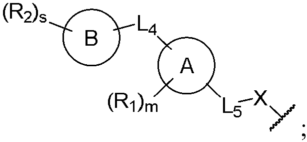 in another embodiment, R_{D} is

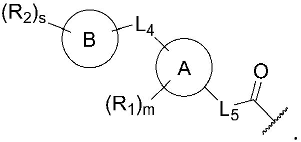

In one embodiment, 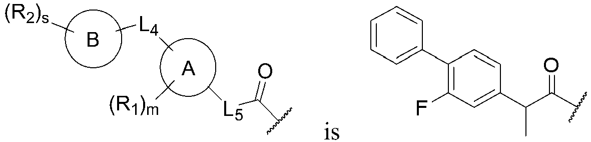 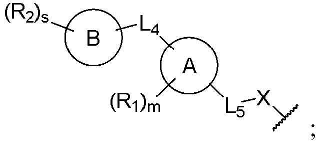 in another embodiment, 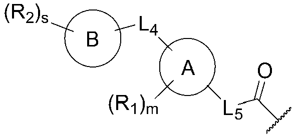 is 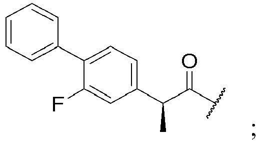 in another embodiment, 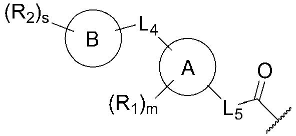 is 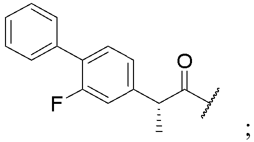 in another embodiment, 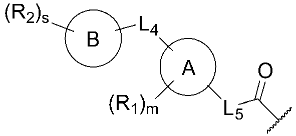 is 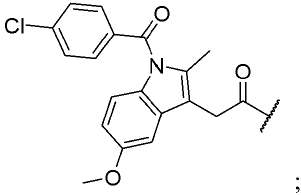 in another embodiment, 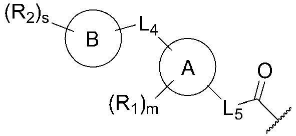 is 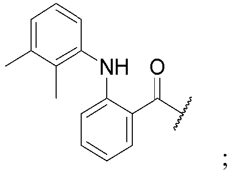 in another embodiment, 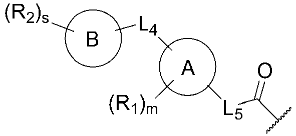 is 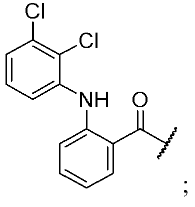 in another embodiment,

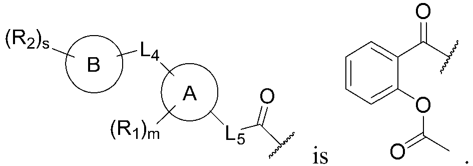

In a more specific embodiment, 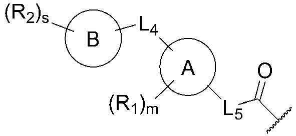 is selected from the following structures: 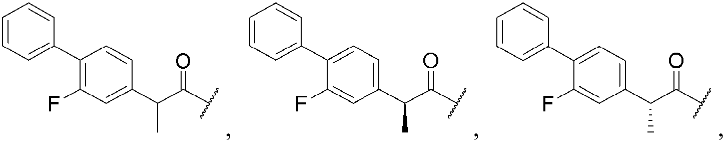 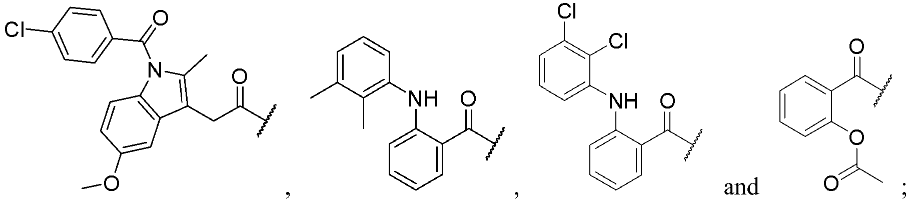 in another more specific embodiment, 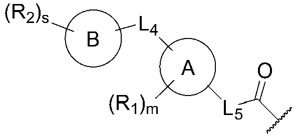 is selected from 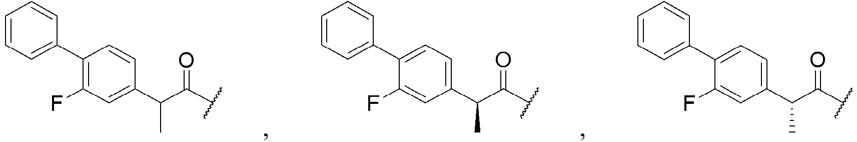 and 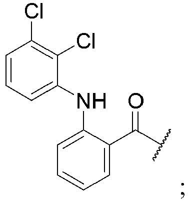 in another more specific embodiment, 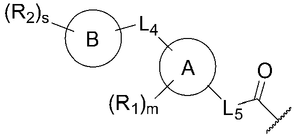 is selected from

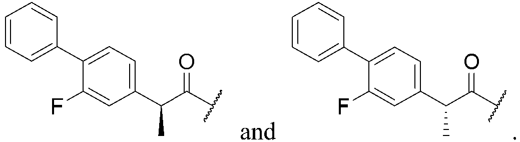

### ring A

In one embodiment, ring A is C₆₋₁₀ arylene, alternatively phenylene, for example, 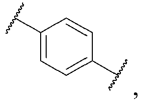 for example, 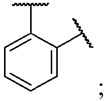 in another embodiment, ring A is 5- to 10-membered heteroarylene, alternatively 9- to 10-membered heteroarylene(e.g., indolylene, e.g., 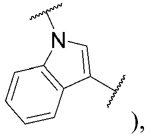 alternatively 5- to 6-membered heteroarylene; in another embodiment, ring A is C₆₋₁₀ aryl fused C₅₋₁₀ cycloalkyl; in another embodiment, ring A is C₆₋₁₀ aryl fused 5- to 10-membered heterocyclyl; in another embodiment, ring A is 5- to 10-membered heteroaryl fused C₅₋₁₀ cycloalkyl; in another embodiment, ring A is 5- to 10-membered heteroaryl fused 5- to 10-membered heterocyclyl.

In a more specific embodiment, ring A is selected from C₆₋₁₀ arylene, 5- to 10-membered heteroarylene, C₆₋₁₀ aryl fused C₅₋₁₀ cycloalkyl, C₆₋₁₀ aryl fused 5- to 10-membered heterocyclyl, 5- to 10-membered heteroaryl fused C₅₋₁₀ cycloalkyl and 5- to 10-membered heteroaryl fused 5- to 10-membered heterocyclyl; in another more specific embodiment, ring A is selected from C₆₋₁₀ arylene and 5- to 10-membered heteroarylene; in another more specific embodiment, ring A is selected from phenylene and 9- to 10-membered heteroarylene; alternatively selected from phenylene and indolylene; in another more specific embodiment, ring A is phenylene; in another more specific embodiment, ring A is selected from 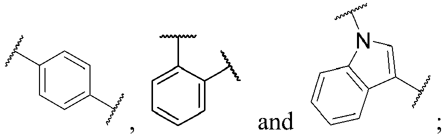 in another more specific embodiment, ring A is selected from 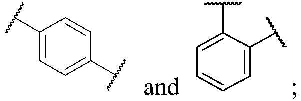 in another more specific embodiment, ring A is

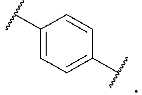

### ring B

In one embodiment, ring B is C₃₋₁₀ cycloalkyl, alternatively C₃₋₇ cycloalkyl; in another embodiment, ring B is 3- to 10-membered heterocyclyl, alternatively 3- to 7-membered heterocyclyl; in another embodiment, ring B is C₆₋₁₀ aryl, alternatively phenyl; in another embodiment, ring B is 5- to 10-membered heteroaryl, alternatively 5- to 6-membered heteroaryl; in another embodiment, L₄-ring B and R₂ are absent.

In a more specific embodiment, ring B is selected from C₃₋₁₀ cycloalkyl, 3- to 10-membered heterocyclyl, C₆₋₁₀ aryl and 5- to 10-membered heteroaryl; in another more specific embodiment, ring B is selected from C₆₋₁₀ aryl and 5- to 10-membered heteroaryl; in another more specific embodiment, ring B is selected from C₃₋₇ cycloalkyl, 3- to 7-membered heterocyclyl, phenyl and 5- to 6-membered heteroaryl; in another more specific embodiment, ring B is selected from phenyl and 5- to 6-membered heteroaryl.

### R₁

In one embodiment, R₁ is H; in another embodiment, R₁ is D; in another embodiment, R₁ is halogen, alternatively F; in another embodiment, R₁ is CN; in another embodiment, R₁ is -NO₂; in another embodiment, R₁ is -ORₐ, alternatively -OMe; in another embodiment, R₁ is - SRₐ; in another embodiment, R₁ is -NR_{b}R_{c}; in another embodiment, R₁ is -C(O)Rₐ; in another embodiment, R₁ is -OC(O)Rₐ, alternatively -OC(O)CH₃; in another embodiment, R₁ is - C(O)ORₐ; in another embodiment, R₁ is -NR_{b}C(O)Rₐ; in another embodiment, R₁ is - C(O)NR_{b}R_{c}; in another embodiment, R₁ is -S(O)Rₐ; in another embodiment, R₁ is -S(O)₂Rₐ; in another embodiment, R₁ is C₁₋₁₈ alkyl, alternatively C₁₋₁₀ alkyl, alternatively C₁₋₆ alkyl, alternatively Me; in another embodiment, R₁ is C₁₋₁₈ haloalkyl, alternatively C₁₋₁₀ haloalkyl, alternatively C₁₋₆ haloalkyl; in another embodiment, R₁ is C₃₋₁₄ cycloalkyl, alternatively C₃₋₁₀ cycloalkyl, alternatively C₃₋₁₀ cycloalkyl, alternatively C₃₋₇ cycloalkyl; in another embodiment, R₁ is 3- to 14-membered heterocyclyl, alternatively 3- to 10-membered heterocyclyl, alternatively 3- to 7-membered heterocyclyl; in another embodiment, R₁ is C₆₋₁₄ aryl, alternatively C₆₋₁₀ aryl, alternatively phenyl; in another embodiment, R₁ is 5- to 14-membered heteroaryl, alternatively 5- to 10-membered heteroaryl, alternatively 5- to 6-membered heteroaryl; in another embodiment, R₁ is optionally substituted with 1, 2, or 3 independent R₁ₛ; in another embodiment, R₁ is unsubstituted.

In a more specific embodiment, R₁ is independently selected from H, D, halogen, CN, -NO₂, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, - S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl; in another more specific embodiment, R₁ is independently selected from H, D, halogen, CN, -NO₂, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, -S(O)Rₐ, -S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5-to 10-membered heteroaryl; in another more specific embodiment, R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, - C(O)NR_{b}R_{c}, C₁₋₆ alkyl, C₁₋₆ haloalkyl, C₃₋₇ cycloalkyl and 3- to 7-membered heterocyclyl; in another more specific embodiment, R₁ is independently selected from H, D, halogen, CN, - ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl; in another more specific embodiment, R₁ is independently selected from H, D, halogen, CN, -ORₐ, -NR_{b}R_{c}, -OC(O)Rₐ, -C(O)ORₐ, C₁₋₆ alkyl and C₁₋₆ haloalkyl; in another more specific embodiment, R₁ is independently selected from H, D, halogen, -ORₐ, -OC(O)Rₐ, C₁₋₆ alkyl and C₁₋₆ haloalkyl; in another more specific embodiment, R₁ is independently selected from H, F, Me, -OMe and -OC(O)CH₃.

In a more specific embodiment, R₁ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, R₁ is independently selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl; in another more specific embodiment, R₁ is independently selected from H, D and halogen; in another more specific embodiment, R₁ is independently H or F.

### R₂

In one embodiment, R₂ is H; in another embodiment, R₂ is D; in another embodiment, R₂ is halogen, alternatively Cl; in another embodiment, R₂ is CN; in another embodiment, R₂ is -NO₂; in another embodiment, R₂ is -ORₐ; in another embodiment, R₂ is -SRₐ; in another embodiment, R₂ is -NR_{b}R_{c}; in another embodiment, R₂ is -C(O)Rₐ; in another embodiment, R₂ is -OC(O)Rₐ; in another embodiment, R₂ is -C(O)ORₐ; in another embodiment, R₂ is - NR_{b}C(O)Rₐ; in another embodiment, R₂ is -C(O)NR_{b}R_{c}; in another embodiment, R₂ is -S(O)Rₐ; in another embodiment, R₂ is -S(O)₂Rₐ; in another embodiment, R₂ is C₁₋₁₈ alkyl, alternatively C₁₋₁₀ alkyl, alternatively C₁₋₆ alkyl, alternatively Me; in another embodiment, R₂ is C₁₋₁₈ haloalkyl, alternatively C₁₋₁₀ haloalkyl, alternatively C₁₋₆ haloalkyl; in another embodiment, R₂ is C₃₋₁₄ cycloalkyl, alternatively C₃₋₁₀ cycloalkyl, alternatively C₃₋₇ cycloalkyl; in another embodiment, R₂ is 3- to 14-membered heterocyclyl, alternatively 3- to 10-membered heterocyclyl, alternatively 3- to 7-membered heterocyclyl; in another embodiment, R₂ is C₆₋₁₄ aryl, alternatively C₆₋₁₀ aryl, alternatively phenyl; in another embodiment, R₂ is 5- to 14-membered heteroaryl, alternatively 5- to 10-membered heteroaryl, alternatively 5- to 6-membered heteroaryl; in another embodiment, R₂ is optionally substituted with 1, 2, or 3 independent R₂ₛ; in another embodiment, R₂ is unsubstituted.

In a more specific embodiment, R₂ is independently selected from H, D, halogen, CN, -NO₂, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, - S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl; in another more specific embodiment, R₂ is independently selected from H, D, halogen, CN, -NO₂, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, -S(O)Rₐ, -S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, R₂ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5-to 10-membered heteroaryl; in another more specific embodiment, R₂ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, - C(O)NR_{b}R_{c}, C₁₋₆ alkyl, C₁₋₆ haloalkyl, C₃₋₇ cycloalkyl, 3- to 7-membered heterocyclyl, phenyl and 5- to 6-membered heteroaryl; in another more specific embodiment, R₂ is independently selected from H, D, halogen, CN, -ORₐ, -SRₐ, -NR_{b}R_{c}, -C(O)Rₐ, -OC(O)Rₐ, -C(O)ORₐ, - NR_{b}C(O)Rₐ, -C(O)NR_{b}R_{c}, C₁₋₆ alkyl and C₁₋₆ haloalkyl; in another more specific embodiment, R₂ is independently selected from H, D, halogen, CN, C₁₋₆ alkyl and C₁₋₆ haloalkyl; in another more specific embodiment, R₂ is independently selected from H, D, halogen, C₁₋₆ alkyl and C₁₋₆ haloalkyl; in another more specific embodiment, R₂ is independently selected from H, Cl and Me; in another more specific embodiment, R₂ is independently selected from H and Cl.

In a more specific embodiment, R₂ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, R₂ is independently selected from H, D, halogen, CN, C₁₋₆ alkyl, C₁₋₆ haloalkyl, C₃₋₇ cycloalkyl and 3- to 7-membered heterocyclyl; in another more specific embodiment, R₂ is independently selected from H, D, halogen, C₁₋₆ alkyl and C₁₋₆ haloalkyl; in another more specific embodiment, R₂ is independently selected from H, D and halogen; in another more specific embodiment, R₂ is independently H or D.

### R₁ₛ and R₂ₛ

In one embodiment, R₁ₛ is H; in another embodiment, R₁ₛ is D; in another embodiment, R₁ₛ is halogen; in another embodiment, R₁ₛ is CN; in another embodiment, R₁ₛ is -ORₐ; in another embodiment, R₁ₛ is -SRₐ; in another embodiment, R₁ₛ is -NR_{b}R_{c}; in another embodiment, R₁ₛ is -OC(O)Rₐ; in another embodiment, R₁ₛ is -C(O)ORₐ; in another embodiment, R₁ₛ is -NR_{b}C(O)Rₐ; in another embodiment, R₁ₛ is -C(O)NR_{b}R_{c}; in another embodiment, R₁ₛ is C₁₋₁₀ alkyl, alternatively C₁₋₆ alkyl; in another embodiment, R₁ₛ is C₁₋₁₀ haloalkyl, alternatively C₁₋₆ haloalkyl; in another embodiment, R₁ₛ is C₃₋₁₀ cycloalkyl, alternatively C₃₋₇ cycloalkyl; in another embodiment, R₁ₛ is 3- to 10-membered heterocyclyl, alternatively 3- to 7-membered heterocyclyl.

In one embodiment, R₂ₛ is H; in another embodiment, R₂ₛ is D; in another embodiment, R₂ₛ is halogen; in another embodiment, R₂ₛ is CN; in another embodiment, R₂ₛ is -ORₐ; in another embodiment, R₂ₛ is -SRₐ; in another embodiment, R₂ₛ is -NR_{b}R_{c}; in another embodiment, R₂ₛ is -OC(O)Rₐ; in another embodiment, R₂ₛ is -C(O)ORₐ; in another embodiment, R₂ₛ is -NR_{b}C(O)Rₐ; in another embodiment, R₂ₛ is -C(O)NR_{b}R_{c}; in another embodiment, R₂ₛ is C₁₋₁₀ alkyl, alternatively C₁₋₆ alkyl; in another embodiment, R₂ₛ is C₁₋₁₀ haloalkyl, alternatively C₁₋₆ haloalkyl; in another embodiment, R₂ₛ is C₃₋₁₀ cycloalkyl, alternatively C₃₋₇ cycloalkyl; in another embodiment, R₂ₛ is 3- to 10-membered heterocyclyl, alternatively 3- to 7-membered heterocyclyl.

In a more specific embodiment, R₁ₛ is independently selected from H, D, halogen, CN, C₁₋₁₀ alkyl, C₁₋₁₀ haloalkyl, C₃₋₁₀ cycloalkyl and 3- to 10-membered heterocyclyl; in another more specific embodiment, R₁ₛ is independently selected from H, D, halogen, CN, C₁₋₆ alkyl, C₁₋₆ haloalkyl, C₃₋₇ cycloalkyl and 3- to 7-membered heterocyclyl; in another more specific embodiment, R₁ₛ is independently selected from H, D, halogen, C₁₋₆ alkyl and C₁₋₆ haloalkyl.

In a more specific embodiment, R₂ₛ is independently selected from H, D, halogen, CN, C₁₋₁₀ alkyl, C₁₋₁₀ haloalkyl, C₃₋₁₀ cycloalkyl and 3- to 10-membered heterocyclyl; in another more specific embodiment, R₂ₛ is independently selected from H, D, halogen, CN, C₁₋₆ alkyl, C₁₋₆ haloalkyl, C₃₋₇ cycloalkyl and 3- to 7-membered heterocyclyl; in another more specific embodiment, R₂ₛ is independently selected from H, D, halogen, C₁₋₆ alkyl and C₁₋₆ haloalkyl.

### m

In one embodiment, m is 0; in another embodiment, m is 1; in another embodiment, m is 2; in another embodiment, m is 3; in another embodiment, m is 4; in another embodiment, m is 5.

In a more specific embodiment, m=0, 1, 2, 3, 4 or 5; in another more specific embodiment, m=0, 1, 2, 3 or 4; in another more specific embodiment, m=0, 1 or 2; in another more specific embodiment, m=0 or 1.

### s

In one embodiment, s is 0; in another embodiment, s is 1; in another embodiment, s is 2; in another embodiment, s is 3; in another embodiment, s is 4; in another embodiment, s is 5.

In a more specific embodiment, s=0, 1, 2, 3, 4 or 5; in another more specific embodiment, s=0, 1 or 2.

### L₄

In one embodiment, L₄ is a chemical bond ; in another embodiment, L₄ is -CR₄R'₄-, alternatively -CH₂-; in another embodiment, L₄ is -NR_{b}-, alternatively -NH-; in another embodiment, L₄ is O; in another embodiment, L₄ is S; in another embodiment, L₄ is -C(O)-; in another embodiment, L₄ is -OC(O)-; in another embodiment, L₄ is -C(O)O-; in another embodiment, L₄ is -NR_{b}C(O)-; in another embodiment, L₄ is -C(O)NR_{b}-; in another embodiment, L₄ is -S(O)-; in another embodiment, L₄ is -S(O)₂-; in another embodiment, L₄ and the carbon atom on ring A to which L₄ is attached together form 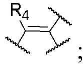 in another embodiment, L₄ and the carbon atom on ring A to which L₄ is attached do not form

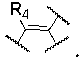

In a more specific embodiment, L₄ is selected from chemical bond, -CR₄R'₄-, -NR_{b}-, O, S, -C(O)-, -OC(O)-, -C(O)O-, -NR_{b}C(O)-, -C(O)NR_{b}-, -S(O)- and -S(O)₂-; in another more specific embodiment, L₄ is selected from chemical bond, -CR₄R'₄-, -NR_{b}-, O, S, -C(O)- and - S(O)-; in another more specific embodiment, L₄ is selected from chemical bond, -CR₄R'₄-, - NR_{b}-, O and -C(O)-; in another more specific embodiment, L₄ is selected from chemical bond, -NR_{b}- and -C(O)-; in another more specific embodiment, L₄ is selected from chemical bond, - NH- and -C(O)-; in another more specific embodiment, L₄is selected from a chemical bond and -NH-.

### L₅

In one embodiment, L₅ is a chemical bond ; in another embodiment, L₅ is O; in another embodiment, L₅ is S; in another embodiment, L₅ is NR'; in another embodiment, L₅ is C₁₋₁₀ alkylene, alternatively C₁₋₆ alkylene, alternatively C₁₋₄ alkylene, alternatively C₁₋₂ alkylene, alternatively methylene; in another embodiment, L₅ is optionally substituted with one or more independent R₃; in another embodiment, L₅ is optionally substituted with 1, 2, 3, 4, 5, or 6 independent R₃; in another embodiment, L₅ is optionally substituted with 1, 2, or 3 independent R₃; in another embodiment, L₅ is optionally substituted with 1 R₃; in another embodiment, L₅ is unsubstituted.

In a more specific embodiment, L₅ is selected from chemical bond, O, S, NR' and C₁₋₁₀ alkylene; in another more specific embodiment, L₅ is a chemical bond or C₁₋₁₀ alkylene; in another more specific embodiment, L₅ is a chemical bond or C₁₋₆ alkylene; in another more specific embodiment, L₅ is a chemical bond or C₁₋₄ alkylene; in another more specific embodiment, L₅ is a chemical bond or C₁₋₂ alkylene; in another more specific embodiment, L₅ is a chemical bond or methylene; in another more specific embodiment, L₅ is -CH(CH₃)-.

### R₃

In one embodiment, R₃ is H; in another embodiment, R₃ is D; in another embodiment, R₃ is halogen; in another embodiment, R₃ is C₁₋₁₀ alkyl, alternatively C₁₋₆ alkyl, alternatively C₁₋₄ alkyl; in another embodiment, R₃ is C₁₋₁₀ haloalkyl, alternatively C₁₋₆ haloalkyl, alternatively C₁₋₄ haloalkyl.

In a more specific embodiment, R₃ is independently selected from H, D, halogen, C₁₋₁₀ alkyl and C₁₋₁₀ haloalkyl; in another more specific embodiment, R₃ is independently selected from H, D, halogen, C₁₋₆ alkyl and C₁₋₆ haloalkyl; in another more specific embodiment, R₃ is independently selected from H, D, C₁₋₄ alkyl and C₁₋₄ haloalkyl; in another more specific embodiment, R₃ is independently selected from H and Me; in another more specific embodiment, R₃ is Me.

### R₄ and R'₄

In one embodiment, R₄ is H; in another embodiment, R₄ is D; in another embodiment, R₄ is halogen; in another embodiment, R₄ is C₁₋₆ alkyl; in another embodiment, R₄ is C₁₋₆ haloalkyl.

In one embodiment, R'₄ is H; in another embodiment, R'₄ is D; in another embodiment, R'₄ is halogen; in another embodiment, R'₄ is C₁₋₆ alkyl; in another embodiment, R'₄ is C₁₋₆ haloalkyl.

In a more specific embodiment, R₄ and R'₄ are independently selected from H, D, halogen, C₁₋₆ alkyl and C₁₋₆ haloalkyl; in another more specific embodiment, R₄ and R'₄ are independently selected from H, D, C₁₋₆ alkyl and C₁₋₆ haloalkyl; in another more specific embodiment, R₄ and R'₄ are independently H or D.

### X

In one embodiment, X is -C(O)-; in another embodiment, X is -S(O)-; in another embodiment, X is -S(O)₂-.

In a more specific embodiment, X is selected from -C(O)-, -S(O)- and -S(O)₂-.

### U₁, U₂ and U₃

In one embodiment, U₁ is O; in another embodiment, U₁ is S; in another embodiment, U₁ is -NH-; in another embodiment, U₁ is -C(O)-; in another embodiment, U₁ is -O-C(O)-; in another embodiment, U₁ is -NH-C(O)-; in one embodiment, U₁ is -O-CH₂-O-.

In one embodiment, U₂ is O; in another embodiment, U₂ is S; in one embodiment, U₂ is -NH-; in another embodiment, U₂ is -C(O)-; in another embodiment, U₂ is -O-C(O)-; in another embodiment, U₂ is -NH-C(O)-; in one embodiment, U₂ is -O-CH₂-O-.

In one embodiment, U₃ is O; in another embodiment, U₃ is S; in one embodiment, U₃ is -NH-; in another embodiment, U₃ is -C(O)-; in another embodiment, U₃ is -O-C(O)-; in another embodiment, U₃ is -NH-C(O)-; in one embodiment, U₃ is O-CH₂-O-.

In a more specific embodiment, U₁, U₂ and U₃ are each independently selected from O, S, -NH- and -C(O)-; in another more specific embodiment, U₁, U₂ and U₃ is O.

### W₁, W₂ and W₃

In one embodiment, W₁ is H; in another embodiment, W₁ is C₁₋₁₀ alkyl, alternatively C₁₋₆ alkyl, alternatively methyl; in another embodiment, W₁ is C₁₋₁₀ deuterated alkyl, alternatively C₁₋₆ deuterated alkyl, alternatively CD₃; in another embodiment, W₁ is C₁₋₁₀ haloalkyl, alternatively C₁₋₆ haloalkyl; in another embodiment, W₁ is C₃₋₁₀ cycloalkyl, alternatively C₃₋₇ cycloalkyl; in another embodiment, W₁ is 3- to 10-membered heterocyclyl, alternatively 3- to 7-membered heterocyclyl; in another embodiment, W₁ is C₆₋₁₀ aryl, alternatively phenyl; in another embodiment, W₁ is 5- to 10-membered heteroaryl, alternatively 5- to 6-membered heteroaryl.

In one embodiment, W₂ is H; in another embodiment, W₂ is C₁₋₁₀ alkyl, alternatively C₁₋₆ alkyl, alternatively methyl; in another embodiment, W₂ is C₁₋₁₀ deuterated alkyl, alternatively C₁₋₆ deuterated alkyl, alternatively CD₃; in another embodiment, W₂ is C₁₋₁₀ haloalkyl, alternatively C₁₋₆ haloalkyl; in another embodiment, W₂ is C₃₋₁₀ cycloalkyl, alternatively C₃₋₇ cycloalkyl; in another embodiment, W₂ is 3- to 10-membered heterocyclyl, alternatively 3- to 7-membered heterocyclyl; in another embodiment, W₂ is C₆₋₁₀ aryl, alternatively phenyl; in another embodiment, W₂ is 5- to 10-membered heteroaryl, alternatively 5- to 6-membered heteroaryl.

In one embodiment, W₃ is H; in another embodiment, W₃ is C₁₋₁₀ alkyl, alternatively C₁₋₆ alkyl, alternatively methyl; in another embodiment, W₃ is C₁₋₁₀ deuterated alkyl, alternatively C₁₋₆ deuterated alkyl, alternatively CD₃; in another embodiment, W₃ is C₁₋₁₀ haloalkyl, alternatively C₁₋₆ haloalkyl; in another embodiment, W₃ is C₃₋₁₀ cycloalkyl, alternatively C₃₋₇ cycloalkyl; in another embodiment, W₃ is 3- to 10-membered heterocyclyl, alternatively 3- to 7-membered heterocyclyl; in another embodiment, W₃ is C₆₋₁₀ aryl, alternatively phenyl; in another embodiment, W₃ is 5- to 10-membered heteroaryl, alternatively 5- to 6-membered heteroaryl.

In a more specific embodiment, W₁ is selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, W₁ is selected from C₁₋₁₀ alkyl, C₁₋₁₀ deuterated alkyl, C₁₋₁₀ haloalkyl, C₃₋₁₀ cycloalkyl and 3- to 10-membered heterocyclyl; in another more specific embodiment, W₁ is selected from C₁₋₁₀ alkyl, C₁₋₁₀ deuterated alkyl and C₁₋₁₀ haloalkyl; in another more specific embodiment, W₁ is selected from C₁₋₆ alkyl, C₁₋₆ deuterated alkyl and C₁₋₆ haloalkyl; in another more specific embodiment, W₁ is C₁₋₆ alkyl or C₁₋₆ deuterated alkyl; in another more specific embodiment, W₁ is methyl or CD₃; in another more specific embodiment, W₁ is C₁₋₆ alkyl; in another more specific embodiment, W₁, W₂ and W₃ is methyl.

In a more specific embodiment, W₂ is selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, W₂ is selected from C₁₋₁₀ alkyl, C₁₋₁₀ deuterated alkyl, C₁₋₁₀ haloalkyl, C₃₋₁₀ cycloalkyl and 3- to 10-membered heterocyclyl; in another more specific embodiment, W₂ is selected from C₁₋₁₀ alkyl, C₁₋₁₀ deuterated alkyl and C₁₋₁₀ haloalkyl; in another more specific embodiment, W₂ is selected from C₁₋₆ alkyl, C₁₋₆ deuterated alkyl and C₁₋₆ haloalkyl; in another more specific embodiment, W₂ is C₁₋₆ alkyl or C₁₋₆ deuterated alkyl; in another more specific embodiment, W₂ is methyl or CD₃; in another more specific embodiment, W₂ is C₁₋₆ alkyl; in another more specific embodiment, W₂ is methyl.

In a more specific embodiment, W₃ is selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl; in another more specific embodiment, W₃ is selected from C₁₋₁₀ alkyl, C₁₋₁₀ deuterated alkyl, C₁₋₁₀ haloalkyl, C₃₋₁₀ cycloalkyl and 3- to 10-membered heterocyclyl; in another more specific embodiment, W₃ is selected from C₁₋₁₀ alkyl, C₁₋₁₀ deuterated alkyl and C₁₋₁₀ haloalkyl; in another more specific embodiment, W₃ is selected from C₁₋₆ alkyl, C₁₋₆ deuterated alkyl and C₁₋₆ haloalkyl; in another more specific embodiment, W₃ is C₁₋₆ alkyl or C₁₋₆ deuterated alkyl; in another more specific embodiment, W₃ is methyl or CD₃; in another more specific embodiment, W₃ is C₁₋₆ alkyl; in another more specific embodiment, W₃ is methyl.

### L

In one embodiment, L is a chemical bond; in another embodiment, L is 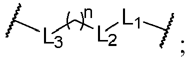 in another embodiment, L is 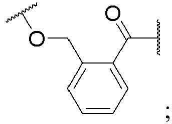 in another embodiment, L is 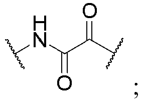 in another embodiment, L is 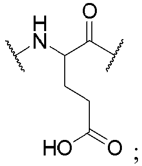 in another embodiment, L is 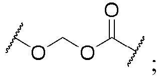 in another embodiment, L is 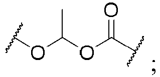 in another embodiment, L is 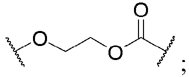 in another embodiment, L is 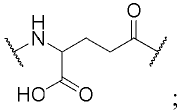 in another embodiment, L 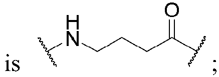 in another embodiment, L is 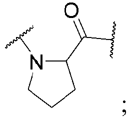 in another embodiment, L is 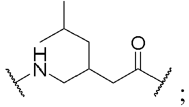 in another embodiment, L is 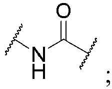 in another embodiment, L is 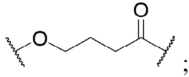 in another embodiment, L is 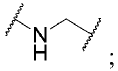 in another embodiment, L is

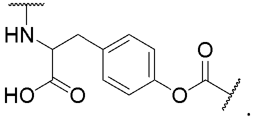

In one embodiment, the methylene group in 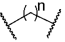 is optionally substituted with 1, 2, 3, 4, 5 or 6 independent R; in another embodiment, the methylene group in 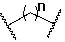 is optionally substituted with 1, 2, or 3 R; in another embodiment, the methylene group in 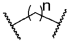 is optionally substituted with 1 R.

In a more specific embodiment, L is selected from a chemical bond, 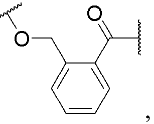 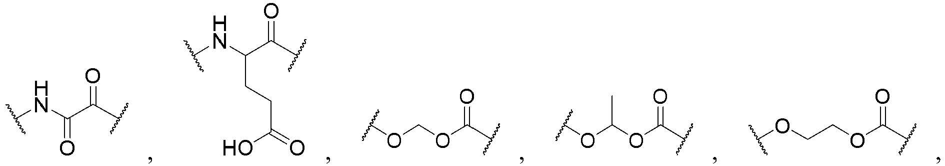 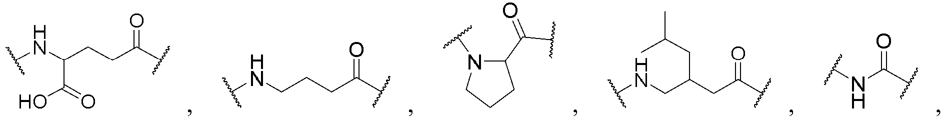 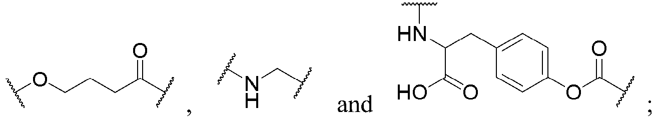 in another more specific embodiment, L is selected from a chemical bond, 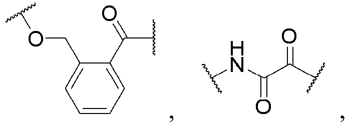 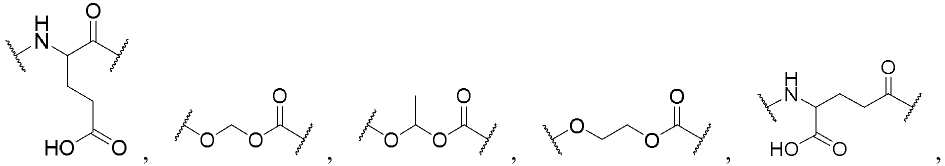 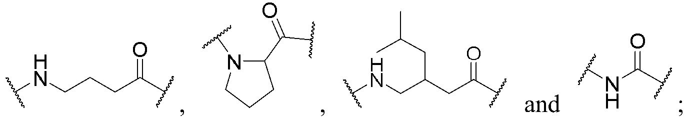 in another more specific embodiment, L is selected from a chemical bond, 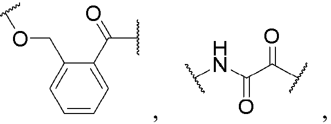 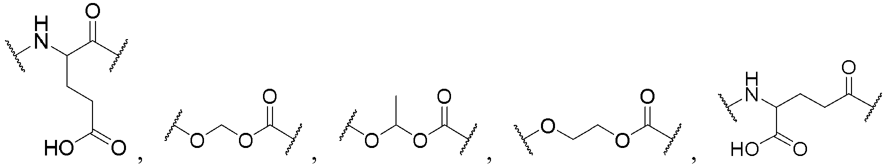 and 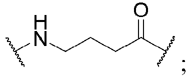 in another more specific embodiment, L is selected from a chemical bond, 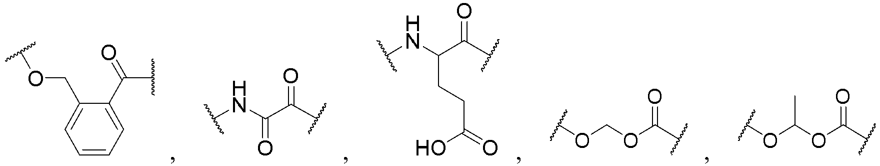 and 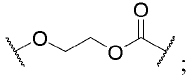 in another more specific embodiment, L is selected from a chemical bond, 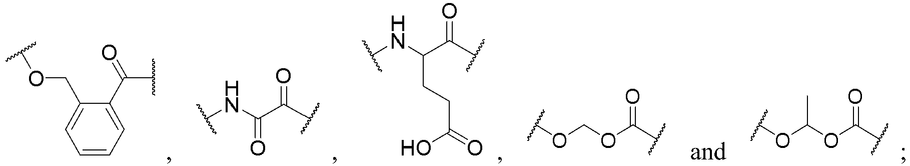 in another more specific embodiment, L is selected from:

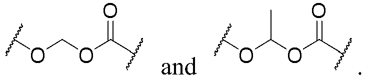

In a more specific embodiment,

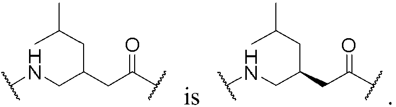

In a more specific embodiment,

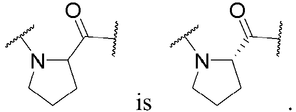

In a more specific embodiment,

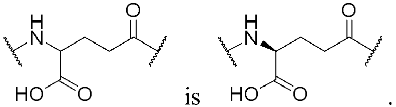

In a more specific embodiment,

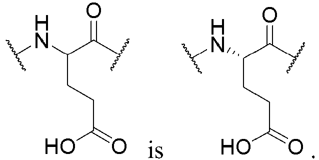

In a more specific embodiment, 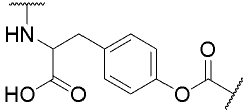 is

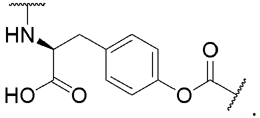

### L₁

In one embodiment, L₁ is a chemical bond; in another embodiment, L₁ is -C(O)-; in another embodiment, L₁ is -OC(O)-; in another embodiment, L₁ is -NR_{b}C(O)-, for example, is -NHC(O)-; in another embodiment, L₁ is -S(O)-; in another embodiment, L₁ is -S(O)₂-; in another embodiment, L₁ is -OS(O)₁₋₂-; in another embodiment, L₁ is -NR_{b}S(O)₁₋₂-, for example, is -NHS(O)₁₋₂-.

In a more specific embodiment, L₁ is selected from chemical bond, -C(O)-, -OC(O)-and -NHC(O)-; in another more specific embodiment, L₁ is selected from -C(O)- and -OC(O)-; in another more specific embodiment, L₁ is -C(O)-.

In a more specific embodiment, L₁ is selected from -C(O)-, -OC(O)- and -NHC(O)-; in another more specific embodiment, L₁ is selected from -OC(O)- and -NHC(O)-; in another more specific embodiment, L₁ is selected from -OC(O)-.

### L₂

In one embodiment, L₂ is a chemical bond; in another embodiment, L₂ is C₃₋₇ cycloalkylene; in another embodiment, L₂ is 3- to 7-membered heterocyclylene; in another embodiment, L₂ is phenylene, for example, 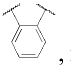 for example, 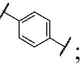 in another embodiment, L₂ is 5- to 6-membered heteroarylene.

In a more specific embodiment, L₂ is selected from chemical bond, C₃₋₇ cycloalkylene, 3- to 7-membered heterocyclylene, phenylene and 5- to 6-membered heteroarylene; in another more specific embodiment, L₂ is selected from chemical bond, phenylene and 5- to 6-membered heteroarylene; in another more specific embodiment, L₂ is selected from a chemical bond and phenylene; in another more specific embodiment, L₂ is selected from chemical bond, 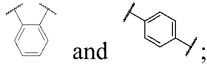 in another more specific embodiment, L₂ is selected from a chemical bond and

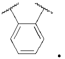

### L₃

In one embodiment, L₃ is O; in another embodiment, L₃ is S; in another embodiment, L₃ is NR', for example, NH; in another embodiment, L₃ is -C(O)-; in another embodiment, L₃ is -S(O)-; in another embodiment, L₃ is -S(O)₂-.

In a more specific embodiment, L₃ is selected from O, S and NR'; in another more specific embodiment, L₂ is selected from O and NR'; in another more specific embodiment, L₂ is selected from O and NH; in another more specific embodiment, L₂ is selected from O and S.

### n

In one embodiment, n is 0; in another embodiment, n is 1; in another embodiment, n is 2; in another embodiment, n is 3; in another embodiment, n is 4; in another embodiment, n is 5; in another embodiment, n is 6.

In a more specific embodiment, n is selected from 0, 1, 2 and 3; in another more specific embodiment, n is selected from 1, 2 and 3; in another more specific embodiment, n is 1 or 2.

### R

In one embodiment, R is H; in another embodiment, R is D; in another embodiment, R is halogen; in another embodiment, R is CN; in another embodiment, R is =O; in another embodiment, R is -ORₐ; in another embodiment, R is -SRₐ; in another embodiment, R is -NR_{b}R_{c}; in another embodiment, R is -C₀₋₁₀ alkylene-C(O)Rₐ, alternatively -C₀₋₆ alkylene-C(O)Rₐ, alternatively -C₀₋₃ alkylene-C(O)Rₐ; in another embodiment, R is -C₀₋₁₀ alkylene-OC(O)Rₐ, alternatively -C₀₋₆ alkylene-OC(O)Rₐ, alternatively -C₀₋₃ alkylene-OC(O)Rₐ; in another embodiment, R is -C₀₋₁₀ alkylene-C(O)ORₐ, alternatively -C₀₋₆ alkylene-C(O)ORₐ, alternatively -C₀₋₃ alkylene-C(O)ORₐ, alternatively C_{O-3} alkylene-C(O)OH, for example,-C(O)OH, for example, -(CH₂)₂C(O)OH; in another embodiment, R is -C₀₋₁₀ alkylene-NR_{b}C(O)Rₐ, alternatively -C₀₋₆ alkylene-NR_{b}C(O)Rₐ, alternatively -C₀₋₃ alkylene-NR_{b}C(O)Rₐ; in another embodiment, R is -C₀₋₁₀ alkylene-C(O)NR_{b}R_{c}, alternatively -C₀₋₆ alkylene-C(O)NR_{b}R_{c}, alternatively -C₀₋₃ alkylene-C(O)NR_{b}R_{c}; in another embodiment, R is C₁₋₁₀ alkyl, alternatively C₁₋₆ alkyl(e.g., isobutyl), alternatively C₁₋₃ alkyl(e.g., methyl); in another embodiment, R is C₁₋₁₀ haloalkyl, alternatively C₁₋₆ haloalkyl, alternatively C₁₋₃ haloalkyl; in another embodiment, R is C₃₋₆ cycloalkyl; in another embodiment, R is 3- to 6-membered heterocyclyl.

In a more specific embodiment, R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ; in another more specific embodiment, R is independently selected from H, D, =O, C₁₋₆ alkyl, C₁₋₆ haloalkyl, -C₀₋₆ alkylene-C(O)ORₐ and -C₀₋₆ alkylene-OC(O)Rₐ; in another more specific embodiment, R is independently selected from H, D, =O, C₁₋₆ alkyl, C₁₋₆ haloalkyl and -C₀₋₆ alkylene-C(O)ORₐ; in another more specific embodiment, R is independently selected from H, D, =O, C₁₋₃ alkyl, C₁₋₃ haloalkyl and -C₀₋₆ alkylene-C(O)ORₐ; in another more specific embodiment, R is independently selected from H, D, =O, C₁₋₃ alkyl and -C₀₋₆ alkylene-C(O)ORₐ; in another more specific embodiment, R is independently selected from H, D, =O, C₁₋₃ alkyl and -C₀₋₃ alkylene-C(O)ORₐ; in another more specific embodiment, R is independently selected from H, D, =O and -C₀₋₃ alkylene-C(O)OH.

In a more specific embodiment, R is independently selected from H, =O, methyl, isobutyl, -C(O)OH and -(CH₂)₂C(O)OH; in another more specific embodiment, R is independently selected from H, =O, methyl, -C(O)OH and -(CH₂)₂C(O)OH; in another more specific embodiment, R is independently selected from H, =O, methyl and -(CH₂)₂C(O)OH; in another more specific embodiment, R is independently selected from H, =O and - (CH₂)₂C(O)OH.

In a more specific embodiment, R is independently selected from H, D, halogen, C₁₋₆ alkyl and C₁₋₆ haloalkyl; in another more specific embodiment, R is independently selected from H, D, C₁₋₆ alkyl and C₁₋₆ haloalkyl; in another more specific embodiment, R is independently selected from H, D, C₁₋₃ alkyl and C₁₋₃ haloalkyl; in another more specific embodiment, R is independently selected from H and Me.

### R'

In one embodiment, R' is H; in another embodiment, R' is C₁₋₁₈ alkyl, alternatively C₁₋₁₀ alkyl, alternatively C₁₋₆ alkyl; in another embodiment, R' is C₁₋₁₈ haloalkyl, alternatively C₁₋₁₀ haloalkyl, alternatively C₁₋₆ haloalkyl; in another embodiment, R' is C₃₋₁₀ cycloalkyl, alternatively C₃₋₇ cycloalkyl; in another embodiment, R' is 3- to 10-membered heterocyclyl, alternatively 3- to 7-membered heterocyclyl; in another embodiment, R' and R are taken together with the atoms to which they are attached to form 3- to 10-membered heterocyclyl, alternatively to 3- to 7-membered heterocyclyl, alternatively to form 4- to 6-membered heterocyclyl, alternatively to form 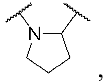 alternatively to form 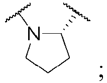 in another embodiment, R' and R are not taken together with the atoms to which they are attached to form a ring.

In a more specific embodiment, R' is selected from H, C₁₋₁₀ alkyl, C₁₋₁₀ haloalkyl, C₃₋₁₀ cycloalkyl and 3- to 10-membered heterocyclyl; in another more specific embodiment, R' is selected from H, C₁₋₆ alkyl and C₁₋₆ haloalkyl.

### Rₐ, R_{b} and R_{c}

In one embodiment, Rₐ is H; in another embodiment, Rₐ is C₁₋₁₀ alkyl, alternatively C₁₋₆ alkyl; in another embodiment, Rₐ is C₁₋₁₀ haloalkyl, alternatively C₁₋₆ haloalkyl; in another embodiment, R_{b}, R_{c} are taken together with the atoms to which they are attached to form 3- to 10-membered heterocyclyl, alternatively to form 5- to 7-membered heterocyclyl; in another embodiment, R_{b}, R_{c} are not taken together with the atoms to which they are attached to form a ring.

In a more specific embodiment, Rₐ, R_{b} and R_{c} are each independently selected from H, C₁₋₆ alkyl and C₁₋₆ haloalkyl.

Any one of the technical solutions, or any combination thereof, in any one of the specific embodiments can be combined with any one of the technical solutions, or any combination thereof, in other specific embodiments. For example, any one of the technical solutions, or any combination thereof, of R_{D} can be combined with any one of the technical solutions, or any combination thereof, of U₁, U₂, U₃, W₁, W₂, W₃, L, L₁, L₂, L₃, n, R, R', ring A, ring B, R₁, R₂, R₁ₛ, R₂ₛ, m, s, L₄, L₅, R₃, R₄, R'₄, X, Rₐ, R_{b} and R_{c}, and the like. The present disclosure is intended to include all combinations of these technical solutions, not all of which are described herein due to limited space.

In a more specific embodiment, the present disclosure provides a compound of formula (I), or an isotopic variant, a tautomer, a stereoisomer, a prodrug, a polymorph, a hydrate or a solvate thereof, or a pharmaceutically acceptable salt thereof: 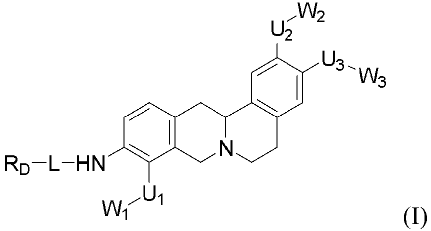 wherein,
R_{D} is a residue formed by a drug molecule;
U₁, U₂ and U₃ are each independently selected from O, S, -NH-, -C(O)-, -O-C(O)-,-NH-C(O)- and -O-CH₂-O-;
W₁, W₂ and W₃ are each independently selected from H, C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl;
L is a chemical bond or 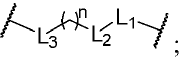 alternatively 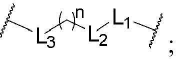
L₁ is selected from chemical bond, -C(O)-, -OC(O)-, -NR_{b}C(O)-, -S(O)-, -S(O)₂-,-OS(O)₁₋₂- and -NR_{b}S(O)₁₋₂-;
L₂ is selected from chemical bond, C₃₋₇ cycloalkylene, 3- to 7-membered heterocyclylene, phenylene and 5- to 6-membered heteroarylene;
L₃ is selected from O, S, NR', -C(O)-, -S(O)- and -S(O)₂-;
n is selected from 0, 1, 2, 3, 4, 5 and 6;
the methylene group in 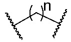 is optionally substituted with 1, 2, 3, 4, 5 or 6 independent R;
R is independently selected from H, D, halogen, CN, =O, -ORₐ, -SRₐ, -NR_{b}R_{c}, -C₀₋₁₀ alkylene-C(O)Rₐ, -C₀₋₁₀ alkylene-OC(O)Rₐ, -C₀₋₁₀ alkylene-C(O)ORₐ, -C₀₋₁₀ alkylene-NR_{b}C(O)Rₐ, -C₀₋₁₀ alkylene-C(O)NR_{b}R_{c}, C₁₋₁₀ alkyl, C₁₋₁₀ haloalkyl, C₃₋₆ cycloalkyl and 3- to 6-membered heterocyclyl;
R' is selected from H, C₁₋₁₈ alkyl, C₁₋₁₈ haloalkyl, C₃₋₁₀ cycloalkyl and 3- to 10-membered heterocyclyl, or R' and R are taken together with the atoms to which they are attached to form 3- to 10-membered heterocyclyl;
Rₐ, R_{b} and R_{c} are each independently selected from H, C₁₋₁₀ alkyl and C₁₋₁₀ haloalkyl, or R_{b} and R_{c} are taken together with the atoms to which they are attached to form 3- to 10-membered heterocyclyl;
wherein each of the above groups is optionally deuterated, up to full deuteration.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R_{D} is a residue formed by a drug molecule, the drug molecule is an anti-inflammatory agent and a derivative thereof, alternatively a non-steroidal anti-inflammatory agent, for example, loxoprofen, flurbiprofen, fenoprofen, ketoprofen, tolmetin, bromofenic acid, tiaprofenic acid, indomethacin, sulindac, ketorolac, nimesulide, mefenamic acid, clofenamic acid, diclofenac, aspirin, ibuprofen, naproxen, nabumetone, etodolac, rofecoxib, celecoxib, piroxicam, meloxicam, and oxyphenbutazone, and derivatives thereof.

In a more specific embodiment, the drug molecule is selected from loxoprofen, flurbiprofen, indomethacin, mefenamic acid, clofenamic acid, and aspirin, and derivatives thereof; alternatively selected from flurbiprofen and clofenamic acid, and derivatives thereof; alternatively flurbiprofen and a derivative thereof; alternatively the flurbiprofen is S-flurbiprofen; alternatively the flurbiprofen is R-flurbiprofen.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, U₁, U₂ and U₃ are each independently selected from O, S, -NH- and -C(O)-, alternatively O.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl, alternatively selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively methyl or CD₃, alternatively C₁₋₆ alkyl, alternatively methyl.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, L₁ is selected from chemical bond, -C(O)-, -OC(O)- and -NHC(O)-, alternatively selected from chemical bond, -C(O)- and -OC(O)-, alternatively selected from -C(O)- and - OC(O)-, alternatively -C(O)-.

In a more specific embodiment, L₁ is selected from -C(O)-, -OC(O)- and -NHC(O)-; alternatively -OC(O)- and -NHC(O)-; alternatively -OC(O)-.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, L₂ is selected from a chemical bond, C₃₋₇ cycloalkylene, 3- to 7-membered heterocyclylene, phenylene and 5- to 6-membered heteroarylene, alternatively selected from chemical bond, phenylene and 5- to 6-membered heteroarylene, alternatively selected from a chemical bond and phenylene, alternatively selected from chemical bond, 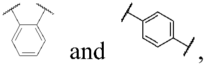 alternatively selected from a chemical bond and 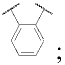 alternatively a chemical bond.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, L₃ is selected from O, S and NR'; alternatively selected from O and NR'; alternatively is O and S; alternatively is O; alternatively is NR'.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, n is selected from 0, 1, 2 and 3, alternatively selected from 1, 2 and 3, alternatively 1 or 2, alternatively 1.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, the methylene group in 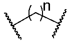 is optionally substituted with 1, 2, or 3 independent R, alternatively optionally substituted with 1 R.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ; alternatively selected from H, D, =O, C₁₋₆ alkyl, C₁₋₆ haloalkyl, -C₀₋₆ alkylene-C(O)ORₐ and -C₀₋₆ alkylene-OC(O)Rₐ; alternatively selected from H, D, =O, C₁₋₆ alkyl, C₁₋₆ haloalkyl and -C₀₋₆ alkylene-C(O)ORₐ; alternatively selected from H, D, =O, C₁₋₃ alkyl, C₁₋₃ haloalkyl and -C₀₋₆ alkylene-C(O)ORₐ; alternatively selected from H, D, =O, C₁₋₃ alkyl and-C₀₋₆ alkylene-C(O)ORₐ; alternatively selected from H, D, =O, C₁₋₃ alkyl and -C₀₋₃ alkylene-C(O)ORₐ; alternatively selected from H, D, =O and -C₀₋₃ alkylene-C(O)OH.

In a more specific embodiment, R is independently selected from H, =O, methyl, isobutyl, -C(O)OH and -(CH₂)₂C(O)OH; alternatively selected from H, =O, methyl, -C(O)OH and -(CH₂)₂C(O)OH; alternatively selected from H, =O, methyl and -(CH₂)₂C(O)OH; alternatively selected from H, =O and -(CH₂)₂C(O)OH.

In a more specific embodiment, R is independently selected from H, D, halogen, C₁₋₆ alkyl and C₁₋₆ haloalkyl; alternatively H, D, C₁₋₆ alkyl and C₁₋₆ haloalkyl; alternatively selected from H, D, C₁₋₃ alkyl and C₁₋₃ haloalkyl; alternatively selected from H and Me.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R' is selected from H, C₁₋₁₀ alkyl, C₁₋₁₀ haloalkyl, C₃₋₁₀ cycloalkyl and 3- to 10-membered heterocyclyl, alternatively selected from H, C₁₋₆ alkyl and C₁₋₆ haloalkyl, alternatively H.

In a more specific embodiment, R' and R are taken together with the atoms to which they are attached to form 3- to 10-membered heterocyclyl, alternatively to form 3- to 7-membered heterocyclyl, alternatively to form 4- to 6-membered heterocyclyl, alternatively to form 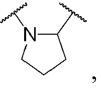 alternatively to form

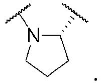

In a more specific embodiment, R' and R are not taken together with the atoms to which they are attached to form a ring.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, Rₐ, R_{b} and R_{c} are each independently selected from H, C₁₋₆ alkyl and C₁₋₆ haloalkyl, alternatively H.

In a more specific embodiment, R_{b} and R_{c} are taken together with the atoms to which they are attached to form 3- to 7-membered heterocyclyl.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R_{D} is 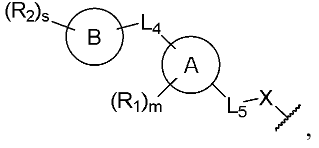 alternatively 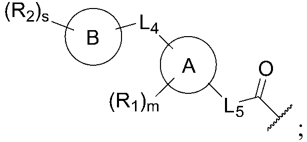 wherein,
ring A is selected from C₆₋₁₀ arylene, 5- to 10-membered heteroarylene, C₆₋₁₀ aryl fused C₅₋₁₀ cycloalkyl, C₆₋₁₀ aryl fused 5- to 10-membered heterocyclyl, 5- to 10-membered heteroaryl fused C₅₋₁₀ cycloalkyl and 5- to 10-membered heteroaryl fused 5- to 10-membered heterocyclyl;
ring B is selected from C₃₋₁₀ cycloalkyl, 3- to 10-membered heterocyclyl, C₆₋₁₀ aryl and 5- to 10-membered heteroaryl, alternatively selected from C₆₋₁₀ aryl and 5- to 10-membered heteroaryl, or -L₄-ring B and R₂ are absent;
R₁ is independently selected from H, D, halogen, CN, -NO₂, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, -S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl, which is optionally substituted with 1, 2, or 3 independent R₁ₛ;
R₁ₛ is independently selected from H, D, halogen, CN, -ORₐ, -SRₐ, -NR_{b}R_{c}, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl and 3- to 10-membered heterocyclyl;
m=0, 1, 2, 3, 4 or 5;
R₂ is independently selected from H, D, halogen, CN, -NO₂, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, -S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl, which is optionally substituted with 1, 2, or 3 independent R₂ₛ;
R₂ₛ is independently selected from H, D, halogen, CN, -ORₐ, -SRₐ, -NR_{b}R_{c}, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl and 3- to 10-membered heterocyclyl;
s=0, 1, 2, 3, 4 or 5;
L₄ is selected from chemical bond, -CR₄R'₄-, -NR_{b}-, O, S, -C(O)-, -OC(O)-, -C(O)O-, -NR_{b}C(O)-, -C(O)NR_{b}-, -S(O)- and -S(O)₂-;
or, L₄ and the carbon atom on ring A to which L₄ is attached are taken together to form 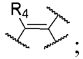
L₅ is selected from chemical bond, O, S, NR' and C₁₋₁₀ alkylene, which is optionally substituted with one or more independent R₃;
R₃ is independently selected from H, D, halogen, C₁₋₁₀ alkyl and C₁₋₁₀ haloalkyl;
R₄ and R'₄ are independently selected from H, D, halogen, C₁₋₆ alkyl and C₁₋₆ haloalkyl;
X is selected from -C(O)-, -S(O)- and -S(O)₂-, alternatively -C(O)-.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, ring A is selected from C₆₋₁₀ arylene and 5- to 10-membered heteroarylene; alternatively selected from phenylene and 9- to 10-membered heteroarylene; alternatively selected from phenylene and indolylene; alternatively is phenylene; alternatively selected from 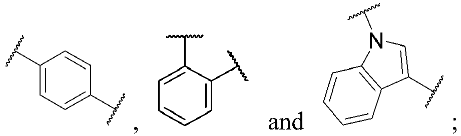 alternatively selected from 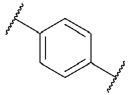 and 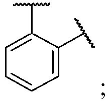 alternatively

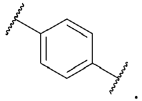

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, ring B is selected from C₃₋₇ cycloalkyl, 3- to 7-membered heterocyclyl, phenyl and 5-to 6-membered heteroaryl; alternatively selected from phenyl and 5- to 6-membered heteroaryl; alternatively is C₆₋₁₀ aryl, alternatively phenyl.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R₁ is independently selected from H, D, halogen, CN, -NO₂, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, -S(O)Rₐ, -S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c}, -C(O)Rₐ, -OC(O)Rₐ, - C(O)ORₐ, -NR_{b}C(O)Rₐ, -C(O)NR_{b}R_{c}, C₁₋₆ alkyl, C₁₋₆ haloalkyl, C₃₋₇ cycloalkyl and 3- to 7-membered heterocyclyl; alternatively selected from H, D, halogen, CN, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl; alternatively selected from H, D, halogen, CN, -ORₐ, -NR_{b}R_{c}, -OC(O)Rₐ, -C(O)ORₐ, C₁₋₆ alkyl and C₁₋₆ haloalkyl; alternatively selected from H, D, halogen, -ORₐ, -OC(O)Rₐ, C₁₋₆ alkyl and C₁₋₆ haloalkyl; alternatively selected from H, F, Me, -OMe and -OC(O)CH₃.

In a more specific embodiment, R₁ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl; alternatively H, D and halogen; alternatively is H or F; alternatively is halogen; alternatively is F.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R₂ is independently selected from H, D, halogen, CN, -NO₂, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, -S(O)Rₐ, -S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c},-C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c}, -C(O)Rₐ, -OC(O)Rₐ,-C(O)ORₐ, -NR_{b}C(O)Rₐ, -C(O)NR_{b}R_{c}, C₁₋₆ alkyl, C₁₋₆ haloalkyl, C₃₋₇ cycloalkyl, 3- to 7-membered heterocyclyl, phenyl and 5- to 6-membered heteroaryl; alternatively selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ,-C(O)NR_{b}R_{c}, C₁₋₆ alkyl and C₁₋₆ haloalkyl; alternatively selected from H, D, halogen, CN, C₁₋₆ alkyl and C₁₋₆ haloalkyl; alternatively selected from H, D, halogen, C₁₋₆ alkyl and C₁₋₆ haloalkyl; alternatively selected from H, Cl and Me; alternatively selected from H and Cl.

In a more specific embodiment, R₂ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl, alternatively selected from H, D and halogen; yet alternatively is H or D.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R₁ₛ and R₂ₛ are each independently selected from H, D, halogen, CN, C₁₋₁₀ alkyl, C₁₋₁₀ haloalkyl, C₃₋₁₀ cycloalkyl and 3- to 10-membered heterocyclyl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, m=0, 1, 2, 3 or 4; alternatively m=0, 1 or 2; alternatively m=0 or 1; alternatively m=1.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, s=0, 1 or 2; alternatively s=0.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, L₄ is selected from chemical bond, -CR₄R'₄-, -NR_{b}-, O, S, -C(O)- and -S(O)-; alternatively selected from chemical bond, -CR₄R'₄-, -NR_{b}-, O and -C(O)-; alternatively selected from chemical bond, -NR_{b}- and -C(O)-; alternatively selected from chemical bond,-NH- and -C(O)-; alternatively selected from a chemical bond and -NH-; alternatively is a chemical bond.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, L₅ is a chemical bond or C₁₋₁₀ alkylene, alternatively is a chemical bond or C₁₋₆ alkylene, alternatively is a chemical bond or C₁₋₄ alkylene, alternatively is a chemical bond or C₁₋₂ alkylene, alternatively is a chemical bond or methylene, alternatively -CH(CH₃)-;
alternatively, L₅ is optionally substituted with 1, 2, 3, 4, 5, or 6 independent R₃, alternatively optionally substituted with 1, 2, or 3 independent R₃, alternatively, optionally substituted with 1 R₃.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R₃ is independently selected from H, D, halogen, C₁₋₆ alkyl and C₁₋₆ haloalkyl; alternatively selected from H, D, C₁₋₄ alkyl and C₁₋₄ haloalkyl; alternatively selected from H and Me; alternatively is Me.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, R₄ and R'₄ are independently selected from H, D, C₁₋₆ alkyl and C₁₋₆ haloalkyl, alternatively H or D.

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, having the following structure: 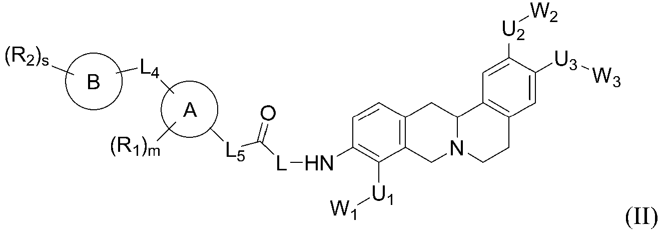 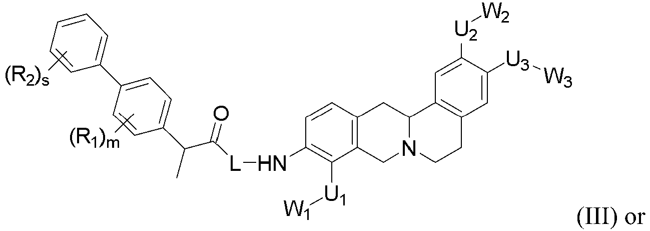 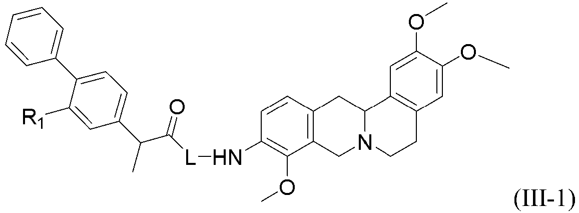 wherein the variables are as defined herein.

In a more specific embodiment, the present disclosure provides a compound of formula (II) described above, or an isotopic variant, a tautomer, a stereoisomer, a prodrug, a polymorph, a hydrate or a solvate thereof, or a pharmaceutically acceptable salt thereof, 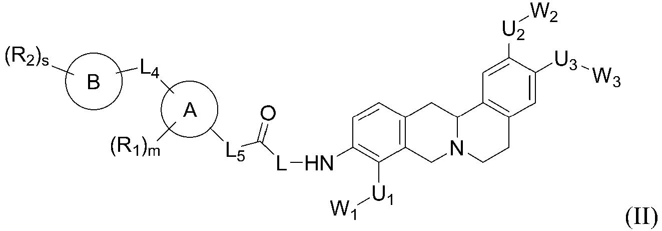 wherein,
ring A is selected from C₆₋₁₀ arylene and 5- to 10-membered heteroarylene;
ring B is selected from C₃₋₁₀ cycloalkyl, 3- to 10-membered heterocyclyl, C₆₋₁₀ aryl and 5- to 10-membered heteroaryl, alternatively selected from C₃₋₇ cycloalkyl, 3- to 7-membered heterocyclyl, phenyl and 5- to 6-membered heteroaryl, or -L₄-ring B and R₂ are absent;
R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from H, D, halogen, CN, -ORₐ, -SRₐ, -NR_{b}R_{c}, -C(O)Rₐ, -OC(O)Rₐ, -C(O)ORₐ,-NR_{b}C(O)Rₐ, -C(O)NR_{b}R_{c}, C₁₋₆ alkyl, C₁₋₆ haloalkyl, C₃₋₇ cycloalkyl and 3- to 7-membered heterocyclyl, which is optionally substituted with 1, 2, or 3 independent R₁ₛ;
R₁ₛ is independently selected from H, D, halogen, CN, C₁₋₁₀ alkyl, C₁₋₁₀ haloalkyl, C₃₋₁₀ cycloalkyl and 3- to 10-membered heterocyclyl, alternatively selected from H, D, halogen, CN, C₁₋₆ alkyl, C₁₋₆ haloalkyl, C₃₋₇ cycloalkyl and 3- to 7-membered heterocyclyl;
m=0, 1, 2, 3, 4 or 5;
R₂ is selected from H, D, halogen, CN, -ORₐ, -SRₐ, -NR_{b}R_{c}, -C(O)Rₐ, -OC(O)Rₐ, - C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ,-C(O)NR_{b}R_{c}, C₁₋₆ alkyl, C₁₋₆ haloalkyl, C₃₋₇ cycloalkyl and 3- to 7-membered heterocyclyl, which is optionally substituted with 1, 2, or 3 independent R₂ₛ;
R₂ₛ is independently selected from H, D, halogen, CN, C₁₋₁₀ alkyl, C₁₋₁₀ haloalkyl, C₃₋₁₀ cycloalkyl and 3- to 10-membered heterocyclyl, alternatively selected from H, D, halogen, CN, C₁₋₆ alkyl, C₁₋₆ haloalkyl, C₃₋₇ cycloalkyl and 3- to 7-membered heterocyclyl;
s=0, 1, 2, 3, 4 or 5;
L₄ is selected from chemical bond, -CR₄R'₄-, -NR_{b}-, O, S, -C(O)- and -S(O)-;
L₅ is a chemical bond or C₁₋₆ alkylene, which is optionally substituted with 1, 2, 3, 4, 5, or 6 independent R₃;
R₃ is independently selected from H, D, halogen, C₁₋₆ alkyl and C₁₋₆ haloalkyl;
R₄ and R'₄ are independently selected from H, D, halogen, C₁₋₆ alkyl and C₁₋₆ haloalkyl;
U₁, U₂ and U₃ are each independently selected from O, S, -NH-, -C(O)-, -O-C(O)-,-NH-C(O)- and -O-CH₂-O-;
W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl;
L is a chemical bond or 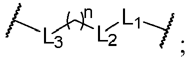
L₁ is selected from chemical bond, -C(O)-, -OC(O)- and -NHC(O)-;
L₂ is selected from chemical bond, C₃₋₇ cycloalkylene, 3- to 7-membered heterocyclylene, phenylene and 5- to 6-membered heteroarylene;
L₃ is selected from O, S and NR';
n is selected from 0, 1, 2, 3, 4, 5 and 6;
the methylene group in 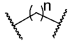 is optionally substituted with 1, 2, or 3 independent R;
R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ, alternatively selected from H, D, =O, C₁₋₆ alkyl, C₁₋₆ haloalkyl, -C₀₋₆ alkylene-C(O)ORₐ and -C₀₋₆ alkylene-OC(O)Rₐ;
R' is selected from H, C₁₋₆ alkyl, C₁₋₆ haloalkyl, C₃₋₇ cycloalkyl and 3- to 7-membered heterocyclyl, or R' and R are taken together with the atoms to which they are attached to form 3- to 7-membered heterocyclyl;
Rₐ, R_{b} and R_{c} are each independently selected from H, C₁₋₆ alkyl and C₁₋₆ haloalkyl, or R_{b} and R_{c} are taken together with the atoms to which they are attached to form 3- to 7-membered heterocyclyl;
wherein each of the above groups is optionally deuterated, up to full deuteration.

In a more specific embodiment, the present disclosure provides a compound of formula (II) described above, or an isotopic variant, a tautomer, a stereoisomer, a prodrug, a polymorph, a hydrate or a solvate thereof, or a pharmaceutically acceptable salt thereof, wherein,
ring A is selected from C₆₋₁₀ arylene and 5- to 10-membered heteroarylene, alternatively selected from phenylene and 9- to 10-membered heteroarylene;
ring B is selected from C₆₋₁₀ aryl and 5- to 10-membered heteroaryl, alternatively selected from phenyl and 5- to 6-membered heteroaryl; alternatively is C₆₋₁₀ aryl, alternatively phenyl, or -L₄-ring B and R₂ are absent;
R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl, alternatively selected from H, D, halogen, CN, -ORₐ, -NR_{b}R_{c}, -OC(O)Rₐ, -C(O)ORₐ, C₁₋₆ alkyl and C₁₋₆ haloalkyl, alternatively selected from H, D, halogen, -ORₐ, -OC(O)Rₐ, C₁₋₆ alkyl and C₁₋₆ haloalkyl, alternatively H, D and halogen, which is optionally substituted with 1, 2, or 3 independent R₁ₛ;
R₁ₛ is independently selected from H, D, halogen, C₁₋₆ alkyl and C₁₋₆ haloalkyl;
m=0, 1, 2, 3, 4 or 5, alternatively m=0, 1 or 2;
R₂ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl, alternatively selected from H, D, halogen, CN, C₁₋₆ alkyl and C₁₋₆ haloalkyl, alternatively selected from H, D, halogen, C₁₋₆ alkyl and C₁₋₆ haloalkyl, alternatively H, D and halogen, which is optionally substituted with 1, 2, or 3 independent R₂ₛ;
R₂ₛ is independently selected from H, D, halogen, C₁₋₆ alkyl and C₁₋₆ haloalkyl;
s=0, 1, 2, 3, 4 or 5, alternatively s=0, 1 or 2;
L₄ is selected from chemical bond, -CR₄R'₄-, -NR_{b}-, O and -C(O)-, alternatively selected from chemical bond, -NR_{b}- and -C(O)-;
L₅ is a chemical bond or C₁₋₄ alkylene, alternatively is a chemical bond or C₁₋₂ alkylene, which is optionally substituted with 1, 2, or 3 independent R₃;
R₃ is independently selected from H, D, C₁₋₄ alkyl and C₁₋₄ haloalkyl;
R₄ and R'₄ are independently selected from H, D, C₁₋₆ alkyl and C₁₋₆ haloalkyl, alternatively selected from H and D;
U₁, U₂ and U₃ are each independently selected from O, S, -NH- and -C(O)-, alternatively O;
W₁, W₂ and W₃ are each independently selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively C₁₋₆ alkyl;
L is a chemical bond or 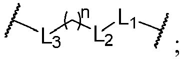
L₁ is selected from chemical bond, -C(O)- and -OC(O)-, alternatively selected from - C(O)- and -OC(O)-, alternatively -C(O)-;
L₂ is selected from chemical bond, phenylene and 5- to 6-membered heteroarylene, alternatively selected from a chemical bond and phenylene;
L₃ is selected from O and NR', alternatively NR'; alternatively is O;
n is selected from 0, 1, 2 and 3, alternatively selected from 1, 2 and 3, alternatively 1 or 2, alternatively 1;
the methylene group in 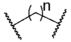 is optionally substituted with 1, 2, or 3 independent R, alternatively optionally substituted with 1 R;
R is independently selected from H, D, =O, C₁₋₆ alkyl, C₁₋₆ haloalkyl and -C₀₋₆ alkylene-C(O)ORₐ, alternatively selected from H, D, =O, C₁₋₃ alkyl, C₁₋₃ haloalkyl and -C₀₋₆ alkylene-C(O)ORₐ, alternatively selected from H, D, =O, C₁₋₃ alkyl and -C₀₋₆ alkylene-C(O)ORₐ, alternatively selected from H, D, =O, C₁₋₃ alkyl and -C₀₋₃ alkylene-C(O)ORₐ, alternatively selected from H, D, =O and -C₀₋₃ alkylene-C(O)OH;
R' is selected from H, C₁₋₆ alkyl and C₁₋₆ haloalkyl, alternatively H, or R' and R are taken together with the atoms to which they are attached to form 4- to 6-membered heterocyclyl; Alternatively, R' and R do not form a ring;
Rₐ, R_{b} and R_{c} are each independently selected from H, C₁₋₆ alkyl and C₁₋₆ haloalkyl.

In a more specific embodiment, the present disclosure provides a compound of formula (II) described above, or an isotopic variant, a tautomer, a stereoisomer, a prodrug, a polymorph, a hydrate or a solvate thereof, or a pharmaceutically acceptable salt thereof, wherein,
ring A is selected from phenylene and indolylene, alternatively selected from 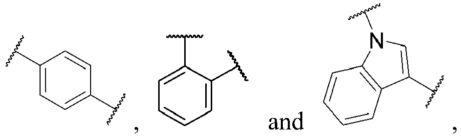 alternatively phenylene, alternatively selected from 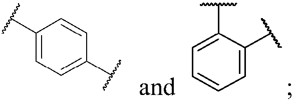
ring B is phenyl, or -L₄-ring B and R₂ are absent;
R₁ is independently selected from H, F, Me, -OMe and -OC(O)CH₃, alternatively H or F;
m=0, 1 or 2;
R₂ is independently selected from H, Cl and Me, alternatively selected from H and Cl;
s=0, 1 or 2;
L₄ is selected from chemical bond, -NH- and -C(O)-; alternatively selected from a chemical bond and -NH-; alternatively is a chemical bond;
L₅ is a chemical bond or methylene, which is optionally substituted with 1 R₃; alternatively, L₅ is -CH(CH₃)-;
R₃ is independently selected from H and Me; alternatively is Me;
U₁, U₂ and U₃ are O;
W₁, W₂ and W₃ are methyl or CD₃, alternatively methyl;
L is a chemical bond or 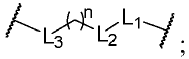
L₁ is selected from chemical bond, -C(O)- and -OC(O)-;
L₂ is selected from chemical bond, 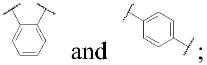
L₃ is selected from O and NR';
n is 0, 1, 2 and 3;
the methylene group in 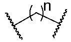 is optionally substituted with 1 R;
R is independently selected from H, =O, methyl, isobutyl, -C(O)OH and - (CH₂)₂C(O)OH, alternatively selected from H, =O, methyl, -C(O)OH and -(CH₂)₂C(O)OH, alternatively selected from H, =O, methyl and -(CH₂)₂C(O)OH, alternatively selected from H, =O and -(CH₂)₂C(O)OH;
R' is H, or R' and R are taken together with the atoms to which they are attached to form 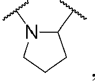 alternatively to form

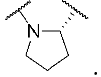

In a more specific embodiment, 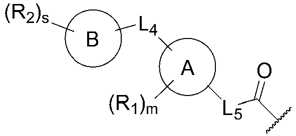 is selected from the following structures: 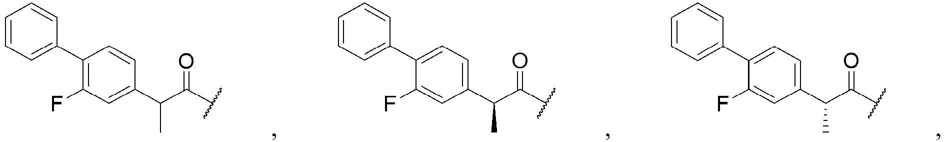 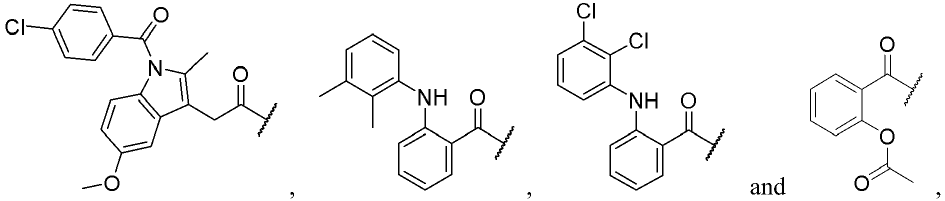 alternatively selected from 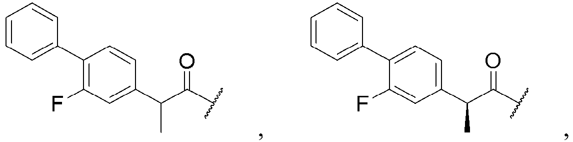 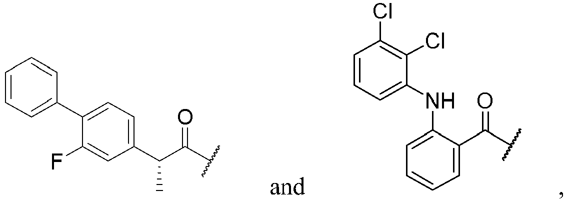 alternatively selected from

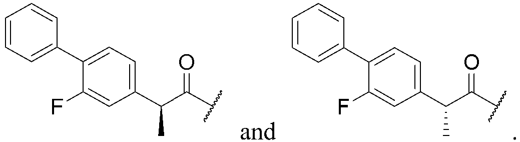

In a more specific embodiment, the present disclosure provides a compound of formula (III) described above, or an isotopic variant, a tautomer, a stereoisomer, a prodrug, a polymorph, a hydrate or a solvate thereof, or a pharmaceutically acceptable salt thereof, 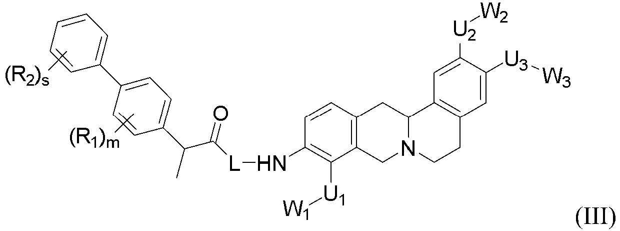 wherein,
R₁ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, which is optionally substituted with 1, 2, or 3 independent R₁ₛ;
R₁ₛ is independently selected from H, D, halogen, CN, C₁₋₁₀ alkyl, C₁₋₁₀ haloalkyl, C₃₋₁₀ cycloalkyl and 3- to 10-membered heterocyclyl;
m=0, 1, 2, 3 or 4;
R₂ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, which is optionally substituted with 1, 2, or 3 independent R₂ₛ;
R₂ₛ is independently selected from H, D, halogen, CN, C₁₋₁₀ alkyl, C₁₋₁₀ haloalkyl, C₃₋₁₀ cycloalkyl and 3- to 10-membered heterocyclyl;
s=0, 1, 2, 3, 4 or 5;
U₁, U₂ and U₃ are each independently selected from O, S, -NH-, -C(O)-, -O-C(O)-, - NH-C(O)- and -O-CH₂-O-;
W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl;
L is a chemical bond or 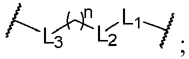
L₁ is selected from chemical bond, -C(O)-, -OC(O)- and -NHC(O)-;
L₂ is selected from chemical bond, C₃₋₇ cycloalkylene, 3- to 7-membered heterocyclylene, phenylene and 5- to 6-membered heteroarylene;
L₃ is selected from O, S and NR';
n is selected from 0, 1, 2, 3, 4, 5 and 6;
the methylene group in 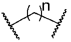 is optionally substituted with 1, 2, or 3 independent R;
R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ, alternatively selected from H, D, =O, C₁₋₆ alkyl, C₁₋₆ haloalkyl, -C₀₋₆ alkylene-C(O)ORₐ and -C₀₋₆ alkylene-OC(O)Rₐ;
R' is selected from H, C₁₋₆ alkyl, C₁₋₆ haloalkyl, C₃₋₇ cycloalkyl and 3- to 7-membered heterocyclyl, or R' and R are taken together with the atoms to which they are attached to form 3- to 7-membered heterocyclyl;
Rₐ, R_{b} and R_{c} are each independently selected from H, C₁₋₆ alkyl and C₁₋₆ haloalkyl, or R_{b} and R_{c} are taken together with the atoms to which they are attached to form 3- to 7-membered heterocyclyl;
wherein each of the above groups is optionally deuterated, up to full deuteration.

In a more specific embodiment, the present disclosure provides a compound of formula (III) described above, or an isotopic variant, a tautomer, a stereoisomer, a prodrug, a polymorph, a hydrate or a solvate thereof, or a pharmaceutically acceptable salt thereof, wherein,
R₁ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl, alternatively H, D and halogen, which is optionally substituted with 1, 2, or 3 independent R₁ₛ;
R₁ₛ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl;
m=0, 1, 2, 3 or 4, alternatively m=0, 1 or 2;
R₂ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl, alternatively selected from H, D and halogen, yet alternatively is H or D, which is optionally substituted with 1, 2, or 3 independent R₂ₛ;
R₂ₛ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl;
s=0, 1, 2, 3, 4 or 5, alternatively s=0, 1 or 2;
U₁, U₂ and U₃ are each independently selected from O, S, -NH- and -C(O)-, alternatively O;
W₁, W₂ and W₃ are each independently selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively C₁₋₆ alkyl;
L is a chemical bond or 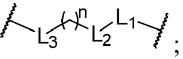
L₁ is selected from chemical bond, -C(O)- and -OC(O)-, alternatively selected from - C(O)- and -OC(O)-, alternatively -C(O)-;
L₂ is selected from chemical bond, phenylene and 5- to 6-membered heteroarylene, alternatively selected from a chemical bond and phenylene;
L₃ is selected from O and NR', alternatively NR'; alternatively is O;
n is selected from 0, 1, 2 and 3, alternatively selected from 1, 2 and 3, alternatively 1 or 2, alternatively 1;
the methylene group in 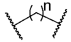 is optionally substituted with 1, 2, or 3 independent R, alternatively optionally substituted with 1 R;
R is independently selected from H, D, =O, C₁₋₆ alkyl, C₁₋₆ haloalkyl and -C₀₋₆ alkylene-C(O)ORₐ, alternatively selected from H, D, =O, C₁₋₃ alkyl, C₁₋₃ haloalkyl and -C₀₋₆ alkylene-C(O)ORₐ, alternatively selected from H, D, =O, C₁₋₃ alkyl and -C₀₋₆ alkylene-C(O)ORₐ, alternatively selected from H, D, =O, C₁₋₃ alkyl and -C₀₋₃ alkylene-C(O)ORₐ, alternatively selected from H, D, =O and -C₀₋₃ alkylene-C(O)OH;
R' is selected from H, C₁₋₆ alkyl and C₁₋₆ haloalkyl, alternatively H, or R' and R are taken together with the atoms to which they are attached to form 4- to 6-membered heterocyclyl; Alternatively, R' and R do not form a ring;
Rₐ is independently selected from H, C₁₋₆ alkyl and C₁₋₆ haloalkyl.

In a more specific embodiment, the present disclosure provides a compound of formula (III) described above, or an isotopic variant, a tautomer, a stereoisomer, a prodrug, a polymorph, a hydrate or a solvate thereof, or a pharmaceutically acceptable salt thereof, wherein,
R₁ is H or F; m=1;
R₂ is H; s=0;
U₁, U₂ and U₃ are O;
W₁, W₂ and W₃ are methyl or CD₃, alternatively methyl;
L is a chemical bond or 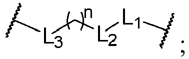
L₁ is selected from chemical bond, -C(O)- and -OC(O)-;
L₂ is selected from chemical bond, 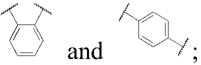
L₃ is selected from O and NR';
n is 0, 1, 2 and 3;
the methylene group in 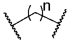 is optionally substituted with 1 R;
R is independently selected from H, =O, methyl, isobutyl, -C(O)OH and - (CH₂)₂C(O)OH, alternatively selected from H, =O, methyl, -C(O)OH and -(CH₂)₂C(O)OH, alternatively selected from H, =O, methyl and -(CH₂)₂C(O)OH, alternatively selected from H, =O and -(CH₂)₂C(O)OH;
R' is H, or R' and R are taken together with the atoms to which they are attached to form 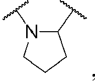 alternatively to form

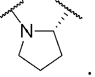

In a more specific embodiment, L is selected from chemical bond, 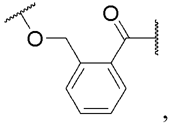 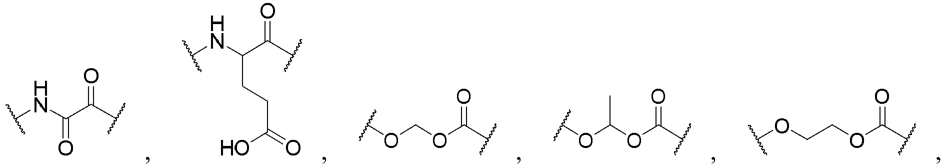 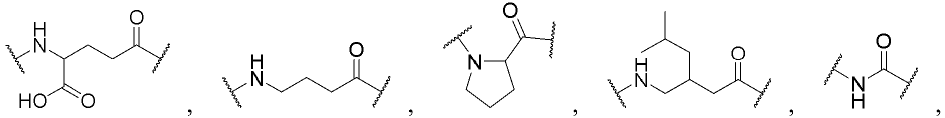 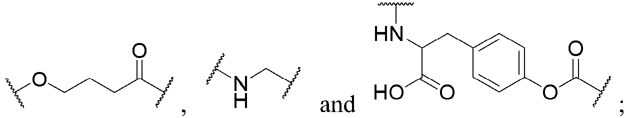 alternatively selected from: a chemical bond, 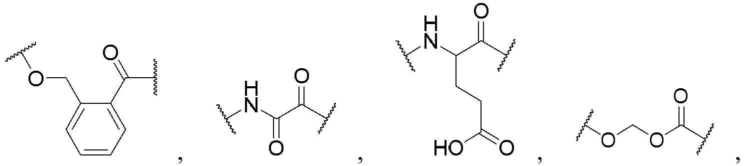 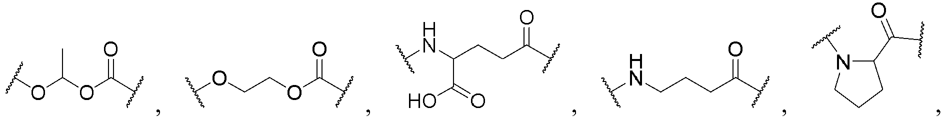 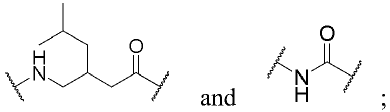 alternatively selected from: a chemical bond, 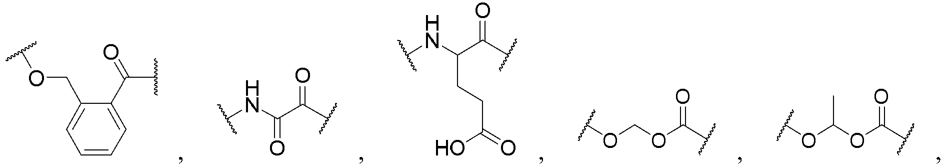 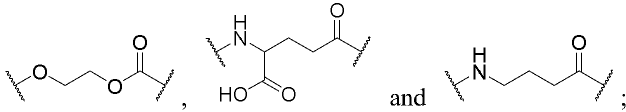 alternatively selected from: a chemical bond, 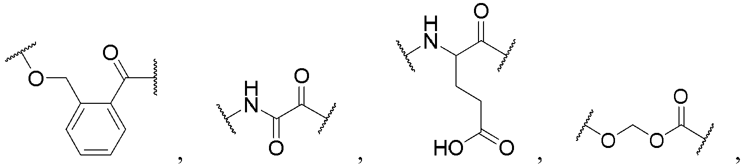 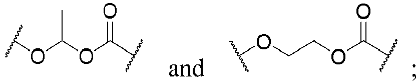 alternatively selected from: a chemical bond,

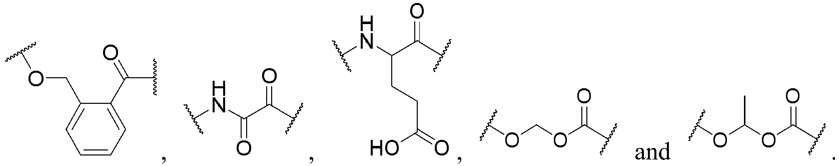

In a more specific embodiment,

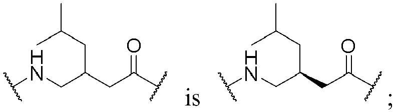

In a more specific embodiment,

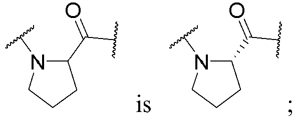

In a more specific embodiment,

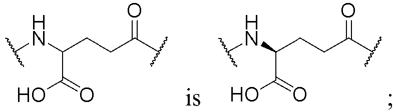

In a more specific embodiment,

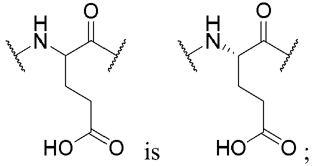

In a more specific embodiment, 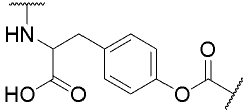 is

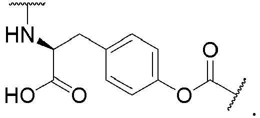

In a more specific embodiment, the present disclosure provides a compound of formula (III-1) described above, or an isotopic variant, a tautomer, a stereoisomer, a prodrug, a polymorph, a hydrate or a solvate thereof, or a pharmaceutically acceptable salt thereof, 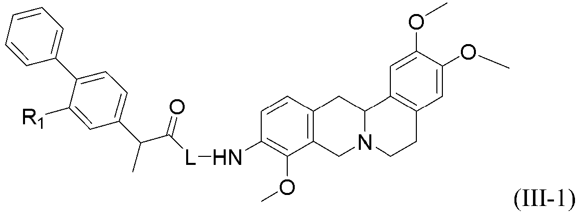 wherein, the variables are as defined herein.

In a more specific embodiment,

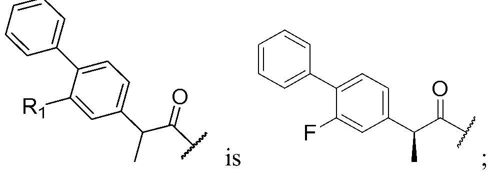

In a more specific embodiment,

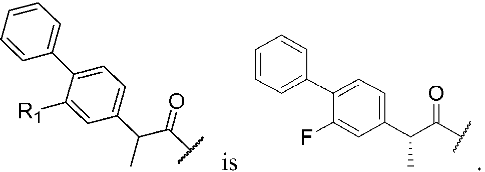

In a more specific embodiment, the present disclosure provides a compound of formula (II), formula (III), or formula (III-1) described above, or an isotopic variant, a tautomer, a stereoisomer, a prodrug, a polymorph, a hydrate or a solvate thereof, or a pharmaceutically acceptable salt thereof, wherein,
L is 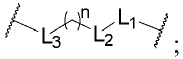
L₁ is selected from -C(O)-, -OC(O)- and -NHC(O)-; alternatively -OC(O)- and - NHC(O)-; alternatively -OC(O)-;
L₂ is a chemical bond;
L₃ is selected from O, S and NR'; alternatively O and S; alternatively is O;
n is selected from 1, 2 and 3, alternatively 1 or 2, alternatively 1;
the methylene group in 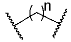 is optionally substituted with 1, 2 or 3 (alternatively 1) independent R;
R is independently selected from H, D, halogen, C₁₋₆ alkyl and C₁₋₆ haloalkyl; alternatively H, D, C₁₋₆ alkyl and C₁₋₆ haloalkyl; alternatively selected from H, D, C₁₋₃ alkyl and C₁₋₃ haloalkyl; alternatively selected from H and Me;
the other groups are defined as in the present disclosure;
alternatively, L is

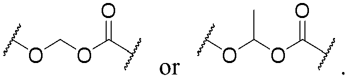

In a more specific embodiment, the present disclosure provides the compound of formula (I) described above, or an isotopic variant, a tautomer, a stereoisomer, a prodrug, a polymorph, a hydrate or a solvate thereof, or a pharmaceutically acceptable salt thereof, wherein, the compound is selected from the group consisting of:

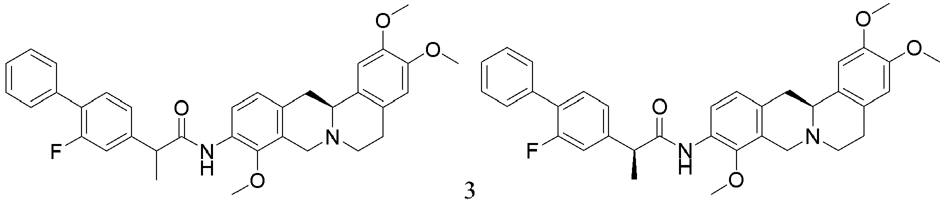

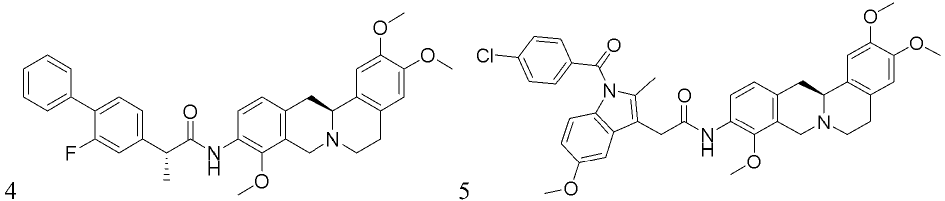

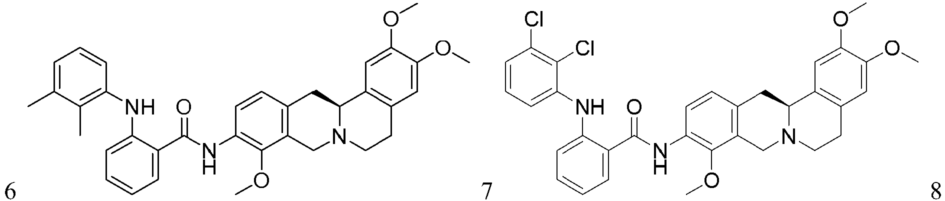

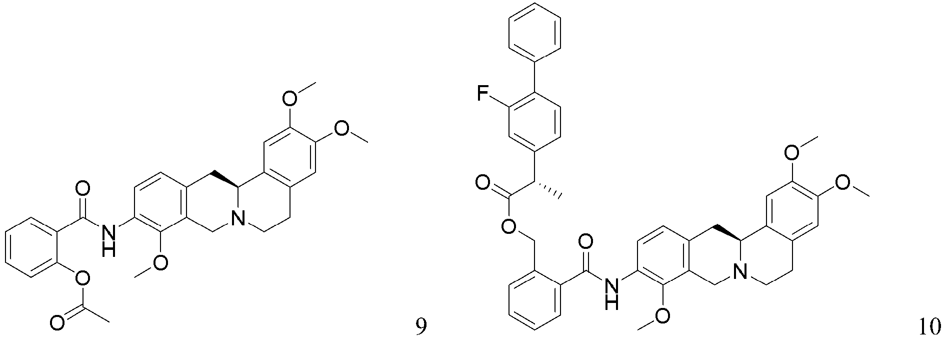

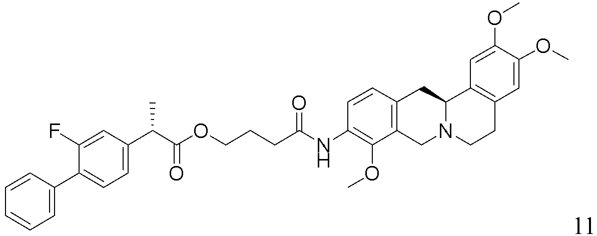

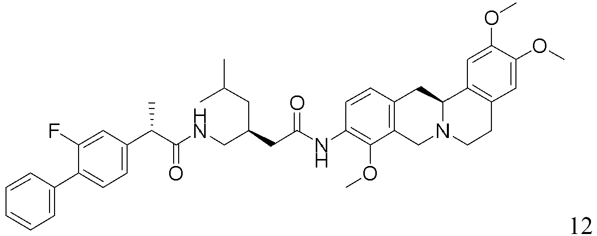

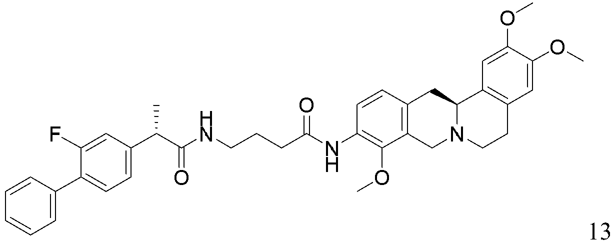

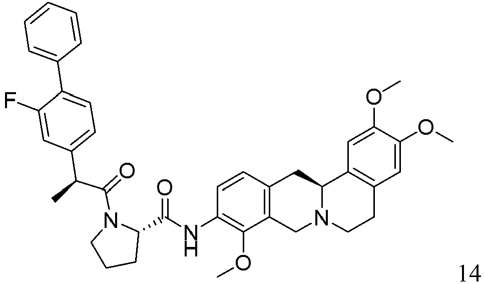

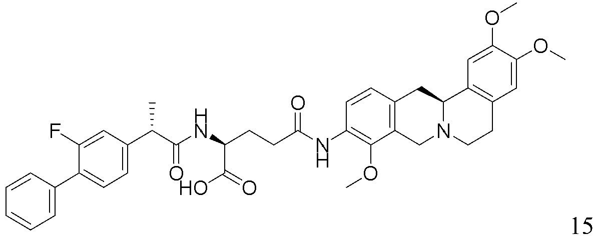

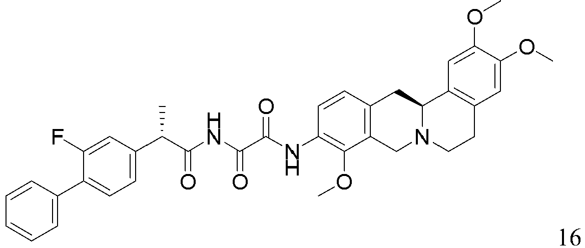

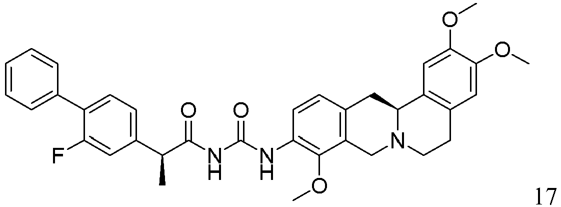

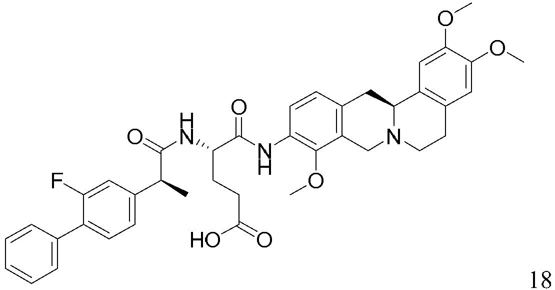

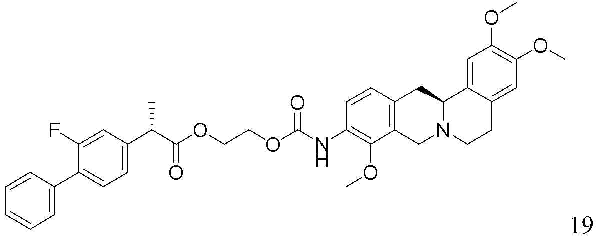

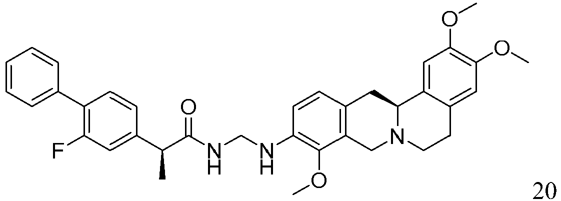

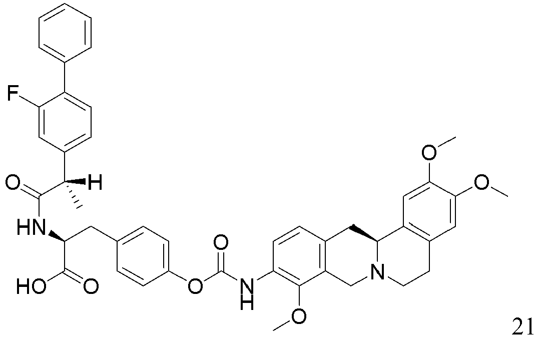

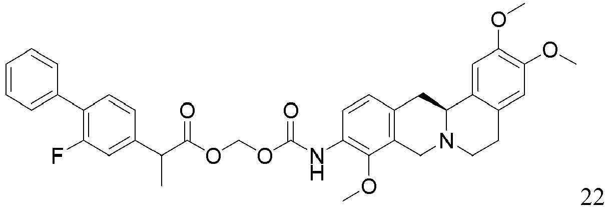

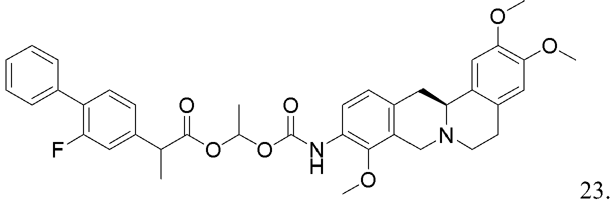

The compounds of the present disclosure may comprise one or more asymmetric centers and may therefore exist in various stereoisomeric forms, e.g., enantiomeric and/or diastereomeric forms. For example, the compounds of the present disclosure may be individual enantiomers, diastereomers, or geometric isomers (e.g., cis and trans isomers), or may be in the form of a mixture of stereoisomers, including racemic mixtures and mixtures enriched in one or more stereoisomers. Isomers may be separated from the mixture by methods known to those skilled in the art, including chiral high-performance liquid chromatography (HPLC) and the formation and crystallization of chiral salts; or alternative isomers may be prepared by asymmetric synthesis.

The compounds of the present disclosure may exist in the form of tautomers. The tautomer is a functional group isomer resulting from the rapid movement of an atom in a molecule between two positions. The tautomer is a special functional group isomer. A pair of tautomers can be interconverted, but usually one isomer that is relatively stable is the main existing form thereof. The most prominent examples are enol and keto tautomers.

Those skilled in the art will appreciate that an organic compound may form a complex with a solvent in which it reacts or from which it precipitates or crystallizes. These complexes are called "solvates". When the solvent is water, the complex is called a "hydrate". The present disclosure encompasses all solvates of the compounds of the present disclosure.

The term "solvate" refers to the form of a compound or a salt thereof in association with a solvent, usually formed by a solvolysis reaction. This physical association may involve hydrogen bonding. Conventional solvents include water, methanol, ethanol, acetic acid, DMSO, THF, diethyl ether, etc. The compounds described herein can be prepared, for example, in a crystalline form, and can be solvated. Suitable solvates include pharmaceutically acceptable solvates and further include stoichiometric solvates and non-stoichiometric solvates. In some cases, the solvate will be capable of isolation, for example, when one or more solvent molecules are incorporated in the crystal lattice of the crystalline solid. The "solvate" includes both solution-phase and isolatable solvates. Representative solvates include hydrates, ethanolates and methanolates.

The term "hydrate" refers to a compound in combination with water. Usually, the ratio of the number of water molecules contained in a hydrate of a compound to the number of molecules of the compound in the hydrate is determined. Therefore, the hydrate of a compound can be represented, for example, by general formula R·x H₂O, wherein R is the compound, and x is a number greater than 0. A given compound may form more than one type of hydrate, including, for example, a monohydrate (x is 1), a lower hydrate (x is a number greater than 0 and less than 1, for example, a hemihydrate (R·0.5 H₂O)) and a polyhydrate (x is a number greater than 1, for example, a dihydrate (R·2 H₂O) and a hexahydrate (R·6 H₂O)).

The compounds of the present disclosure may be in amorphous or crystalline forms (polymorphs). Furthermore, the compounds of the present disclosure may exist in one or more crystalline forms. Therefore, the present disclosure includes within its scope all amorphous or crystalline forms of the compounds of the present disclosure. The term "polymorph" refers to a crystalline form of a compound (or a salt, hydrate, or solvate thereof) in a specific crystal packing arrangement. All polymorphs have the same elemental composition. Different crystalline forms usually have different X-ray diffraction patterns, infrared spectra, melting points, density, hardness, crystal shape, optoelectronic properties, stability and solubility. The recrystallization solvent, crystallization rate, storage temperature and other factors may cause one crystalline form to predominate. Various polymorphs of a compound can be prepared by crystallization under different conditions.

The present disclosure further comprises isotopically labeled compounds (isotopic variants), which are equivalent to those described in formula (I), except that one or more atoms are replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The compounds of the present disclosure containing the above isotopes and/or other isotopes of other atoms, prodrugs thereof, and pharmaceutically acceptable salts of the compounds or prodrugs are all within the scope of the present disclosure. Certain isotopically labeled compounds of the present disclosure, such as those into which radioisotopes (e.g., ³H and ¹⁴C) are incorporated, are useful in drug and/or substrate tissue distribution assays. Tritium (i.e., ³H) and carbon-14 (i.e., ¹⁴C) isotopes are particularly alternative for their ease of preparation and detectability. Furthermore, substitution with heavier isotopes such as deuterium (i.e., ²H) may afford therapeutic benefits (e.g., increased *in vivo* half-life or reduced dose) resulting from greater metabolic stability and hence may be alternative in some circumstances. Isotopically labeled compounds of formula (I) of the present disclosure and prodrugs thereof can generally be prepared by using readily available isotopically labeled reagents to replace non-isotopically labeled reagents in the following procedures and/or the processes disclosed in the examples and preparation examples.

Furthermore, prodrugs are also included within the context of the present disclosure. As used herein, the term "prodrug" refers to a compound that is converted in vivo by hydrolysis, for example, in the blood, to its active form that has a medical effect. Pharmaceutically acceptable prodrugs are described in T. Higuchi and V. Stella, Prodrugs as Novel Delivery Systems, A.C.S. Symposium Series, Vol. 14; Edward B. Roche, ed., Bioreversible Carriers in Drug Design, American Pharmaceutical Association and Pergamon Press, 1987; and D. Fleisher, S. Ramon and H. Barbra, "Improved oral drug delivery: solubility limitations overcome by the use of prodrugs", Advanced Drug Delivery Reviews (1996) 19(2) 115-130, each of which is incorporated herein by reference.

A prodrug is any covalently bonded compound of the present disclosure which, when administered to a patient, releases the parent compound in vivo. Prodrugs are generally prepared by modifying functional groups in such a way that the modification can be made by conventional manipulation or cleavage in vivo to produce the parent compound. Prodrugs include, for example, the compounds of the present disclosure in which a hydroxyl, amino or sulfhydryl group is bonded to any group that can be cleaved to form the hydroxyl, amino or sulfhydryl group when the compounds are administered to a patient. Thus, representative examples of prodrugs include, but are not limited to, acetate/amide, formate/amide and benzoate/amide derivatives of hydroxyl, sulfhydryl and amino functional groups of the compound of formula (I). In addition, in the case of carboxylic acid (-COOH), esters such as methyl ester and ethyl ester can be used. The ester itself can be active and/or can be hydrolyzed under the conditions in the human body. Suitable pharmaceutically acceptable in vivo hydrolysable ester groups include those that break down readily in the human body to release the parent acid or a salt thereof.

The present disclosure further provides a pharmaceutical formulation comprising a therapeutically effective amount of the compound of formula (I) or a therapeutically acceptable salt thereof and a pharmaceutically acceptable carrier, diluent, or excipient. All of these forms are within the present disclosure.

### Pharmaceutical composition and kit

In another aspect, the present disclosure provides a pharmaceutical composition comprising the compound of the present disclosure (also referred to as the "active ingredient") and a pharmaceutically acceptable excipient. In some embodiments, the pharmaceutical composition comprises an effective amount of the compound of the present disclosure. In some embodiments, the pharmaceutical composition comprises a therapeutically effective amount of the compound of the present disclosure. In some embodiments, the pharmaceutical composition comprises a prophylactically effective amount of the compound of the present disclosure.

A pharmaceutically acceptable excipient for use in the present disclosure refers to a non-toxic carrier, adjuvant, or vehicle that does not destroy the pharmacological activity of the compound being formulated together. Pharmaceutically acceptable carriers, adjuvants, or vehicles that may be used in the composition of the present disclosure include, but are not limited to, ion exchangers, aluminium oxide, aluminium stearate, lecithin, serum proteins (e.g., human serum albumin), buffer substances (e.g., phosphates), glycine, sorbic acid, potassium sorbate, partial glyceride mixtures of saturated vegetable fatty acids, water, salts or electrolytes (e.g., protamine sulfate), disodium hydrogen phosphate, potassium hydrogen phosphate, sodium chloride, zinc salts, silica gel, magnesium trisilicate, polyvinylpyrrolidone, cellulose-based substances, polyethylene glycol, sodium carboxymethylcellulose, polyacrylates, waxes, polyethylene-polyoxypropylene-block polymers, polyethylene glycol, and lanolin.

Suitable preparations for administering the compounds of the present disclosure will be apparent to those of ordinary skill in the art and include, for example, tablets, pills, capsules, suppositories, troches, lozenges, solutions (particularly solutions for injection (subcutaneous, intravenous, and intramuscular) and infusion (injectables)), elixirs, syrups, cachets, emulsions, inhalants or dispersible powders. The content of the pharmaceutically active compound(s) should be in the range of 0.1 to 90 wt%, alternatively 0.5 to 50 wt%, of the composition as a whole, i.e., in an amount sufficient to achieve the dosage range specified below. If necessary, the specified dose may be administered several times a day.

The present disclosure further comprises a kit (e.g., a pharmaceutical package). The provided kit may include the compounds of the present disclosure, an additional therapeutic agent, as well as first and second containers (such as vials, ampoules, bottles, syringes and/or dispersible packages or other suitable containers) containing the compounds of the present disclosure and an additional therapeutic agent. In some embodiments, the provided kit may optionally further include a third container containing a pharmaceutical excipient for diluting or suspending the compounds of the present disclosure and/or an additional therapeutic agent. In some embodiments, the compounds of the present disclosure and an additional therapeutic agent provided in the first container and the second container are combined to form a unit dosage form.

### Administration

The pharmaceutical composition provided by the present disclosure may be given via many routes, including but not limited to: oral administration, parenteral administration, inhalation administration, topical administration, rectal administration, nasal administration, buccal administration, vaginal administration, administration via an implant, or other routes of administration. For example, the parenteral administration as used herein includes subcutaneous administration, intradermal administration, intravenous administration, intramuscular administration, intra-articular administration, intra-arterial administration, intrasynovial administration, intrasternal administration, intracerebrospinal administration, intralesional administration, and intracranial injection or infusion techniques.

Typically, an effective amount of the compound provided herein is administered. The actual amount of the compound administered can be determined by a physician according to the relevant circumstances, including the condition being treated, the route of administration chosen, the compound actually administered, the age, weight and response of an individual patient, the severity of the patient's symptoms, etc.

When used to prevent the condition of the present disclosure, the compound provided herein is administered to a subject at risk of developing the condition, typically based on the advice and under the supervision of a physician, at dosage levels as described above. Subjects at risk of developing a specific condition generally include those with a family history of the condition or those identified through genetic testing or screening as being particularly susceptible to developing the condition.

The pharmaceutical composition provided herein can also be administered chronically ("chronic administration"). Chronic administration refers to administration of a compound or pharmaceutical composition thereof over an extended period of time, e.g., 3 months, 6 months, 1 year, 2 years, 3 years, 5 years, and so on, or may continue indefinitely, e.g., for the remainder of the subject's life. In some embodiments, chronic administration is intended to provide a constant level of the compound in the blood over an extended period of time, e.g., within a therapeutic window.

Various methods of administration may be used to further deliver the pharmaceutical compositions of the present disclosure. For example, in some embodiments, the pharmaceutical composition can be administered by bolus, for instance, in order to increase the concentration of the compound in the blood to an effective level. The bolus dose depends on the target systemic level of the active ingredient in the body. For example, an intramuscular or subcutaneous bolus dose results in a slow release of the active ingredient, while a bolus administered directly into a vein (e.g., via an intravenous (IV) drip) enables a faster delivery, causing the concentration of the active ingredient in the blood to rise rapidly to an effective level. In other embodiments, the pharmaceutical composition may be administered as a continuous infusion, for example, via an intravenous (IV) drip, so as to provide a steady-state concentration of the active ingredient in the subject's body. Furthermore, in other embodiments, a bolus dose of the pharmaceutical composition may be administered first, followed by a continuous infusion.

Oral compositions may take the form of bulk liquid solutions or suspensions, or bulk powders. More typically, however, for the convenience of precise dosing, the composition is provided in unit dosage form. The term "unit dosage form" refers to physically discrete units suitable as unitary dosages for human patients and other mammals, each unit containing a predetermined amount of active material suitable to produce the desired therapeutic effect, in association with a suitable pharmaceutical excipient. Typical unit dosage forms include prefilled and pre-measured ampoules or syringes for liquid compositions, or pills, tablets, capsules and the like in the case of solid compositions. In such compositions, the compound typically constitutes a minor component (about 0.1 to about 50% by weight, or alternatively about 1 to about 40% by weight), with the remainder consisting of various carriers or excipients and processing aids useful for forming the desired administration form.

For oral dosing, a representative regimen is one to five oral doses, especially two to four oral doses, typically three oral doses per day. Using these dosing regimens, each dose provides about 0.01 to about 20 mg/kg of the compound of the present disclosure, alternatively each dose provides about 0.1 to about 10 mg/kg, especially about 1 to about 5 mg/kg.

In order to provide blood levels similar to those achieved with an injection dose, or blood levels lower than those with an injection dose, a transdermal dose is typically selected in an amount of about 0.01 to about 20% by weight, alternatively about 0.1 to about 20% by weight, alternatively about 0.1 to about 10% by weight, and yet alternatively about 0.5 to about 15% by weight.

The injection dosage level ranges from about 0.1 mg/kg/hour to at least 10 mg/kg/hour from about 1 to about 120 hours, especially from 24 to 96 hours. To achieve adequate steady-state levels, a pre-loading bolus of about 0.1 mg/kg to about 10 mg/kg or more may also be administered. For a human patient weighing 40 to 80 kg, the maximum total dose should not exceed approximately 2 g/day.

Liquid forms suitable for oral administration may include suitable aqueous or nonaqueous carriers, as well as buffering agents, suspending agents and dispersing agents, coloring agents, flavoring agents, and the like. Solid forms may include, for example, any of the following components, or compounds with similar properties: binders, such as microcrystalline cellulose, tragacanth gum or gelatin; excipients, such as starch or lactose; disintegrants, such as alginic acid, Primogel or corn starch; lubricants, such as magnesium stearate; glidants, such as colloidal silica; sweeteners, such as sucrose or saccharin; or flavoring agents, such as peppermint, methyl salicylate, or orange flavoring agents.

Injectable compositions are typically based on injectable sterile saline or phosphate-buffered saline, or other injectable excipients known in the art. As described above, in such compositions, the active compound typically constitutes a minor component, usually about 0.05 to 10% by weight, with the remainder consisting of injectable excipients and the like.

Transdermal compositions are typically formulated as a topical ointment or cream containing the active ingredient. When formulated as an ointment, the active ingredient is typically combined with paraffin or water-miscible ointment bases. Alternatively, the active ingredient may be formulated as a cream with, for example, an oil-in-water cream base. Such a transdermal preparation is well known in the art and generally includes other components for enhancing the stable skin penetration of the active ingredient or the preparation. All such known transdermal preparations and components are included within the scope provided by the present disclosure.

The compounds of the present disclosure may also be administered via transdermal devices. Thus, transdermal administration may be accomplished using patches of the reservoir or porous membrane type, or a variety of solid matrices.

The above-mentioned components of the compositions for oral administration, injection or topical administration are merely representative. Additional materials and processing techniques are described in Section 8 of Remington's Pharmaceutical Sciences, 17th edition, 1985, Mack Publishing Company, Easton, Pennsylvania, which is incorporated herein by reference.

The compounds of the present disclosure may also be administered in sustained release form or from a sustained release drug delivery system. Descriptions of representative sustained release materials can be found in Remington's Pharmaceutical Sciences.

The present disclosure further relates to pharmaceutically acceptable preparations of the compounds of the present disclosure. In one embodiment, the preparation comprises water. In another embodiment, the preparation comprises a cyclodextrin derivative. The most common cyclodextrins are α-, β-, and γ-cyclodextrins composed of 6, 7, and 8 α-1,4-linked glucose units, respectively, which optionally include one or more substituents on the linked sugar moieties, including but not limited to: methylated, hydroxyalkylated, acylated, and sulfoalkyl ether substitutions. In some embodiments, the cyclodextrin is a sulfoalkyl ether β-cyclodextrin, such as sulfobutyl ether β-cyclodextrin, also known as Captisol. See, e.g., U.S. 5,376,645. In some embodiments, the preparation includes hexapropyl-β-cyclodextrin (e.g., 10-50% in water).

### Indication

The compounds of the present disclosure exert therapeutic effects through sedation or sleep aid, providing therapeutic benefits to a large number of patients with central nervous system related diseases. The compounds of the present disclosure also possess excellent analgesic, antidepressant, and anti-addiction effects, and can be used to prepare analgesic, antidepressant, and anti-addiction drugs.

In one embodiment, the pain is selected from the group consisting of neuralgia, perioperative pain (including preoperative, intraoperative or post-operative pain, such as somatic or visceral pain caused by postoperative trauma or surgical incisions, pain caused by visceral injury, and other comprehensive types of pain), and cancer pain (such as pain caused by cancer).

In one embodiment, the pain is acute pain or chronic pain (including acute/chronic pain before, during, or after surgery, acute neuralgia, or chronic neuralgia).

In one embodiment, the neuralgia is central pain, such as spinal pain, thalamic pain, pontine pain, medullary pain, or cerebral cortical pain.

In one embodiment, the postoperative pain is pain caused by surgery, such as pain resulting from abdominal surgery, orthopedic surgery, cesarean section, or brain surgery or other surgery.

### Example

The technical solutions of the present disclosure will now be clearly and completely described with reference to the accompanying drawings. Obviously, the described embodiments are merely some, not all, of the embodiments of the present disclosure. All other examples obtained by those skilled in the art based on the examples of the present disclosure are within the scope of protection of the present disclosure.

### Abbreviations:

PE: petroleum ether;
EA: ethyl acetate;
DMAP: 4-dimethylaminopyridine;
DCM: dichloromethane;
DMF: N, N-dimethylformamide;
DCC: dicyclohexylcarbodiimide;
DBU: 1,8-diazabicyclo[5.4.0]undec-7-ene;
EDCI: 1-ethyl-(3-dimethylaminopropyl)carbodiimide;
DIEA: N,N-diisopropylethylamine;
EtOH: ethanol;
Et3N: triethylamine;
HATU: N,N,N',N'-tetramethyl-O-(7-azabenzotriazol-1-yl)uranium hexafluorophosphate;
ACN: acetonitrile;
THF: tetrahydrofuran;
rt: room temperature;
LCMS: Liquid Chromatography-Mass Spectrometry;
TLC: thin-layer chromatography.

The present disclosure will be described in detail below with reference to the specific examples, and the synthesis method of the compound of the present disclosure is not particularly limited, and can be synthesized by any method known to those skilled in the art.

### Example 1

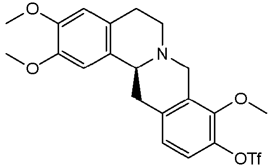

### Synthesis of 1-1

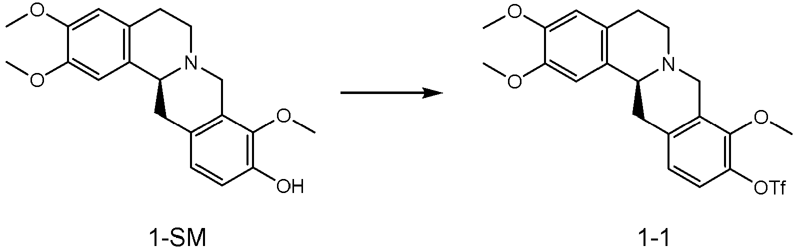

In a 500 ml reaction flask, 1-SM 20.0 g (1.0 eq) and 200 ml dichloromethane were added. 8 g triethylamine was added under a nitrogen atmosphere. 18 g trifluoromethanesulfonic anhydride was added dropwise under ice bath, and the mixture was warmed to room temperature and stirred overnight. LC/MS indicated the reaction was complete. 200 ml of purified water was added to the reaction solution. The mixture was stirred and the layers were separated. The aqueous phase was extracted with 100 ml of dichloromethane. The dichloromethane phases were combined, washed with 150 ml of saturated sodium chloride, dried with anhydrous sodium sulfate, and concentrated by filtration. 330g of residue was purified by silica gel column chromatography to give 1-1 (26.97 g), yield 94.8 %. MS:474. ¹H NMR (400 MHz, DMSO-*d*₆) δ 7.30 (d, *J=* 8.5 Hz, 1H), 7.11 (d, *J =* 8.6 Hz, 1H), 6.89 (s, 1H), 6.70 (s, 1H), 4.13 (d, *J =* 16.1 Hz, 1H), 3.83 (s, 3H), 3.74 (d, *J =* 9.4 Hz, 6H), 3.53 (dd, J = 16.5, 6.6 Hz, 3H), 3.15 (dd, *J=* 11.1, 3.7 Hz, 1H), 2.99 - 2.86 (m, 1H), 2.63 (dd, *J* = 15.6, 9.6 Hz, 3H).

### Example 2

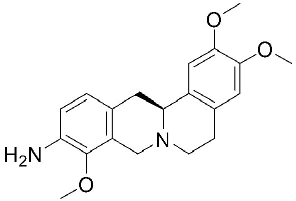

### Synthesis of 3-1

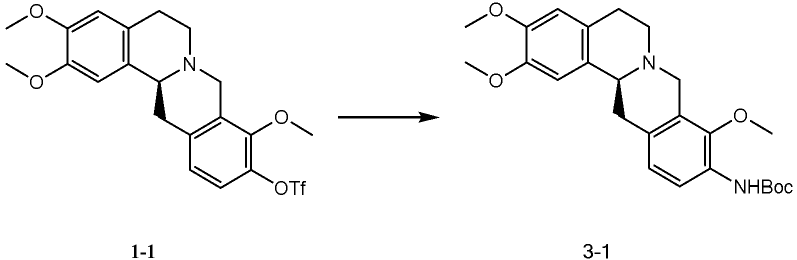

A 1 L reaction flask was charged with 1-1 (20.0 g, 1.0 eq), tert-butyl carbamate, tripotassium phosphate, tris(dibenzylideneacetone)dipalladium(0), 2-(biphenyl)di-tert-butylphosphine, and 200 mL of 1,4-dioxane. The mixture was heated to 90°C under a nitrogen atmosphere and stirred overnight. LC/MS indicated the reaction was complete. The reaction mixture was filtered, and the filter cake was rinsed with 50 mL of EA. The filtrate was concentrated to dryness. The residue was purified by silica gel column chromatography to afford 22.0 g of 3-1 as a yellow solid, which was used directly in the next step without further purification.

### Synthesis of Example 2

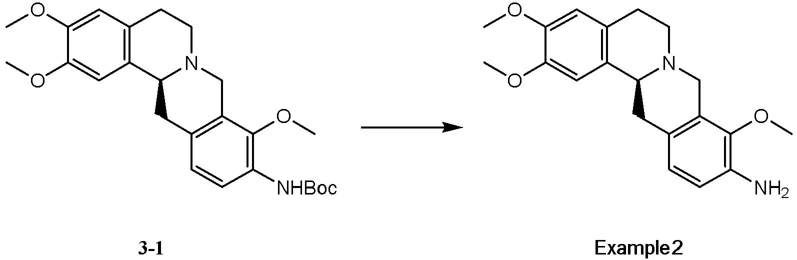

A 50 mL reaction flask was charged with 2.5 g of 3-1 and 10 mL of dichloromethane. A solution of hydrochloric acid in dioxane (10 mL) was added dropwise, and the mixture was stirred at room temperature overnight. LC/MS indicated the reaction was complete. The mixture was concentrated to dryness. Then, 20 mL of DCM was added, followed by 20 mL of saturated sodium carbonate solution to adjust the pH to alkaline. The layers were separated, and the DCM phase was washed with 20 mL of saturated sodium chloride solution, dried over anhydrous sodium sulfate, filtered, and concentrated. The residue was purified by silica gel column chromatography to afford 1.05 g of Example 2 as a solid, with a yield of 54.4%. MS: 326, ¹H NMR (400 MHz, DMSO-*d*₆) δ 6.85 (s, 1H), 6.67(s, 1H), 6.65 (d, *J* = 8.0 Hz, 1H), 6.55 (d, *J= 8.0* Hz, 1H), 4.65 (s, 2H), 4.05 - 4.01 (m, 1H), 3.74 (s, 3H), 3.72 (s, 3H), 3.63 (s, 3H), 3.41 - 3.35 (m, 2H), 3.27 (dd, *J* = 16.0 Hz, 4.0 Hz, 1H), 3.10 (dd, J = 12.0 Hz, 4.0 Hz, 1H), 2.97 - 2.89 (m, 1H), 2.60 (d, *J* = 16.0 Hz, 1H), 2.48-2.42 (m, 2H).

### Example 3

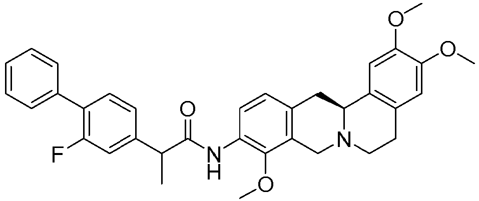

### Synthesis of Example 3

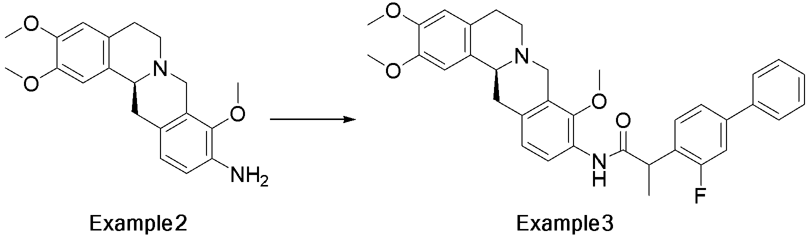

Example 2 (400 mg, 1 eq) was added to 10 mL of DCM. DMAP (158 mg, 1.1 eq) and flurbiprofen (344 mg, 1.2 eq) were added at room temperature, followed by EDCI·HCl (451 mg, 2 eq). The mixture was stirred at room temperature overnight. LCMS indicated no starting material remained. For work-up, the reaction was quenched with water, extracted with DCM, dried over anhydrous sodium sulfate, concentrated, and rotary evaporated to dryness. The residue was purified by Prep-TLC to afford 220 mg of the product. MS: 567, ¹H NMR (400 MHz, DMSO-d6) δ 9.45 (s, 1H), 7.64 (d, J = 8.3 Hz, 1H), 7.50 (dt, J = 22.5, 7.8 Hz, 5H), 7.42 - 7.34 (m, 3H), 6.93 - 6.86 (m, 2H), 6.68 (s, 1H), 4.16 (q, J = 6.9 Hz, 1H), 4.07 (d, J = 15.7 Hz, 1H), 3.74 (s, 3H), 3.72(s, 3H), 3.53 (s, 3H), 3.47 - 3.38 (m, 3H), 3.16 - 3.08 (m, 1H), 2.92 (d, J = 10.8 Hz, 1H), 2.58 (dd, J = 25.3, 14.9 Hz, 3H), 2.47 (d, J = 11.5 Hz, 1H), 1.47 (d, J = 7.0 Hz, 3H).

### Example 4

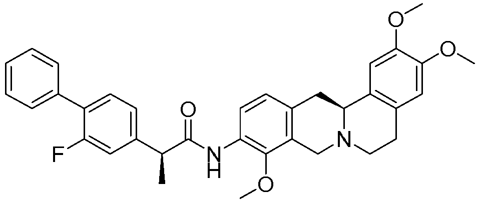

The synthetic method was the same as in Example 3. MS: 567, ¹H NMR (400 MHz, DMSO-d6) δ 9.45 (s, 1H), 7.64 (d, J = 8.3 Hz, 1H), 7.50 (dt, J = 22.5, 7.8 Hz, 5H), 7.42 - 7.34 (m, 3H), 6.93 - 6.86 (m, 2H), 6.68 (s, 1H), 4.16 (q, J = 6.9 Hz, 1H), 4.07 (d, J = 15.7 Hz, 1H), 3.74 (s, 3H), 3.72(s, 3H), 3.53 (s, 3H), 3.47 - 3.38 (m, 3H), 3.16 - 3.08 (m, 1H), 2.92 (d, J = 10.8 Hz, 1H), 2.58 (dd, J = 25.3, 14.9 Hz, 3H), 2.47 (d, J = 11.5 Hz, 1H), 1.47 (d, J = 7.0 Hz, 3H).

### Example 5

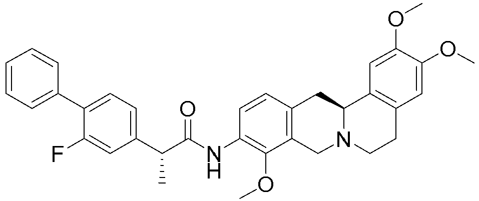

The synthetic method was the same as in Example 3. MS: 567, ¹H NMR (400 MHz, DMSO-d6) δ 9.45 (s, 1H), 7.64 (d, J = 8.3 Hz, 1H), 7.50 (dt, J = 22.5, 7.8 Hz, 5H), 7.42 - 7.34 (m, 3H), 6.93 - 6.86 (m, 2H), 6.68 (s, 1H), 4.16 (q, J = 6.9 Hz, 1H), 4.07 (d, J = 15.7 Hz, 1H), 3.74 (s, 3H), 3.72(s, 3H), 3.53 (s, 3H), 3.47 - 3.38 (m, 3H), 3.16 - 3.08 (m, 1H), 2.92 (d, J = 10.8 Hz, 1H), 2.58 (dd, J = 25.3, 14.9 Hz, 3H), 2.47 (d, J = 11.5 Hz, 1H), 1.47 (d, J = 7.0 Hz, 3H).

### Example 6

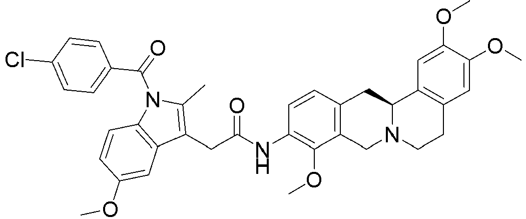

### Synthesis of Example 6

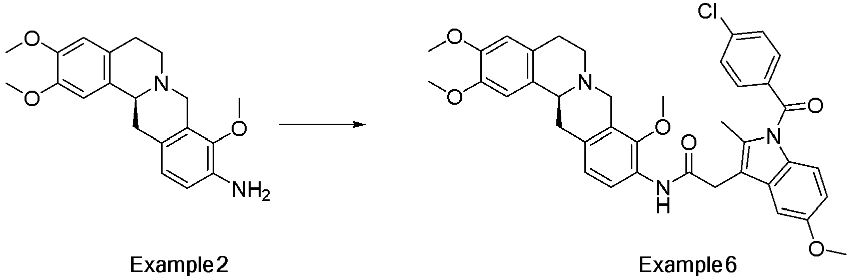

Example 2 (200 mg, 1 eq) was added to 5 mL of DMF. DIEA (759 mg, 10 eq) and indomethacin (252 mg, 1.2 eq) were added at room temperature, followed by the dropwise addition of a solution of HATU (268 mg, 1.2 eq) in DMF. LCMS indicated no starting material remained. The residue purified by reverse-phase column chromatography to afford 282 mg of the product of Example 6. MS: 680, ¹H NMR (400 MHz, DMSO-d6) δ 9.40 (s, 1H), 8.36 (s, 1H), 7.67 (q, J = 8.6, 7.4 Hz, 5H), 7.27 (d, J = 2.0 Hz, 1H), 6.94 (d, J = 9.0 Hz, 1H), 6.89 (d, J = 11.1 Hz, 2H), 6.73 (dd, J = 9.0, 2.5 Hz, 1H), 6.68 (s, 1H), 4.07 (d, J = 15.8 Hz, 1H), 3.87 (s, 2H), 3.77 - 3.72 (m, 9H), 3.59 (s, 3H), 3.46 (s, 1H), 3.42 (d, J = 11.7 Hz, 2H), 3.19 - 3.07 (m, 1H), 2.93 (t, J = 11.2 Hz, 1H), 2.66 - 2.55 (m, 2H), 2.32 (s, 3H).

### Example 7

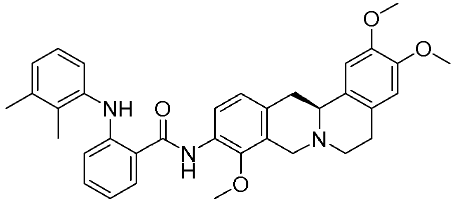

### Synthesis of Example 7

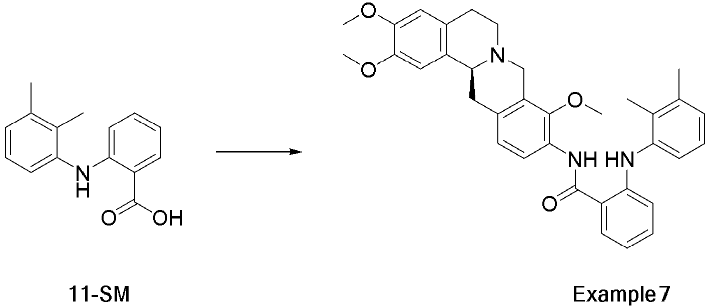

200 mg of Example 2 was dissolved in 4 mL of dichloromethane. Then, 128 mg of 11-SM was added, followed by 195 mg of DMAP under ice-water bath cooling and a nitrogen atmosphere. A solution of 121 mg of DCC in dichloromethane was added to the reaction mixture, and the mixture was stirred at room temperature overnight. The mixture was washed with saturated brine, dried over anhydrous sodium sulfate, filtered, and concentrated. The residue purified by medium-pressure column chromatography, followed by concentration and lyophilization of the eluent, to afford 125.32 mg of the product of Example 7. MS:563, ¹H NMR (400 MHz, DMSO-*d*₆) δ 9.94 (s, 1H), 9.26 (s, 1H), 7.89 (d, *J=* 7.8 Hz, 1H), 7.47 (d, *J =* 8.2 Hz, 1H), 7.32 (t, *J* = 7.1 Hz, 1H), 7.11 - 7.03 (m, 2H), 7.01 - 6.81 (m, 5H), 6.70 (s, 1H), 4.10 (d, *J=* 15.4 Hz, 1H), 3.78 - 3.69 (m, 9H), 3.48 (d, *J=* 13.4 Hz, 3H), 2.68 - 2.56 (m, 2H), 2.27 (s, 3H), 2.09 (s, 3H), 1.24 (s, 5H).

### Example 8

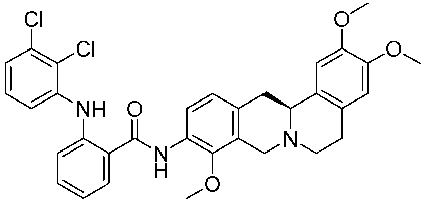

### Synthesis of Example 8

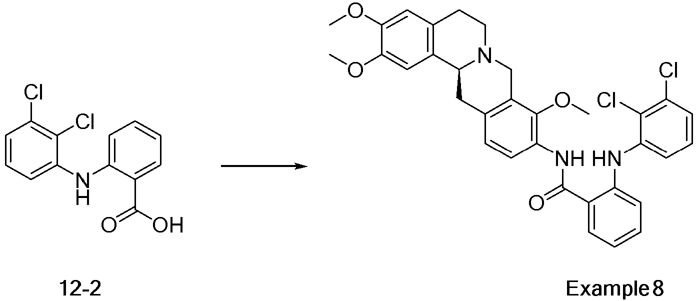

Example 2 (200 mg, 1 eq) was added to 5 mL of DCM. Then, 12-2 (169 mg, 1.02 eq) and DMAP (215 mg, 3 eq) were added at room temperature, followed by the dropwise addition of a solution of DCC (145.4 mg, 1.2 eq) in DCM. The mixture was stirred at room temperature overnight. LCMS indicated no starting material remained. For work-up, the reaction was quenched with water, extracted with DCM, dried over anhydrous sodium sulfate, and concentrated. The residue purified by reverse-phase column chromatography to afford 170 mg of the product of Example 8. MS: 604. ¹H NMR (400 MHz, DMSO-d6) δ 10.04 (s, 1H), 9.72 (s, 1H), 8.34 (s, 1H), 7.95 (d, J = 7.7 Hz, 1H), 7.49 (t, J = 7.6 Hz, 1H), 7.43-7.38 (m, 3H), 7.28 (t, J = 8.1 Hz, 1H), 7.19 (d, J = 7.9 Hz, 1H), 7.10 (t, J = 7.5 Hz, 1H), 6.98 (d, J = 8.2 Hz, 1H), 6.91 (s, 1H), 6.69 (s, 1H), 5.60 (d, J = 8.0 Hz, 1H), 4.10 (d, J = 15.7 Hz, 1H), 3.76(s, 3H), 3.73(s, 3H), 3.69 (s, 3H), 3.51 - 3.44 (m, 3H), 3.36 - 3.29 (m, 1H), 3.14 (d, J = 6.5 Hz, 1H), 2.95 (t, J = 11.2 Hz, 1H), 2.65-2.59 (m, 2H).

### Example 9

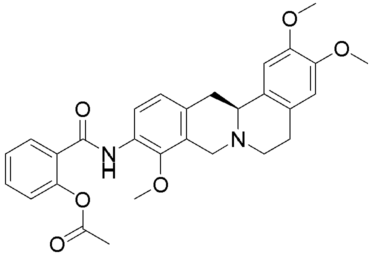

### Synthesis of 14-1

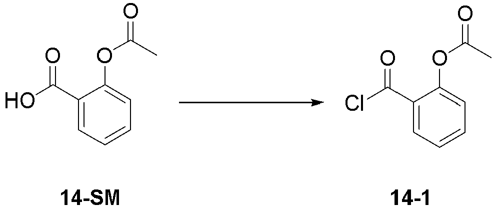

96 mg of 14-SM was dissolved in 4 mL of DCM, and 169 mg of oxalyl chloride was added. The mixture was stirred under ice-water bath cooling. After 5 minutes, the ice-water bath was removed, and the mixture was stirred at room temperature for 30 minutes. The reaction mixture was concentrated, and the concentrate of 14-1 was used directly in the next reaction.

### Synthesis of Example 9

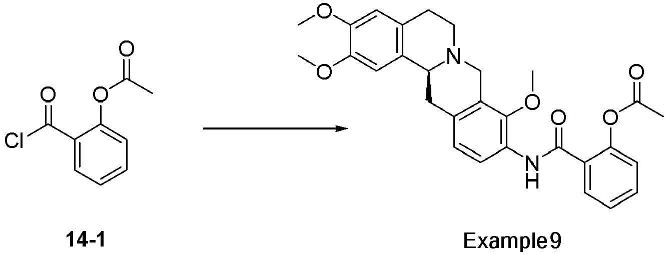

200 mg of **Example 2** was dissolved in 4 mL of DCM, and 593 mg of triethylamine was added. The mixture was stirred at room temperature. The 14-1 obtained in the previous step, dissolved in 2 mL of super-dry DCM, was added dropwise to the above reaction mixture. The mixture was stirred at room temperature for 30 minutes, then washed once each with water and saturated brine, dried over anhydrous sodium sulfate, filtered, and concentrated. The residue purified by silica gel column chromatography to afford 158.21 mg of the product of Example 9 as a solid. MS: 502, mang¹H NMR (400 MHz, DMSO-*d*₆) δ 9.61 (s, 1H), 7.79 (d, *J= 7.4 Hz,* 1H), 7.59 (t, *J =* 6.9 Hz, 2H), 7.41 (t, *J =* 7.6 Hz, 1H), 7.26 (d, *J* = 8.0 Hz, 1H), 6.98 (d, *J =* 8.2 Hz, 1H), 6.90 (d, *J* = 4.5 Hz, 1H), 6.69 (d, *J =* 4.4 Hz, 1H), 4.11 (d, *J =* 15.5 Hz, 1H), 3.84 - 3.71 (m, 12H), 3.46 (s, 3H), 2.64 (d, *J* = 13.8 Hz, 2H), 2.25 (s, 3H), 1.23 (s, 3H).

### Example 10

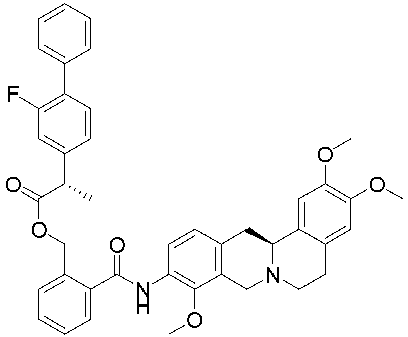

### Synthesis of 15-1

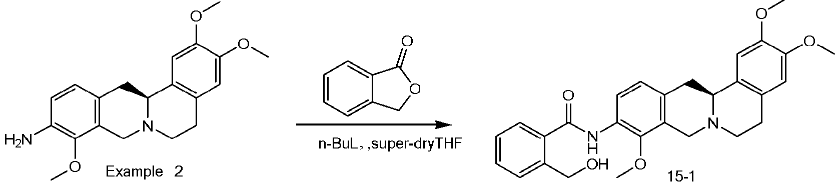

200 mg of Example 2 and 160 mg of isobenzofuranone were dissolved in 5 mL of super-dry THF. Under a nitrogen atmosphere with ice-water bath cooling, 1.1 mL of n-butyllithium solution was added, and the mixture was stirred under ice-water bath cooling. After 30 minutes, the reaction was quenched with water. The mixture was diluted with 50 mL of EA, washed twice with water and once with saturated brine. The mixture was then dried, filtered, and concentrated. Purification: The mixture was diluted with DCM and purified on an 80 g pre-packed silica gel column, eluting with 0-3% MeOH/DCM to afford 320 mg of compound 15-1.

### Synthesis of Example 10

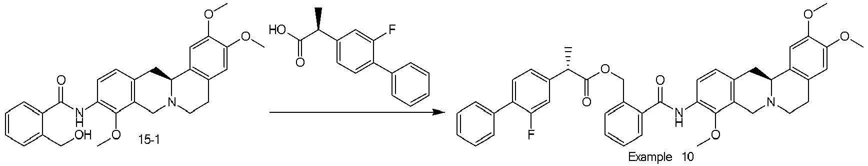

320 mg of 15-1 was dissolved in 5 mL of super-dry DCM. Under ice-water bath cooling, 165 mg of DMAP and 167 mg of DCC were added, and the mixture was stirred under ice-water bath cooling. After 30 minutes, 165 mg of S-flurbiprofen was added, and the mixture was stirred at room temperature overnight. The reaction mixture was filtered, and the filter cake was washed twice with DCM. The organic phase was washed twice with water and once with saturated brine, dried over anhydrous sodium sulfate, filtered, and concentrated. Purification: The mixture was diluted with DCM and purified on a 45 g pre-packed silica gel column, eluting with 0-3% MeOH/DCM. The fractions were collected, concentrated, and lyophilized to afford 138.1 mg of a solid. This solid was further purified by reverse-phase column chromatography and lyophilized to obtain 24.98 mg of Example 10. MS: [M+H] 701; ¹H NMR (400 MHz, DMSO-d6) δ 9.742 (s, 1H), 7.661-7.646 (m, 1H), 7.533-7.375 (m, 10H), 7.253-7.197 (m, 2H), 6.48 (d, J = 8.4 Hz, 1H), 6.889 (s, 1H), 6.696 (s, 1H), 5.384 (d, J = 13.6 Hz, 1H), 5.336 (d, J = 12.8 Hz, 1H), 4.092 (d, J = 15.6 Hz, 1H), 3.939 (q, J = 7.6 Hz, 1H), 3.764 (s, 3H), 3.735 (s, 3H), 3.722 (s, 3H), 3.482-3.426 (m, 3H), 3.150-3.114 (m, 1H), 2.971-2.913 (m, 1H), 2.669-2.564 (m, 3H), 1.440 (d, J = 7.2 Hz, 3H).

### Example 11

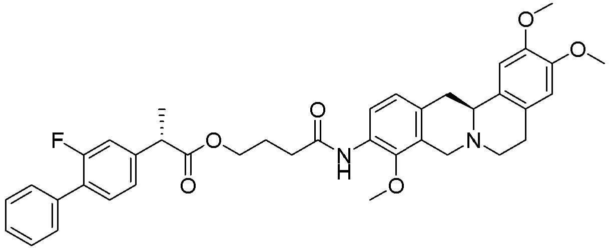

### Synthesis of 18-1

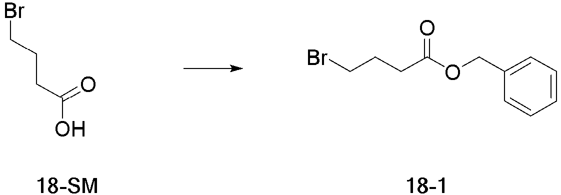

18-SM (300 mg, 1 eq) was added to DMF. After complete dissolution, HATU (762 mg, 1.1 eq) and DIEA (938 mg, 4 eq) were added. The mixture was stirred at 40°C for 1 hour. Benzyl alcohol (196 mg, 1 eq) was then added to the reaction system, and stirring was continued for 2 hours to obtain the desired product. The reaction mixture was washed with water and saturated brine, then concentrated to dryness under reduced pressure. The residue was purified by column chromatography to afford 18-1 (330 mg).

### Synthesis of 18-2

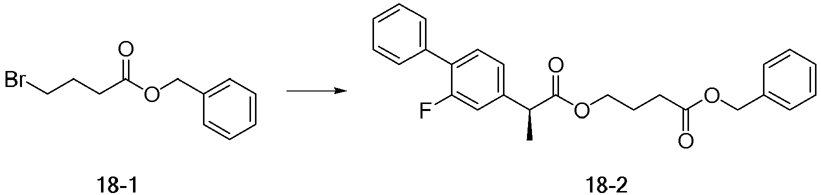

18-1 (300 mg, 1.2 eq) and S-flurbiprofen (238 mg, 1 eq) were dissolved in ACN. K₂CO₃ (404 mg, 3 eq) was added, and the mixture was stirred for 2 hours. The reaction mixture was diluted with ethyl acetate. The organic phase was washed with water and saturated sodium chloride solution, then concentrated to dryness under reduced pressure. The residue was purified by column chromatography to afford 18-2 (420 mg).

### Synthesis of 18-3

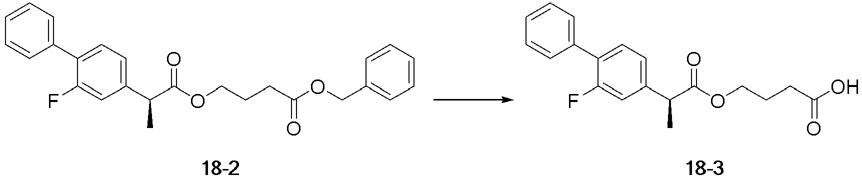

18-2 (400 mg) was dissolved in methanol (3 mL). Palladium hydroxide on carbon was added, and the atmosphere was replaced with H₂. The mixture was stirred at room temperature for 3 hours. LCMS indicated the desired product was obtained. The mixture was filtered, and the filtrate was concentrated to dryness under reduced pressure. The residue was purified by column chromatography to afford 18-3 (200 mg).

### Synthesis of Example 11

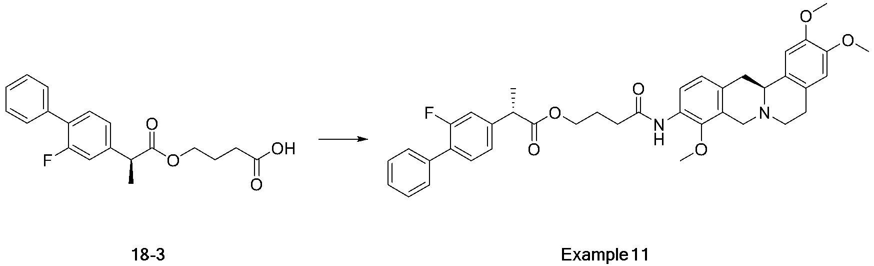

18-3 (200 mg, 1 eq) was dissolved in DCM. Oxalyl chloride (152 mg, 2 eq) and one drop of DMF were added, and the mixture was stirred for 2 hours. It was then concentrated to dryness under reduced pressure and re-dissolved in DCM. This solution was added to a solution of Example 2 (165 mg, 0.8 eq) in DCM. After the reaction was complete, the organic phase was washed with water and saturated sodium chloride solution, then concentrated to dryness under reduced pressure. The residue was purified by column chromatography to afford Example 11 (33 mg). MS: 653, ¹H NMR (400 MHz, DMSO-*d*₆) δ 9.29 (s, 1H), 7.67 (d, *J =* 8.0 Hz, 1H), 7.54 - 7.38 (m, 6H), 7.23 (dd, *J =* 16.0, 12.0 Hz, 2H), 6.89 (s, 2H), 6.69 (s, 1H), 4.15 - 4.05 (m, 3H), 3.88 (d, *J* = 8.0 Hz, 1H), 3.75 - 3.73 (m, 8H), 3.64 (s, 3H), 3.45 - 3.41 (m, 4H), 3.15 - 3.10 (m, 1H), 2.99 - 2.91 (m, 1H), 1.91 - 1.87 (m, 2H), 1.44 (d, *J* = 8.0 Hz, 3H).

### Example 12

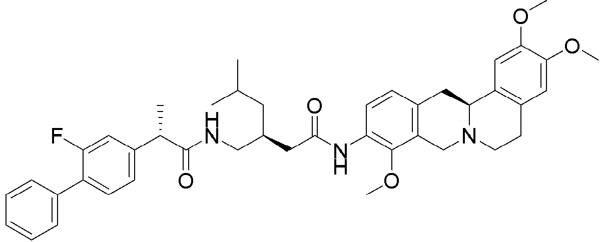

### Synthesis of 19-1

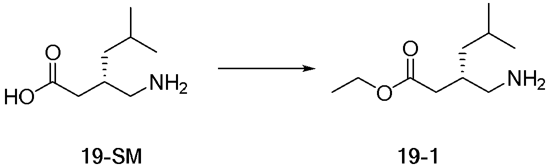

19-SM (300 mg, 1 eq) was added to ethanol. Thionyl chloride (448 mg, 2 eq) was added dropwise, and the mixture was stirred for 1 hour. The mixture was then concentrated to dryness under reduced pressure to afford 19-1 (160 mg). MS: 188.

### Synthesis of 19-2

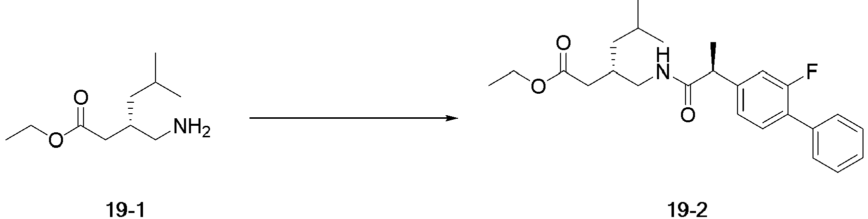

S-Flurbiprofen (200 mg, 1 eq) was dissolved in DCM. Then, 19-1 (153 mg, 1 eq), EDCI (187 mg, 1.2 eq), and DMAP (0 mg, 0.1 eq) were added. The mixture was stirred at room temperature for 2 hours. After the reaction was complete, the mixture was concentrated to dryness. The residue was purified by column chromatography to afford 19-2 (200 mg). MS: 414.

### Synthesis of 19-3

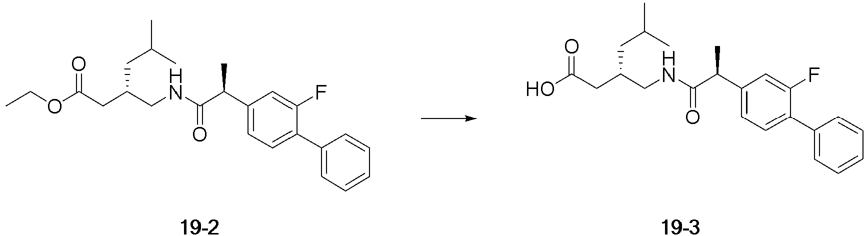

19-2 (200 mg) was dissolved in a mixed solvent of methanol:tetrahydrofuran:water = 1:1:1. Lithium hydroxide monohydrate (101 mg, 5 eq) was added, and the mixture was stirred for 1 hour. The reaction mixture was adjusted to neutral pH with dilute hydrochloric acid, extracted with ethyl acetate, and concentrated to dryness under reduced pressure to afford 200 mg of crude product 19-3. MS: 386.

### Synthesis of Example 12

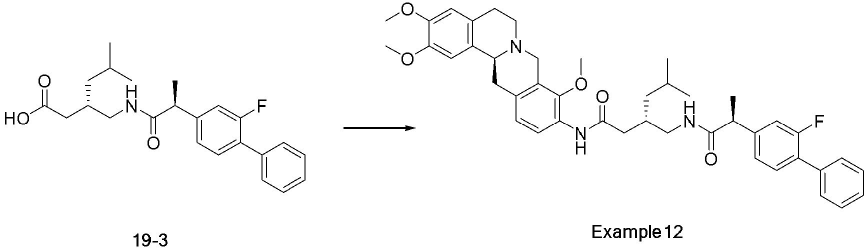

The crude 19-3 (150 mg) was dissolved in DCM. Example 2 (130 mg, 1 eq), EDCI (87 mg, 1.2 eq), and DMAP (5 mg, 0.1 eq) were added, and the mixture was stirred for 2 hours. After the reaction was complete, the reaction mixture was extracted with dichloromethane and concentrated to dryness under reduced pressure. The residue was purified by column chromatography to afford **Example 12** (139.7 mg). MS: 708, ¹H NMR (400 MHz, DMSO-*d*₆) *δ* 9.28 (d, *J* = 8.0 Hz, 1H), 8.05 (s, 1H), 7.66 (d, *J* = 8.0 Hz, 1H), 7.50 - 7.37 (m, 6H), 7.26 - 7.24 (m, 2H), 6.90 (d, *J =* 8.0 Hz, 2H), 6.69 (s, 1H), 4.08 (d, *J =* 16.0 Hz, 1H), 3.75 - 3.65 (m, 10H), 3.46 - 3.41 (m, 3H), 3.14 - 2.91 (m, 4H), 2.69 - 2.58 (m, 2H), 2.54 - 2.46 (m, 1H), 2.29 - 2.28 (m, 2H), 2.05 - 1.99 (m, 1H), 1.66 - 1.61 (m, 1H), 1.38 - 1.30 (m, 4H), 0.85 - 0.74 (m, 7H).

### Example 13

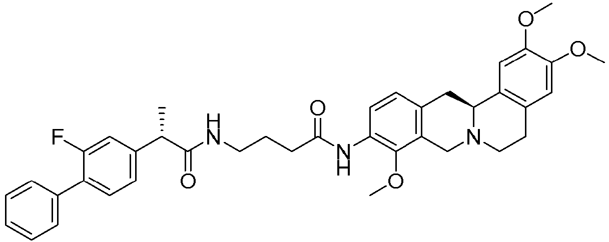

### Synthesis of 20-1

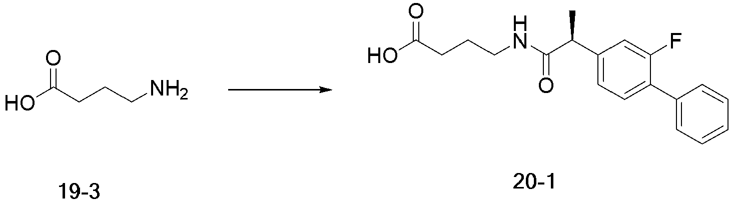

200 mg of S-flurbiprofen was dissolved in 2 mL of super-dry DCM under a nitrogen atmosphere. 0.82 mL of oxalyl chloride solution was added, and the mixture was stirred under ice-water bath cooling. After 30 minutes, the reaction mixture was concentrated. 85 mg of gamma-aminobutyric acid was dissolved in 2 mL of THF, and 2 mL of 2 M NaOH solution was added with stirring. The previous concentrate was dissolved in 2 mL of THF and added dropwise to the reaction mixture. After 30 minutes, the reaction mixture was concentrated. The mixture was diluted with 50 mL of EA, washed twice with water and once with saturated brine. The mixture was then dried, filtered, and concentrated. The residue was purified by medium-pressure chromatography to afford 20-1 (180 mg).

### Synthesis of Example 13

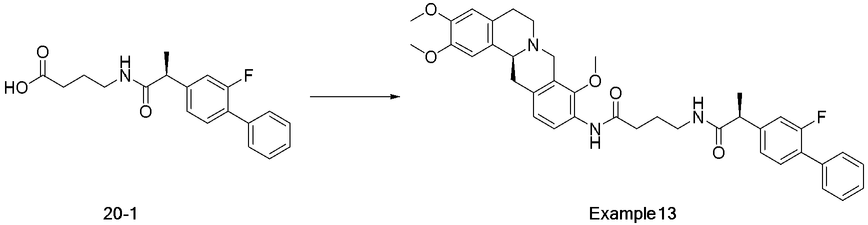

116 mg of 20-1, 120 mg of Example 2, and 161 mg of HATU were dissolved in 2 mL of super-dry DMF. DIEA was added with stirring. After 30 minutes, the reaction mixture was diluted with 50 mL of water and extracted twice with DCM. The organic phases were combined, washed twice with water and once with saturated brine, and dried over anhydrous sodium sulfate. The mixture was purified by medium-pressure chromatography, followed by concentration of the collected fractions and lyophilization, to afford Example 13 (42.62 mg). MS: 651, ¹H NMR (400 MHz, DMSO-*d*₆) δ 9.25 (s, 1H), 7.53 - 7.37 (m, 4H), 7.25-7.22 (m, 2H), 6.90 - 6.85 (m, 2H), 6.69-6.64 (m, 3H), 6.56-6.54 (d, *J* = 8.0 Hz, 1H), 4.66 (s, 2H), 3.75-3.72 (m, 9H), 3.66 - 3.63 (m, 5H), 3.46-3.25 (m, 4H), 3.12 - 3.09 (m, 3H), 2.97-2.89 (m, 2H), 2.63-2.58 (m, 2H), 2.45-2.33 (m, 2H), 1.38-1.34 (m, 2H).

### Example 14

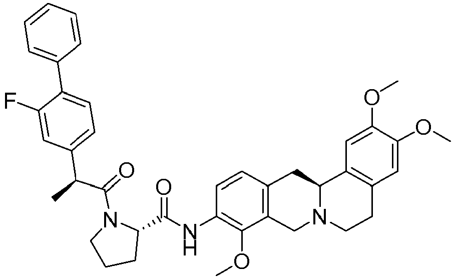

### Synthesis of 21-1

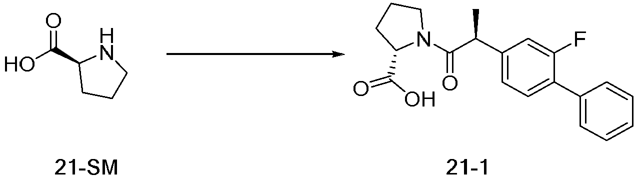

S-Flurbiprofen (300 mg, 1 eq) was added to DCM. After complete dissolution, thionyl chloride (290 mg, 2 eq) was added dropwise, and the mixture was stirred overnight. The mixture was then rotary evaporated to dryness to obtain the acid chloride of S-flurbiprofen. The acid chloride was dissolved in DCM and added to a mixture of 21-SM (141 mg, 1 eq) and TEA (310 mg, 2.5 eq). The mixture was stirred at room temperature for 2 hours. LCMS indicated the desired product. The reaction was quenched with water and extracted three times with dichloromethane. The organic phase was washed with saturated brine, dried over anhydrous sodium sulfate, and filtered. The filtrate was concentrated to dryness under reduced pressure and purified by column chromatography to afford 21-1 (310 mg). MS: 342.

### Synthesis of Example 14

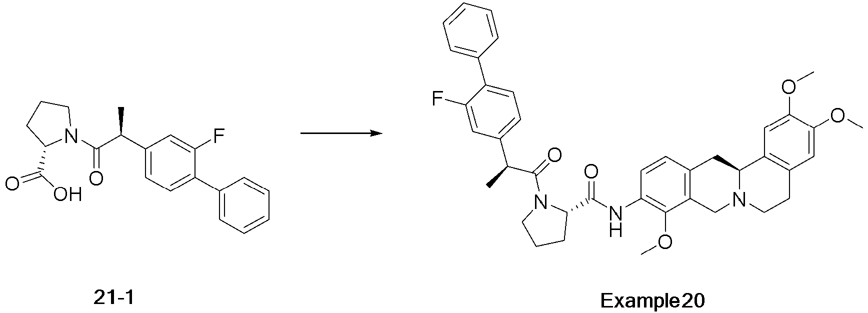

21-1 (300 mg, 1 eq) was dissolved in DCM. Example 2 (300 mg, 1 eq), EDCI (201 mg, 1.2 eq), and DMAP (10 mg, 0.1 eq) were added. The mixture was stirred at room temperature for 2 hours. LCMS indicated the reaction was complete. The mixture was extracted three times with dichloromethane. The organic phase was washed with saturated brine, dried over anhydrous sodium sulfate, and rotary evaporated to dryness. The residue was purified by column chromatography using petroleum ether/ethyl acetate (1:5) to afford the desired product, Example 14 (120 mg). MS: [M+H] 664, ¹H NMR (400 MHz, DMSO-*d*₆) δ 9.41 (s, 1H), 7.71 (d, *J* = 8.0 Hz, 1H), 7.56 - 7.38 (m, 6H), 7.29 - 7.25 (m, 2H), 6.93 - 6.88 (m, 2H), 6.69 (d, *J* = 2.8 Hz, 1H), 4.15 - 4.05 (m, 2H), 3.86 - 3.82 (m, 1H), 3.75 - 3.73(m, 9H), 3.49 - 3.41 (m, 3H), 3.16 - 3.10 (m, 1H), 2.98 - 2.91 (m, 1H), 2.65 - 2.45 (m, 3H), 2.01 - 1.82 (m, 4H), 1.39 - 1.35 (m, 3H).

### Example 15

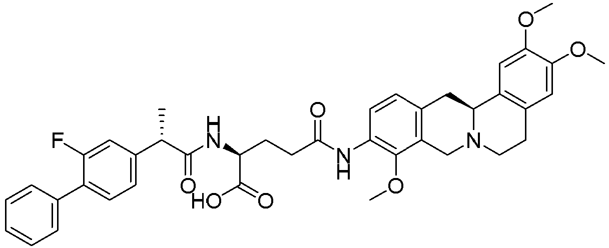

### Synthesis of 22-1

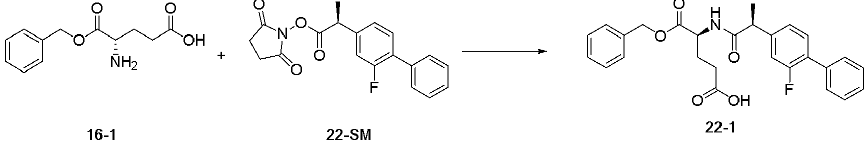

200 mg of 22-SM and 139 mg of 16-1 were dissolved in 5 mL of DMF. 178 mg of triethylamine was added with stirring, and the mixture was stirred at room temperature overnight. The reaction mixture was diluted with water, and the pH of the solution was adjusted to approximately 2 with hydrochloric acid. The aqueous phase was extracted twice with EA. The organic phases were combined, washed twice with water and once with saturated brine, dried over anhydrous sodium sulfate, and filtered. The residue was purified by medium-pressure preparative chromatography to afford 220 mg of **22-1.**

### Synthesis of 22-2

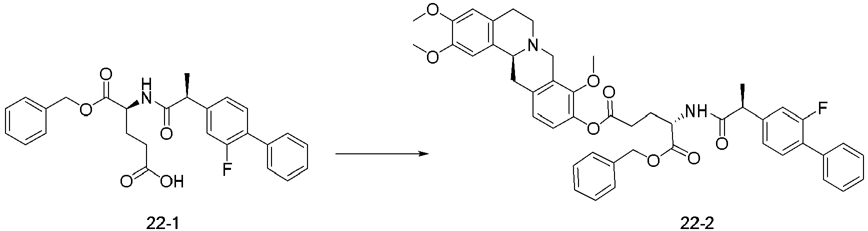

220 mg of **22-1** and 161 mg of **Example 2** were dissolved in 4 mL of DCM. Anhydrous sodium carbonate was added with stirring, and the mixture was stirred at room temperature overnight. The reaction mixture was diluted with DCM, washed twice with water and once with saturated brine, dried over anhydrous sodium sulfate, filtered, and concentrated. The residue was purified by medium-pressure preparative chromatography to afford **22-2** (162 mg).

### Synthesis of Example 15

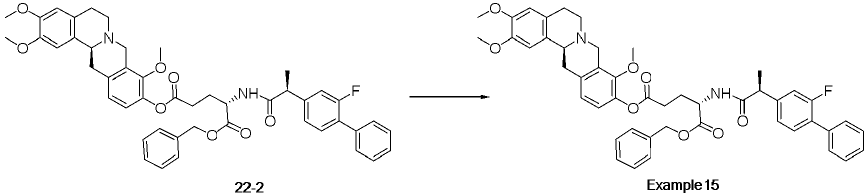

162 mg of 22-2 was dissolved in 4 mL of MeOH. 20 mg of palladium hydroxide was added, and the mixture was purged with hydrogen three times and stirred at room temperature for 2 h. The reaction mixture was filtered through a funnel containing diatomaceous earth, and the filtrate was concentrated. The residue was purified by reverse-phase medium-pressure preparative chromatography, followed by concentration of the collected fractions and lyophilization, to afford **Example 15** (85.35 mg). MS: 696, ¹H NMR (400 MHz, DMSO-*d*₆) δ 9.489 (d, *J* = 40.8 Hz, 1H), 8.321(s, 2H), 7.972(s, 1H), 7.671(dd, *J =* 14.4 Hz, 4.8 Hz, 1H), 7.532 - 7.387(m, 6H), 7.288 - 7.256(m, 2H), 6.903 - 6.853(m, 2H), 6.683(s, 1H), 4.094 - 4.023(m, 2H), 3.851 - 3.841(m, 1H), 3.747 - 3.686(m, 9H), 3.606(s, 2H), 3.466 - 3.397(m, 3H), 3.136 - 3.106(m, 1H), 2.969 - 2.902(m, 1H), 2.670 - 2.442(m, 3H), 2.008 - 1.828(m, 2H), 1.378 - 1.354(m, 3H).

### Example 16

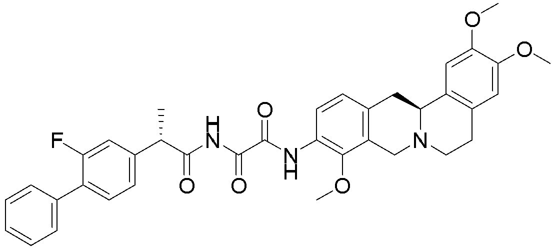

### Synthesis of 23-1

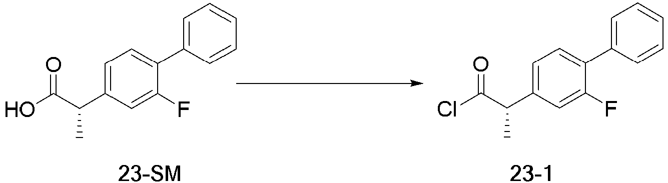

23-SM (500 mg, 1.0 eq) was placed in a single-neck flask. Oxalyl chloride (0.35 mL, 0.2 eq) was added dropwise under a nitrogen atmosphere with ice-water bath cooling. The mixture was stirred at room temperature for 3 h. LCMS monitoring showed no starting material remaining. The mixture was directly concentrated by rotary evaporation to dryness for use in the next step. Methanol was used to determine the product content.

### Synthesis of 23-2

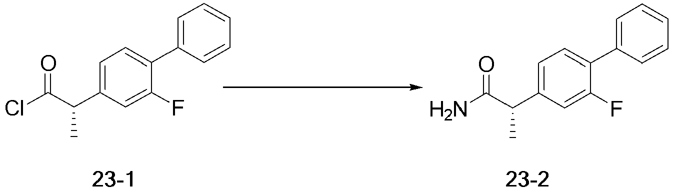

23-1 was taken up in 1 mL of THF, and 15 mL of ammonia solution was added dropwise under ice-water bath cooling. The mixture was stirred at room temperature overnight. LCMS detection showed no starting material remaining. For work-up, the mixture was concentrated by rotary evaporation to dryness and directly purified by reverse-phase column to obtain the product **23-2** (480 mg). MS: 244, ¹H NMR (400 MHz, DMSO-*d*₆) *δ* 7.56 - 7.35 (m, 7H), 7.23 (d, *J =* 9.3 Hz, 2H), 6.92 (s, 1H), 3.64 (q, *J =* 7.0 Hz, 1H), 1.35 (d, *J* = 7.0 Hz, 3H).

### Synthesis of 23-3

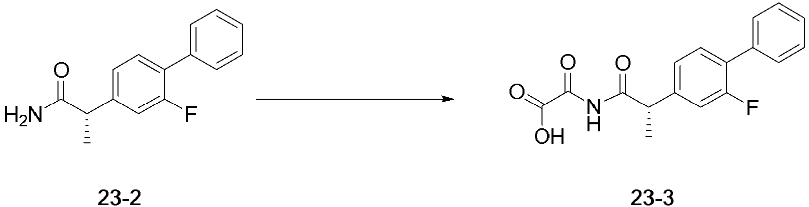

**23-2** (100 mg, 1 eq) was taken up in 2 mL of DCE. Oxalyl chloride (262.1 mg, 5 eq) was added dropwise under ice-water bath cooling. The mixture was stirred at room temperature overnight. LCMS detection showed no starting material remaining. The mixture was concentrated by rotary evaporation to dryness under a stream of nitrogen and used directly in the next step.MS: 316.

### Synthesis of Example 16

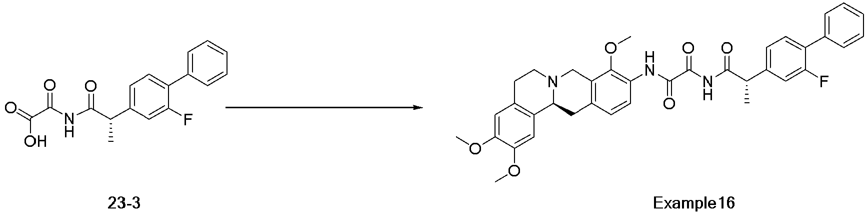

23-3 (crude product) was taken up in 1 mL of super-dry acetonitrile, and then added dropwise to a solution of Example 2 (153.9 mg, 1.1 eq, free form) in acetonitrile (1 mL). The system was heated at 50°C for 3 h. For work-up, the reaction mixture was directly subjected to reverse-phase column chromatography, concentrated, and lyophilized to obtain **Example 16** (67 mg). MS: 638, ¹H NMR (400 MHz, DMSO-d6) δ 9.87 (s, 1H), 7.56 - 7.45 (m, 6H), 7.41 - 7.37 (m, 1H), 7.34 - 7.30 (m, 2H), 6.99 - 6.96 (m, 2H), 6.73 - 6.70 (m, 1H), 5.08 (s, 1H), 4.20 - 4.15(m, 1H), 3.76 - 3.74 (m, 6H), 3.66 - 3.65 (m, 3H), 3.55 - 3.46 (m, 3H), 3.21 - 3.16 (m, 1H), 3.00(s, 1H), 2.72 - 2.67(m,3H), 1.55 (d, J = 4.0 Hz, 3H).

### Example 17

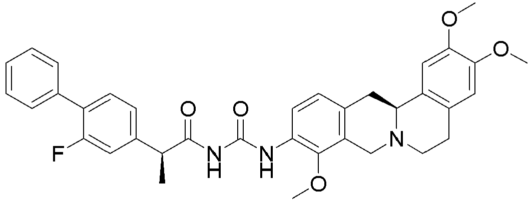

The synthetic procedure for Example 17 was the same as that described for Example 16.

MS: 638, ¹H NMR (400 MHz, DMSO-d6) δ 9.87 (s, 1H), 7.56 - 7.45 (m, 6H), 7.41 - 7.37 (m, 1H), 7.34 - 7.30 (m, 2H), 6.99 - 6.96 (m, 2H), 6.73 - 6.70 (m, 1H), 5.08 (s, 1H), 4.20 - 4.15(m, 1H), 3.76 - 3.74 (m, 6H), 3.66 - 3.65 (m, 3H), 3.55 - 3.46 (m, 3H), 3.21 - 3.16 (m, 1H), 3.00(s, 1H), 2.72 - 2.67(m,3H), 1.55 (d, J = 4.0 Hz, 3H).

### Example 18

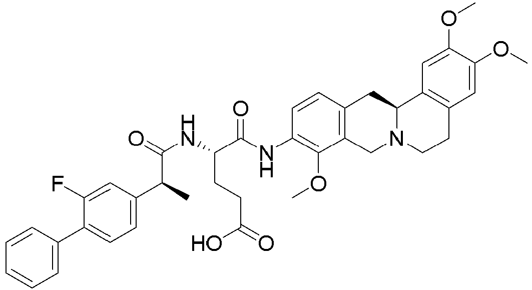

### Synthesis of 25-1

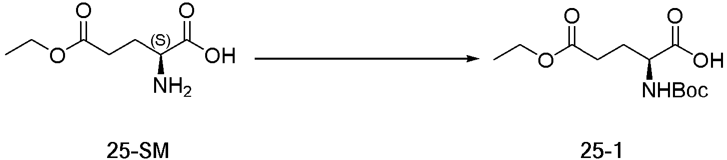

**25-SM** (1 g, 1.0 eq) was dissolved in 17 mL of H₂O. NaHCO₃ (1.06 g, 2.2 eq) was added at 0°C. A solution of (Boc)₂O (1.32 g, 1.06 eq) in dioxane (17 mL) was slowly added dropwise at 0°C. The mixture was stirred at room temperature overnight. LCMS monitoring showed no starting material remaining. The mixture was concentrated, and the pH was adjusted to 3-4 with citric acid. The mixture was extracted with a mixed solvent of DCM/MeOH (10:1), washed with saturated sodium chloride solution, dried over anhydrous sodium sulfate, concentrated by rotary evaporation to dryness. The crude product 25-1 was used directly in the next step. MS: 276.

### Synthesis of 25-2

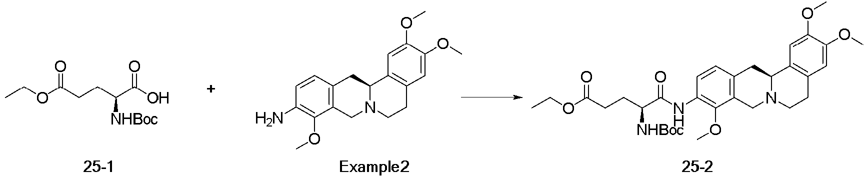

**Example 2** (250 mg, 1.0 eq) was added to 7 mL of DCM, followed by the addition of 25-1 (243 mg, 1.2 eq) and DMAP (98.7 mg, 1.1 eq). After stirring evenly, EDCI (282 mg, 2 eq) was added. The mixture was stirred at room temperature overnight. LCMS detection showed no starting material remaining. The reaction was quenched by adding water, extracted with DCM, washed with saturated ammonium chloride solution, dried over anhydrous sodium sulfate, concentrated by rotary evaporation to dryness. The residue was purified by normal-phase column chromatography to afford the product **25-2** (350 mg). Yield: 79.7%. MS: 598.

### Synthesis of 25-3

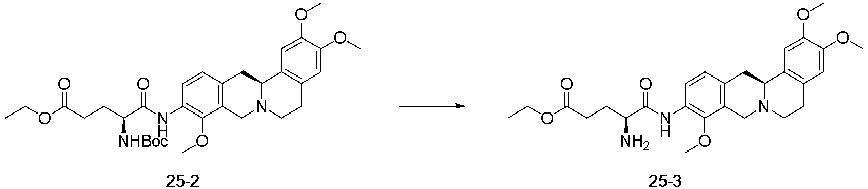

**25-2** (350 mg) was dissolved in 5 mL of DCM. A 4 M HCl solution in 1,4-dioxane (3 mL) was added dropwise at room temperature. The mixture was stirred at room temperature for 2 h. LCMS detection showed no starting material remaining. For work-up, the mixture was concentrated by rotary evaporation to dryness. The mixture was slurried with petroleum ether, followed by filtration. The filter cake was collected and dried to afford 295 mg of crude product **25-3,** which was used directly in the next step. MS: 498.

### Synthesis of 25-4

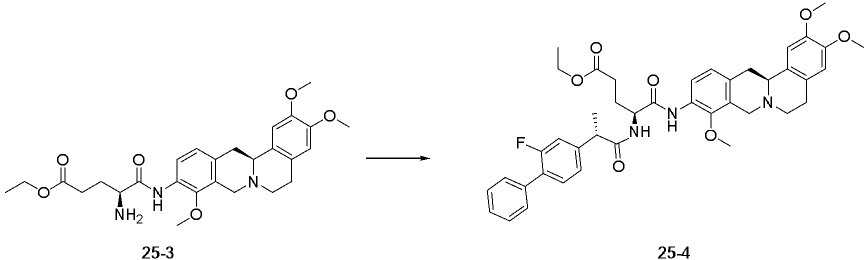

**25-3** (200 mg, 1 eq) was added to 6 mL of DCM. S-Flurbiprofen (118 mg, 1.2 eq) and DMAP (54 mg, 1.1 eq) were added at room temperature. After stirring evenly, EDCI (154 mg, 2.0 eq) was added. The mixture was stirred at room temperature for 4 h. LCMS detection showed no starting material remaining. The reaction was quenched by adding water, extracted with DCM, washed with saturated ammonium chloride solution, dried over anhydrous sodium sulfate, concentrated by rotary evaporation to dryness. The residue was purified by prep-TLC to afford the product **25-4** (198 mg). Yield: 68.1%. MS: 724.

### Synthesis of Example 18

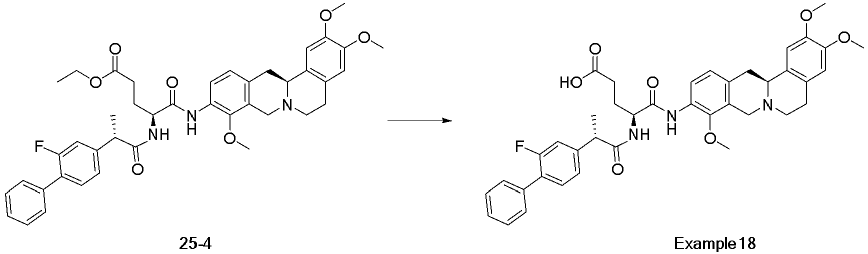

**25-4** (198 mg, 1 eq) was added to 3 mL of EtOH. An aqueous solution (3.2 mL) of lithium hydroxide monohydrate (16 mg, 1.5 eq) was added. After stirring evenly, the mixture was heated at 60°C for 30 min. LCMS detection showed no starting material remaining. The mixture was concentrated, adjusted to pH 3-4 with dilute hydrochloric acid, extracted with DCM, dried over anhydrous sodium sulfate, and concentrated by rotary evaporation to dryness. The residue was purified by reverse-phase column chromatography, followed by concentration and lyophilization to afford **Example 18** (123 mg). Yield: 63.7%. **MS: 696, HNMR:** 1H NMR (400 MHz, DMSO-d6) δ 9.18 (s, 1H), 8.66 (d, J = 8.0 Hz, 1H), 8.36 (s, 1H), 7.66 (d, J = 8.0 Hz, 1H), 7.54 - 7.36 (m, 6H), 7.30 - 7.24 (m, 2H), 6.89 - 6.87 (m, 2H), 6.69 -6.68 (m, 1H), 4.54 (q, J = 8.0 Hz, 1H), 4.02 (d, J = 16.0 Hz, 1H), 3.88 - 3.83 (m, 1H), 3.75 - 3.71 (m, 6H), 3.54 (s, 3H), 3.42 - 3.38 (m, 4H), 3.11 - 3.07 (m, 1H), 2.92-2.90 (m, 1H), 2.63 - 2.59 (m, 1H), 2.46 - 2.43 (m, 1H), 2.30 - 2.26 (m, 2H), 2.05 - 1.99 (m, 1H), 1.92 - 1.88 (m, 1H), 1.95 - 1.81 (m, 1H), 1.40 (d, J = 8.0 Hz, 3H).

### Example 19

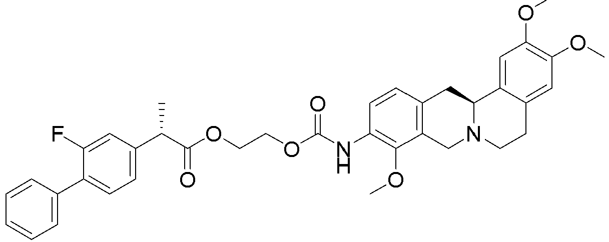

### Synthesis of 26-1

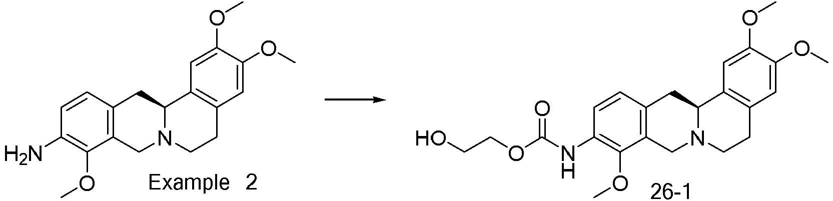

Example 2 (500 mg, 1 eq) was dissolved in 10 mL of DCM. Triphosgene (217 mg, 0.5 eq) and triethylamine (444 mg, 3 eq) were added at 0°C. The mixture was stirred at 0-10°C for 2 hours. Ethylene glycol (982 mg, 10 eq) was then added to the system, and the mixture was stirred at room temperature for 1 hour. LCMS analysis was performed. The reaction mixture was extracted twice with 50 mL of dichloromethane, and washed once with 50 mL of saturated brine. The organic phase was dried over anhydrous sodium sulfate, filtered, and concentrated under reduced pressure to dryness. The residue was purified by column chromatography, eluting with petroleum ether:ethyl acetate = 1:1, to afford 180 mg of the product. Yield: 29%.

### Synthesis of Example 19

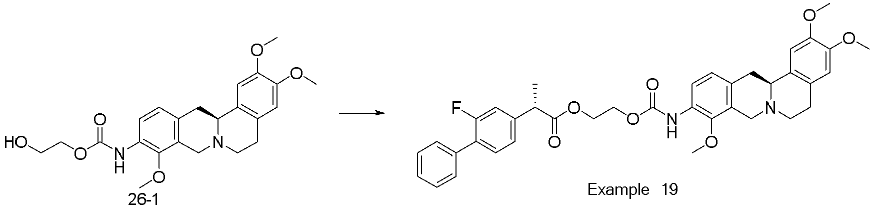

26-1 (180 mg, 1 eq) was dissolved in 10 mL of dichloromethane. S-Flurbiprofen (102 mg, 1 eq), EDCI (96 mg, 1.2 eq), and DMAP (5 mg, 0.1 eq) were added. The mixture was stirred at room temperature for 1 hour, and LCMS analysis was performed. The organic phase was extracted twice with 10 mL of dichloromethane, washed once with saturated brine, dried over anhydrous sodium sulfate, filtered, and concentrated under reduced pressure to dryness. The residue was purified by column chromatography, eluting with petroleum ether:ethyl acetate = 1:1, to afford 72 mg of the product. Yield: 26%. MS: [M+H] 655 HNMR: 1H NMR (400 MHz, DMSO-d6) δ 8.750 (s, 1H), 7.527 - 7.366 (m, 7H), 7.272 - 7.16 (m, 2H), 6.892 - 6.878 (m, 2H), 6.687 (s, 1H), 4.348 - 4.263 (m, 4H), 4.089 (d, J = 15.6 Hz, 1H), 3.912 (q, J = 7.2 Hz, 1H), 3.751(s, 3H), 3.729 (s, 3H), 3.640 (s, 3H), 3.443 - 3.323(m, 3H), 3.114(dd, J1 = 10.4 Hz, J2 = 4.4 Hz, 1H), 2.975-2.907(m, 1H), 2.640-2.456(m, 3H), 1.448(d, J = 7.2 Hz, 3H).

### Example 20

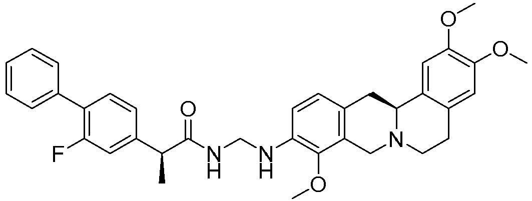

### Synthesis of 27-1

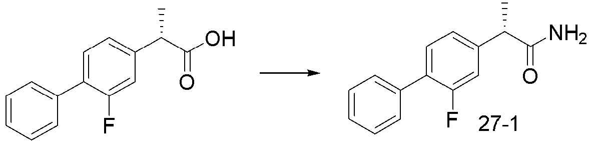

S-Flurbiprofen (500 mg, 1 eq) was dissolved in DCM. EDCI (469 mg, 1.2 eq), DMAP (25 mg, 0.1 eq), NH₄Cl (219 mg, 2 eq), and TEA (413 mg, 2 eq) were added. The mixture was stirred at room temperature for 2 hours. LCMS analysis indicated the reaction was complete. The mixture was concentrated under reduced pressure to dryness, and the residue was purified by column chromatography, eluting with petroleum ether:ethyl acetate = 1:1, to afford the product (397 mg). Yield: 80%.

### Synthesis of 27-2

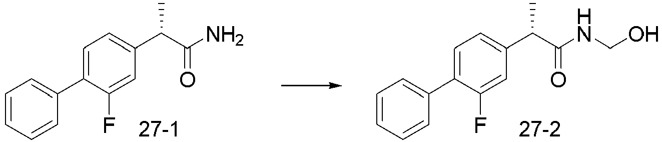

27-1 (243 mg, 1 eq) was dissolved in 30 mL of 37% aqueous formaldehyde solution. Potassium hydroxide (5 mg, 0.1 eq) was added, and the mixture was stirred at 80°C for 5 minutes. The mixture was then allowed to cool to room temperature and stirred overnight. LCMS analysis indicated the disappearance of the starting material, and TLC analysis showed the formation of a new spot. The reaction mixture was extracted once with ethyl acetate (80 mL). The organic phase was washed with saturated brine, dried over anhydrous sodium sulfate, filtered, and concentrated to dryness under reduced pressure. The residue was dissolved in 80 mL of dichloromethane and filtered. The filtrate was concentrated to dryness under reduced pressure. The residue was purified by column chromatography, eluting with petroleum ether:ethyl acetate = 1:1, to afford crude 27-2 (411 mg).

### Synthesis of 27-3

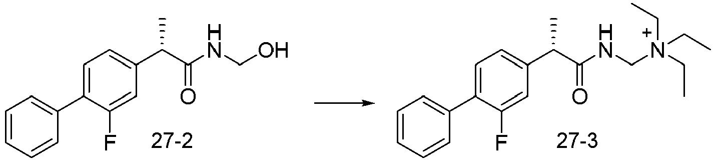

27-2 (300 mg, 1 eq) was dissolved in 10 mL of super-dry DCM. After complete dissolution, thionyl chloride (259 mg, 2 eq) was added at 0°C, and the mixture was stirred at room temperature for 2 hours. To the reaction mixture was added 50 mL of n-hexane, followed by filtration. The filter cake was rinsed with a small amount of n-hexane. The filtrate was concentrated under reduced pressure. The residue was re-dissolved in 10 mL of acetone, and triethylamine (440 mg, 4 eq) was added. The mixture was concentrated to dryness under reduced pressure to afford 208 mg of crude 27-3.

### Synthesis of Example 20

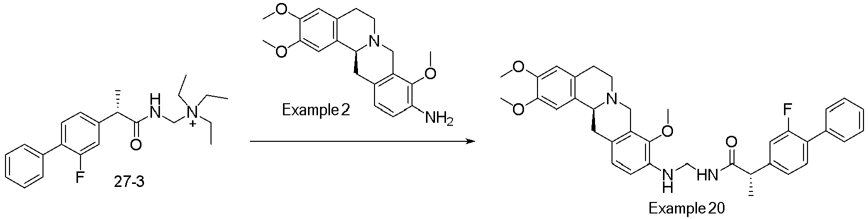

27-3 (200 mg, 1 eq) was dissolved in 2 mL of tetrahydrofuran. Example 2 (191 mg, 1 eq) was added, and the mixture was stirred at room temperature overnight. LCMS analysis was performed. The reaction mixture was extracted with 20 mL of ethyl acetate, dried, filtered, and concentrated to dryness under reduced pressure. The residue was purified by column chromatography, eluting with ethyl acetate:petroleum ether = 1:1, to afford a crude product. The crude product was separated by preparative SFC to yield 18 mg of the target product as an off-white solid, which was odorless and stored at room temperature.

MS: [M+H] 596.4, ¹H NMR (400 MHz, DMSO-d6) δ 8.376(t, J = 6.8 Hz, 1H), 7.526-7.356(m, 6H), 7.200-7.123(m, 2H), 6.817(s, 1H), 6.731-6.704(m, 2H), 6.601(d, J = 11.2 Hz, 1H), 5.457(t, J = 8.8 Hz, 1H), 4.553(t, J = 8.4 Hz, 2H), 4.020(d, J = 19.6 Hz, 1H), 3.738(s, 3H), 3.726(s, 3H), 3.661(q, J *=* 9.6 Hz, 1H), 3.569(s, 3H), 3.400-3.252(m, 3H), 3.113-3.057(m, 1H), 2.972-2.867(m, 1H), 2.635-2.397(m, 3H), 1.325(d, J = 9.6 Hz, 3H).

### Example 21

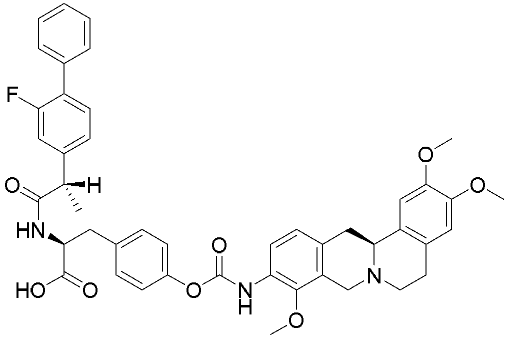

### Synthesis of 28-1

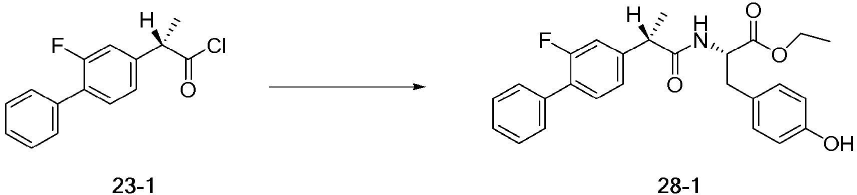

To a 10 mL flask was added 140.0 mg (1.5 eq) of L-tyrosine ethyl ester, 2 mL of DCM, and 295 mg of DIEA (6.0 eq). The mixture was cooled in an ice bath under a nitrogen atmosphere, and a solution of 100.0 mg (1.0 eq) of **23-1** in 1 mL of DCM was added dropwise. The mixture was warmed to room temperature and reacted for 2 h. The reaction was monitored by LC/MS and determined to be complete. Then, 20 mL of purified water was added to the reaction mixture, and the mixture was extracted with 2×20 mL of DCM. The combined DCM phases were washed with 20 mL of saturated sodium chloride, dried over anhydrous sodium sulfate, filtered, and concentrated. The residue was purified by silica gel column chromatography to afford 150 mg of the product 28-1 as a solid, with a yield of 90.4%. MS:436. ¹H NMR (400 MHz, DMSO-*d*₆) δ 9.218 (s, 1H), 8.451 (d, *J =* 8.0 Hz, 1H), 7.535 - 7.183 (m, 6H), 7.214 - 7.183 (m, 2H), 6.981 (d, *J =* 8.4 Hz, 2H), 6.651 (d, *J =* 8.4 Hz, 2H), 4.393 - 4.336 (m, 1H), 3.989 (q, *J =* 7.2 Hz, 2H), 3.730 (q, *J =* 6.8 Hz, 1H), 2.914 (dd, *J =* 14.0Hz, 6.0 Hz, 1H), 2.807 (dd, *J =* 14.0 Hz, 9.2 Hz, 1H), 1.265 (d, *J =* 7.2 Hz, 3H), 1.046 (t, *J =* 7.2 Hz, 3H).

### Synthesis of 28-2

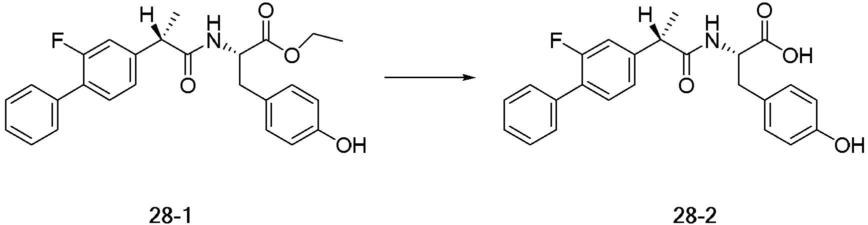

To a 25 mL reaction flask was added 250 mg of **28-1,** followed by 5 mL of THF for dissolution. Lithium hydroxide (121 mg) was dissolved in 2 mL of water and added dropwise to the above reaction mixture. After the addition was complete, the mixture was warmed to room temperature and stirred for 1 h. The reaction was monitored by LC/MS and determined to be complete. The mixture was adjusted to pH of 3 using 1 N hydrochloric acid, and extracted with 2×20 mL of EA. The combined EA phases were washed with 20 mL of saturated sodium chloride, dried over anhydrous sodium sulfate, filtered, and concentrated to dryness to afford **28-2** (220 mg). Yield: 94.0%. MS:408, ¹H NMR (400 MHz, DMSO-*d*₆) *δ* 12.516(s, 1H), 9.198 (s, 1H), 8.315 (d, *J =* 8.0 Hz, 1H), 7.537 - 7.371(m, 6H), 7.220 - 7.177 (m, 2H), 6.993 (d, *J* = 8.4 Hz, 2H), 6.651 (d, *J =* 8.4 Hz, 2H), 4.379 - 4.324 (m, 1H), 3.736 (q, *J* = 7.2 Hz, 1H), 2.956 (dd, *J =* 13.6, 4.8 Hz, 1H), 2.771 (dd, *J =* 14.0 Hz, 5.6 Hz, 1H), 1.245 (d, *J =* 7.2 Hz, 3H).

### Synthesis of 28-3

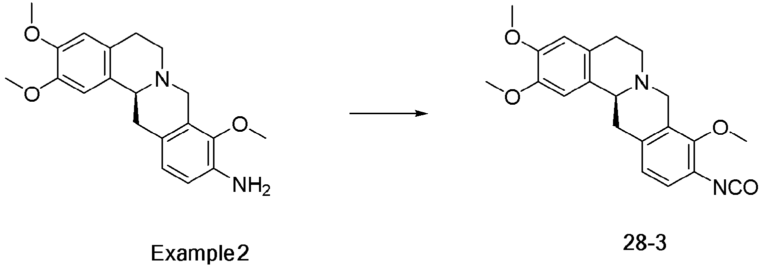

To a 10 mL reaction flask was added 200 mg of **Example 2** (1.0 eq) and 3 mL of DCM. Under a nitrogen atmosphere, the mixture was cooled to 0°C in an ice bath. Triphosgene (160 mg) was dissolved in 2 mL of dichloromethane and added dropwise to the above reaction mixture. The reaction was conducted at low temperature for 2 h. The mixture was diluted with 20 mL of DCM, washed with 20 mL of saturated sodium chloride solution, dried over anhydrous sodium sulfate, filtered, and concentrated to afford 210 mg of **28-3** as a solid, which was used directly in the next step.

### Synthesis of Example 21

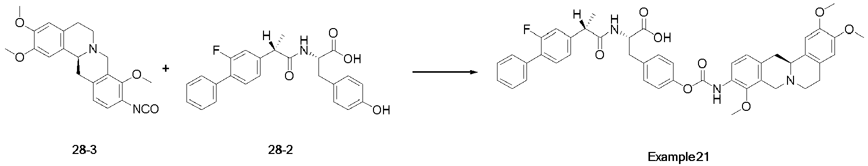

To a 10 mL reaction flask was added **28-2** (85 mg), DCM (2 mL), and triethylamine (100 mL). Under a nitrogen atmosphere, the mixture was cooled in an ice-water bath. A solution of **28-3** (110 mg) in 1 mL of DCM was added dropwise to the above reaction mixture. After the addition was complete, the mixture was stirred at room temperature overnight. The mixture was diluted with 20 mL of dichloromethane and 20 mL of purified water. The organic phase was washed with 20 mL of saturated sodium chloride solution, dried over anhydrous sodium sulfate, filtered, and concentrated. The mixture was purified by silica gel column chromatography to afford 48.4 mg of Example 21 as a solid, with a yield of 31.3%. MS: 774, ¹H NMR (400 MHz, DMSO-*d*₆) δ 9.331(s, 1H), 7.961(s, 1H), 7.529 - 7.360(m, 7H), 7.241 - 7.189 (m, 4H), 7.055 (d, *J=* 8.0 Hz, 2H), 6.938(d, *J* = 8.4 Hz, 1H), 6.891(s, 1H), 6.691 (s, 1H), 4.241 - 4.205 (m, 1H), 4.096 (d, *J* = 16.0Hz, 1H), 3.757 - 3.752(m, 6H), 3.729 (s, 3H), 3.528 - 3.389 (m, 4H), 3.167 - 3.105 (m, 2H), 2.952 - 2.902 (m, 2H), 2.676 - 2.611 (m, 3H), 1.258 (d, *J* = 7.2 Hz, 3H).

### Example 22

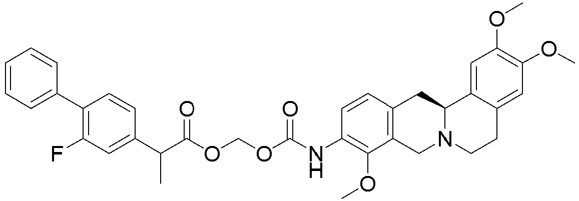

### Synthesis of 29-1

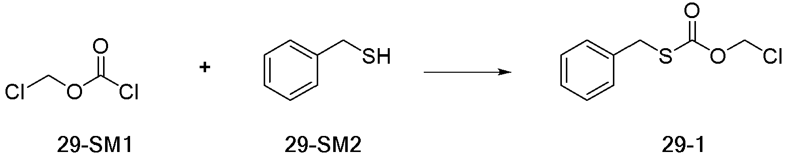

29-SM2 (1 g, 1.0 eq) and DIEA (2.08 g, 2.0 eq) were added to 10 mL of DCM. After complete dissolution, **29-SM1** (1.14 g, 1.1 eq) was slowly added dropwise. The mixture was stirred at room temperature overnight. TLC analysis indicated that almost no starting material remained. The reaction mixture was concentrated under reduced pressure to dryness to afford crude **29-1** (2.5 g), which was used directly in the next step without purification.

### Synthesis of 29-2

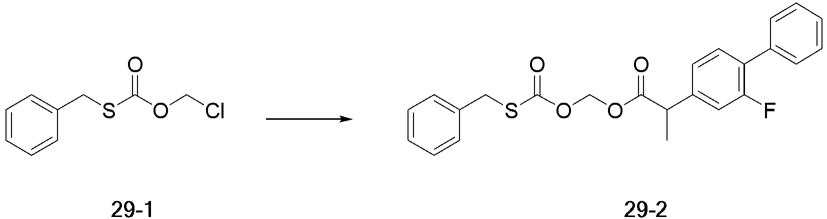

The crude product **29-1** (1.2 g) and **23-SM** (600 mg, 0.44 eq) were dissolved in ACN. DBU (400 mg, 0.47 eq) was added, and the mixture was stirred at 60°C overnight. The reaction mixture was diluted with dichloromethane and extracted with water and dichloromethane. The organic phase was washed with water and saturated sodium chloride solution, then rotary evaporated to dryness. The residue was purified by column chromatography to afford **29-2** (600 mg). Yield: 57%. ¹H NMR (400 MHz, DMSO-*d*₆) δ 7.558 - 7.407(m, 6H), 7.346 - 7.196 (m, 7H), 5.847 (d, *J =* 6.0 Hz, 1H), 5.814 (d, *J=* 6.0 Hz, 1H), 4.014 (q, *J=* 7.2 Hz, 1H), 1.450 (d, *J =* 7.2 Hz, 3H).

### Synthesis of 29-3

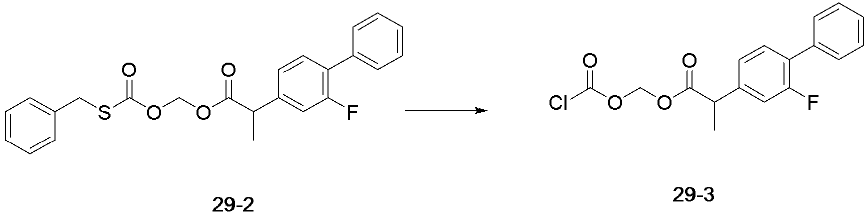

**29-2** (300 mg) was dissolved in dichloromethane. Sulfonyl chloride was added dropwise at 0°C, and the mixture was stirred at room temperature for 3 h. The reaction was quenched with methanol, and the solvent was removed to afford crude **29-3** (300 mg), which was used directly in the next step.

### Synthesis of Example 22

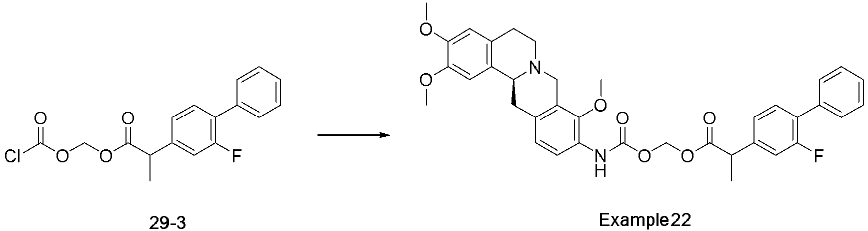

**Example 2** (200 mg, 1 eq) was dissolved in DCM. DIEA (226 mg, 3 eq) was added. After complete dissolution, a solution of **29-3** in dichloromethane was added dropwise at 0°C. The mixture was reacted at room temperature for one hour. The reaction mixture was purified by pre-packed column chromatography to afford **Example 22** (240 mg). Yield: 64%. MS: 641, ¹H NMR (400 MHz, DMSO-*d*₆) δ 9.168 (s, 1H), 7.480 - 7.446 (m, 5H), 7.410 - 7.373 (m, 1H), 7.273 - 7.223 (m, 3H), 6.896 - 6.882 (m, 2H), 6.689 (s, 1H), 5.805 - 5.749 (m, 2H), 4.056 - 3.962 (m, 2H), 3.753 (s, 3H), 3.729 (s, 3H), 3.602 (d, *J =* 1.6 Hz, 3H), 3.453 - 3.395 (m, 3H), 3.109 (dd, *J* = 10.8 Hz, 3.6 Hz, 1H), 2.974 - 2.893 (m, 1H), 2.640 - 2.446 (m, 3H), 1.456 (d, J = 6.8 Hz, 3H).

### Example 23

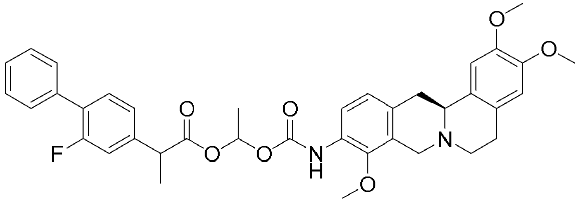

### Synthesis of 30-1

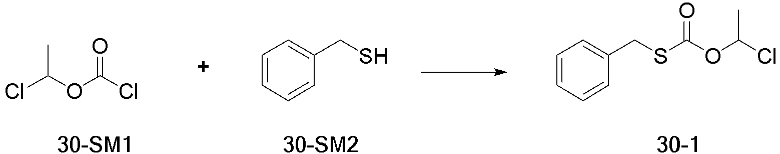

**30-SM2** (1 g, 1 eq) and DIEA (2.08 g, 2 eq) were added to DCM (8 mL). After complete dissolution, **30-SM1** (1.26 g, 1.1 eq) was slowly added dropwise. The mixture was stirred at room temperature overnight and then directly rotary evaporated to dryness to afford the crude desired product (2.5 g), which was used directly in the next step without purification.

### Synthesis of 30-2

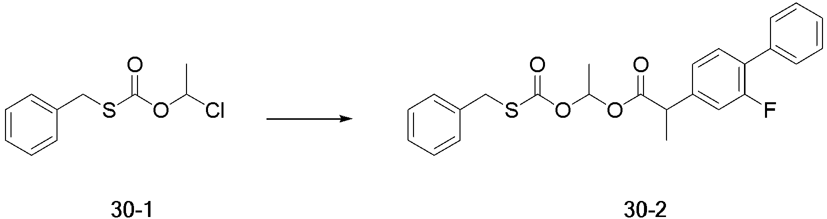

The crude **30-1** (1.29 g) and flurbiprofen (600 mg, 0.44 eq) were dissolved in ACN. DBU (400 mg, 0.47 eq) was added, and the mixture was stirred overnight. The reaction mixture was diluted with dichloromethane and extracted with water and dichloromethane. The organic phase was washed with water and saturated sodium chloride solution, rotary evaporated to dryness, and purified by column chromatography to afford **30-2** (500 mg). MS: 438, ¹H NMR (400 MHz, DMSO-d6) δ 7.570 - 7.185 (m, 13H), 6.938 - 6.875 (m, 1H), 4.225 - 4.174 (m,1H), 4.086 (d, J = 1.6 Hz, 1H), 3.963 (q, J = 7.2 Hz, 1H), 1.474 - 1.418 (m, 6H).

### Synthesis of 30-3

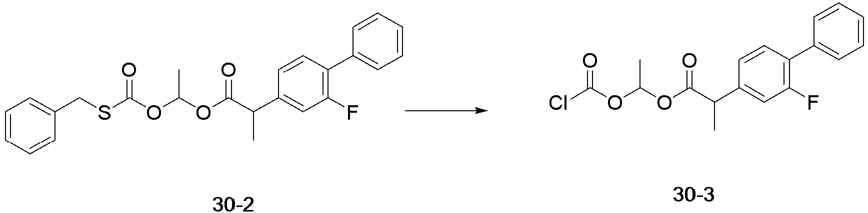

**30-2** (247 mg) was dissolved in dichloromethane (2 mL). Sulfonyl chloride was added dropwise at 0°C, and the mixture was stirred at room temperature for 1 h. The solvent was removed to afford 280 mg of crude product **30-3.**

### Synthesis of Example 23

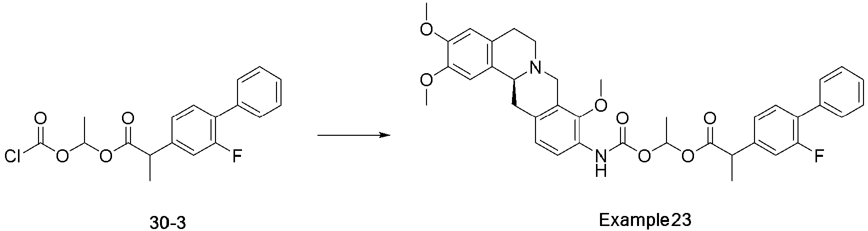

**Example 2** (190 mg, 1 eq) was dissolved in DCM, and DIEA (300 mg, 4 eq) was added. After complete dissolution, a solution of crude **30-3** (280 mg) in dichloromethane was added dropwise at 0°C. The reaction mixture was subjected to work-up, followed by purification by column chromatography to afford a crude product, which was further purified by reverse-phase column chromatography to yield **Example 23** (157 mg). MS: 655, ¹H NMR (400 MHz, DMSO-d6) δ 9.068 (d, J = 48.8 Hz, 1H), 7.564 - 7.405 (m, 6H), 7.299 - 7.203 (m,3H), 6.942 - 6.820 (m, 3H), 6.709 (s, 1H), 4.102 - 4.018 (m, 1H), 3.964 - 3.934(m, 1H), 3.774 (s, 3H), 3.750 (s, 3H), 3.691 - 3.563 (m, 3H), 3.480 - 3.412 (m, 3H), 3.164 - 3.098 (m, 1H), 3.000 - 2.912 (m, 1H), 2.692 - 2.461 (m, 3H), 1.510 - 1.434 (m, 6H).

### Activity Assay

### Assay Example 1

Animals: Mice (CD-1 mice, age: 6-8 weeks, sex: male, body weight: 20-24 g, number: 120, animal, supplier: Beijing Spefu Biotechnology Co., Ltd.). A 7-day quarantine period was observed. Routine health checks were performed by a veterinarian, and animals exhibiting abnormalities were excluded before the assay.

The mice were housed in a clean room, with 5 animals per cage. The housing room temperature was maintained at 22±3°C, with a humidity of 40-70%, and a 12-hour light/dark cycle. The cages were made of polycarbonate. Soft corn cob bedding, sterilized by highpressure steam, was used and changed twice a week. For feed and water: clean-grade rodent feed was purchased from Beijing Keao Xieli Feed Co., Ltd. Drinking water was autoclaved, and the feed was sterilized by cobalt-60 irradiation. Animals had free access to sterile food and water. All mice were weighed and the data were recorded in detail before administration.

The mice were fasted for 16 hours before drug administration. The administration method was: intravenous injection; administration volume: 10 mL/kg; dosage: 10 mg/kg. 15 minutes after administration, 0.6% acetic acid was injected into the abdominal cavity of the mice. It deposited on the visceral and parietal peritoneum, causing deep, extensive, and prolonged pain, which induced a behavioral response in the mice characterized by concave abdomen, extension of the trunk and hind limbs, and elevation of the hips. This response is termed a "writhing response." The number of writhes exhibited by the mice was used as an indicator of the pain response to evaluate the analgesic effect of the compounds. The number of "writhing responses" occurring within 20 minutes after the injection of 0.6% acetic acid was observed and the ratio of this number to the number of "writhing responses" in the control group (which did not receive the test compound) was calculated. This ratio was subtracted from 100%, and the resulting value was recorded as the inhibition rate.

**Table 1**

| | Inhibition rate (%) | | Inhibition rate (%) | | Inhibition rate (%) | | Inhibition rate (%) |
|---|---|---|---|---|---|---|---|
| s-FBS | 59.4 | Example 8 | 82.73 | Example 14 | 67.65 | Example 18 | 100.0 |
| Example 3 | 88.89 | Example 10 | 100.0 | Example 15 | 79.16 | Example 19 | 83.33 |
| Example 4 | 100.0 | Example 12 | 40.25 | Example 16 | 100.0 | Example 22 | 100 |
| Example 5 | 100.0 | Example 13 | 72.16 | Example 17 | 10.65 | Example 23 | 100 |

| | | | | | | | |
|---|---|---|---|---|---|---|---|
| Note: s-FBS refers to S-flurbiprofen. | | | | | | | |

### Assay Example 2 DRG Test

The dorsal root ganglion (DRG) serves as the primary neuron for sensory transmission, with functions in transmitting and regulating bodily sensations, as well as receiving and conducting nociceptive signals. In this assay example, the manual patch-clamp technique was employed to investigate the effects of test substances on the action potentials of DRG neurons isolated from SD rats.

Acute Isolation of DRG: Rats were anesthetized with 20% (w/w) urethane and placed in a prone position. The skin was disinfected with 75% (v/v) alcohol. An incision was made with surgical scissors from the tail to expose the spinal column, and the muscle tissue on both sides of the spinal column was removed. The spinal column, from the caudal vertebrae, was excised and placed into a 10 cm culture dish containing HBSS on ice. The upper one-third of the spinal column cross-section was cut away with scissors. The L4-L6 region was located, and after removing the spinal cord, nerve fibers connected to the dorsal root ganglia were visible. Under a dissecting microscope, the DRGs were carefully extracted and placed into a 35 mm culture dish containing HBSS. After all DRGs were collected, the nerve tissue attached to the DRGs was trimmed away under a microscope. The trimmed DRGs were uniformly minced, transferred to a 15 mL centrifuge tube, and centrifuged at 1000 rpm for 5 minutes. The supernatant was discarded. Subsequently, 5 mL of digestion solution was added, and the mixture was digested in a 37°C, 5% CO₂ incubator for approximately 20 minutes. During digestion, the centrifuge tube was gently shaken to resuspend the tissue, and the tissue was triturated 2-3 times with a pipette until it became filamentous and no tissue clumps remained, indicating complete digestion. Following digestion, 5 mL of complete culture medium was added, and the mixture was centrifuged at 1000 rpm for 5 minutes. The supernatant was discarded. Finally, an appropriate amount of complete culture medium was added, and the cells were gently resuspended. The cell suspension was then seeded onto glass coverslips placed in culture dishes. After allowing the neurons to adhere, the coverslips were used for patch-clamp detection.

Patch-Clamp Detection: The voltage stimulation protocol for recording neuronal action potentials in whole-cell patch-clamp mode was as follows: After establishing a whole-cell seal, the recording mode was switched to CurrentClamp mode. The cell membrane current was clamped at 0 pA. The clamping current was increased incrementally from -20 pA to approximately +360 pA in 20 pA steps, with each step lasting 0.8 s, followed by a return to 0 pA for 1 s. Additionally, changes in drug effect were recorded under 1.5-2 times the Rheobase stimulation, with a stimulation duration of 1 s. Experimental data were acquired using an EPC10 amplifier (HEKA) and stored in PatchMaster (HEKA) software. For the patch-clamp procedure, borosilicate glass capillaries were first pulled into recording electrodes using a microelectrode puller. The electrode, filled with intracellular solution, was then mounted onto a microelectrode holder. Under an inverted microscope, the microelectrode manipulator was used to immerse the electrode tip into the extracellular solution, and the electrode resistance (Rpip) was recorded. The electrode was then gently advanced towards the cell surface, and negative pressure was applied to form a GΩ seal. Fast capacitance compensation was performed at this point. Continued negative pressure was applied to rupture the cell membrane, establishing the whole-cell recording configuration. Finally, slow capacitance compensation was performed, and experimental parameters such as series resistance (Rs) were recorded. Leakage compensation was not applied. After the action potentials recorded in whole-cell mode stabilized, drug administration commenced. Each drug concentration was applied for approximately 5 minutes (or until the action potential stabilized) before testing the next concentration. Multiple concentrations were tested for each compound. The coverslip containing the cells was placed in a recording chamber mounted on an inverted microscope. Blank control external solution and test compound working solutions were sequentially perfused through the recording chamber from low to high concentrations over the cells in the recording chamber using a gravity-fed perfusion method, with solution exchange facilitated by a peristaltic pump during recording. For each cell, the current detected in compound-free external solution served as its own control. At least 3 cells were used for independent replicate measurements (n=3) for each concentration. All electrophysiological experiments were conducted at room temperature.

As shown in Figure 1, 1 µM S-FBS and 1 µM Example 2 did not inhibit action potential conduction; however, 1 µM Example 22 and 1 µM Example 23 inhibited action potentials of DRG neurons to varying degrees, demonstrating nerve conduction blocking effects. This indicates that the analgesic mechanism of action of the compounds of the present disclosure differs from that of S-FBS and Example 2.

The various embodiments in this specification are described in a related manner, and the same or similar parts between the various embodiments can be referred to each other. Each embodiment focuses on the differences from the other embodiments.

The above description is only of preferred embodiments of the present invention and is not intended to limit the scope of the present invention. Any modification, equivalent substitution, improvement, etc. made within the spirit and principle of the present invention are included in the scope of the present invention.

## Claims

1. A compound of formula (I), or an isotopic variant, a tautomer, a stereoisomer, a prodrug, a polymorph, a hydrate or a solvate thereof, or a pharmaceutically acceptable salt thereof: 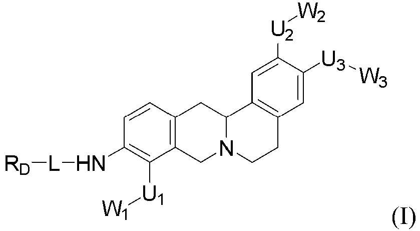 wherein,
R_{D} is a residue formed by a drug molecule;
U₁, U₂ and U₃ are each independently selected from O, S, -NH-, -C(O)-, -O-C(O)-, -NH-C(O)- and -O-CH₂-O-;
W₁, W₂ and W₃ are each independently selected from H, C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl;
L is a chemical bond or 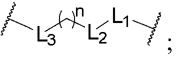 alternatively 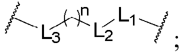
L₁ is selected from chemical bond, -C(O)-, -OC(O)-, -NR_{b}C(O)-, -S(O)-, -S(O)₂-, -OS(O)₁₋₂- and -NR_{b}S(O)₁₋₂-;
L₂ is selected from chemical bond, C₃₋₇ cycloalkylene, 3- to 7-membered heterocyclylene, phenylene and 5- to 6-membered heteroarylene;
L₃ is selected from O, S, NR', -C(O)-, -S(O)- and -S(O)₂-;
n is selected from 0, 1, 2, 3, 4, 5 and 6;
the methylene group in 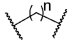 is optionally substituted with 1, 2, 3, 4, 5 or 6 independent R; R is independently selected from H, D, halogen, CN, =O, -ORₐ, -SRₐ, -NR_{b}R_{c}, -C₀₋₁₀ alkylene-C(O)Rₐ, -C₀₋₁₀ alkylene-OC(O)Rₐ, -C₀₋₁₀ alkylene-C(O)ORₐ, -C₀₋₁₀ alkylene-NR_{b}C(O)Rₐ, -C₀₋₁₀ alkylene-C(O)NR_{b}R_{c}, C₁₋₁₀ alkyl, C₁₋₁₀ haloalkyl, C₃₋₆ cycloalkyl and 3- to 6-membered heterocyclyl;
R' is selected from H, C₁₋₁₈ alkyl, C₁₋₁₈ haloalkyl, C₃₋₁₀ cycloalkyl and 3- to 10-membered heterocyclyl, or R' and R are taken together with the atoms to which they are attached to form 3- to 10-membered heterocyclyl;
Rₐ, R_{b} and R_{c} are each independently selected from H, C₁₋₁₀ alkyl and C₁₋₁₀ haloalkyl, or R_{b} and R_{c} are taken together with the atoms to which they are attached to form 3- to 10-membered heterocyclyl;
wherein each of the above groups is optionally deuterated, up to full deuteration.

2. The compound of formula (I) of claim 1, or an isotopic variant, a tautomer, a stereoisomer, a prodrug, a polymorph, a hydrate or a solvate thereof, or a pharmaceutically acceptable salt thereof, wherein, R_{D} is a residue formed by a drug molecule, the drug molecule is an anti-inflammatory agent and a derivative thereof, alternatively a non-steroidal anti-inflammatory agent, such as, loxoprofen, flurbiprofen, fenoprofen, ketoprofen, tolmetin, bromofenic acid, tiaprofenic acid, indomethacin, sulindac, ketorolac, nimesulide, mefenamic acid, clofenamic acid, diclofenac, aspirin, ibuprofen, naproxen, nabumetone, etodolac, rofecoxib, celecoxib, piroxicam, meloxicam, and oxyphenbutazone, and derivatives thereof;
alternatively, the drug molecule is selected from loxoprofen, flurbiprofen, indomethacin, mefenamic acid, clofenamic acid, and aspirin, and derivatives thereof; alternatively selected from flurbiprofen and clofenamic acid, and derivatives thereof; alternatively flurbiprofen and a derivative thereof; alternatively, the flurbiprofen is S-flurbiprofen; alternatively, the flurbiprofen is R-flurbiprofen.

3. The compound of formula (I) of claim 1 or 2, or an isotopic variant, a tautomer, a stereoisomer, a prodrug, a polymorph, a hydrate or a solvate thereof, or a pharmaceutically acceptable salt thereof, wherein, U₁, U₂ and U₃ are each independently selected from O, S, - NH- and -C(O)-, alternatively O.

4. The compound of formula (I) of any one of claims 1-3, or an isotopic variant, a tautomer, a stereoisomer, a prodrug, a polymorph, a hydrate or a solvate thereof, or a pharmaceutically acceptable salt thereof, wherein, W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl, alternatively selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively methyl or CD₃, alternatively C₁₋₆ alkyl, alternatively methyl.

5. The compound of formula (I) of any one of claims 1-4, or an isotopic variant, a tautomer, a stereoisomer, a prodrug, a polymorph, a hydrate or a solvate thereof, or a pharmaceutically acceptable salt thereof, wherein, L₁ is selected from chemical bond, -C(O)-, -OC(O)- and - NHC(O)-, alternatively selected from chemical bond, -C(O)- and -OC(O)-, alternatively selected from -C(O)- and -OC(O)-, alternatively -C(O)-;
alternatively, L₁ is selected from -C(O)-, -OC(O)- and -NHC(O)-; alternatively -OC(O)- and - NHC(O)-; alternatively -OC(O)-.

6. The compound of formula (I) of any one of claims 1-5, or an isotopic variant, a tautomer, a stereoisomer, a prodrug, a polymorph, a hydrate or a solvate thereof, or a pharmaceutically acceptable salt thereof, wherein, L₂ is selected from chemical bond, C₃₋₇ cycloalkylene, 3- to 7-membered heterocyclylene, phenylene and 5- to 6-membered heteroarylene, alternatively selected from chemical bond, phenylene and 5- to 6-membered heteroarylene, alternatively selected from a chemical bond and phenylene, alternatively selected from chemical bond, 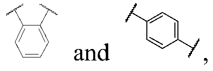 alternatively selected from a chemical bond and 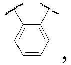 alternatively a chemical bond.

7. The compound of formula (I) of any one of claims 1-6, or an isotopic variant, a tautomer, a stereoisomer, a prodrug, a polymorph, a hydrate or a solvate thereof, or a pharmaceutically acceptable salt thereof, wherein, L₃ is selected from O, S and NR'; alternatively selected from O and NR'; alternatively O and S; alternatively is O; alternatively is NR'.

8. The compound of formula (I) of any one of claims 1-7, or an isotopic variant, a tautomer, a stereoisomer, a prodrug, a polymorph, a hydrate or a solvate thereof, or a pharmaceutically acceptable salt thereof, wherein, n is selected from 0, 1, 2 and 3, alternatively selected from 1, 2 and 3, alternatively 1 or 2, alternatively 1.

9. The compound of formula (I) of any one of claims 1-8, or an isotopic variant, a tautomer, a stereoisomer, a prodrug, a polymorph, a hydrate or a solvate thereof, or a pharmaceutically acceptable salt thereof, wherein, the methylene group in 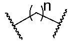 is optionally substituted with 1, 2, or 3 independent R, alternatively optionally substituted with 1 R.

10. The compound of formula (I) of any one of claims 1-9, or an isotopic variant, a tautomer, a stereoisomer, a prodrug, a polymorph, a hydrate or a solvate thereof, or a pharmaceutically acceptable salt thereof, wherein, R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ; alternatively selected from H, D, =O, C₁₋₆ alkyl, C₁₋₆ haloalkyl, -C₀₋₆ alkylene-C(O)ORₐ and -C₀₋₆ alkylene-OC(O)Rₐ; alternatively selected from H, D, =O, C₁₋₆ alkyl, C₁₋₆ haloalkyl and -C₀₋₆ alkylene-C(O)ORₐ; alternatively selected from H, D, =O, C₁₋₃ alkyl, C₁₋₃ haloalkyl and -C₀₋₆ alkylene-C(O)ORₐ; alternatively selected from H, D, =O, C₁₋₃ alkyl and -C₀₋₆ alkylene-C(O)ORₐ; alternatively selected from H, D, =O, C₁₋₃ alkyl and -C₀₋₃ alkylene-C(O)ORₐ; alternatively selected from H, D, =O and -C₀₋₃ alkylene-C(O)OH; alternatively, R is independently selected from H, =O, methyl, isobutyl, -C(O)OH and - (CH₂)₂C(O)OH; alternatively selected from H, =O, methyl, -C(O)OH and -(CH₂)₂C(O)OH; alternatively selected from H, =O, methyl and -(CH₂)₂C(O)OH; alternatively selected from H, =O and -(CH₂)₂C(O)OH;
alternatively, R is independently selected from H, D, halogen, C₁₋₆ alkyl and C₁₋₆ haloalkyl; alternatively H, D, C₁₋₆ alkyl and C₁₋₆ haloalkyl; alternatively selected from H, D, C₁₋₃ alkyl and C₁₋₃ haloalkyl; alternatively selected from H and Me.

11. The compound of formula (I) of any one of claims 1-10, or an isotopic variant, a tautomer, a stereoisomer, a prodrug, a polymorph, a hydrate or a solvate thereof, or a pharmaceutically acceptable salt thereof, wherein, R' is selected from H, C₁₋₁₀ alkyl, C₁₋₁₀ haloalkyl, C₃₋₁₀ cycloalkyl and 3- to 10-membered heterocyclyl, alternatively selected from H, C₁₋₆ alkyl and C₁₋₆ haloalkyl, alternatively H;
alternatively, R' and R are taken together with the atoms to which they are attached to form 3-to 10-membered heterocyclyl, alternatively to form 3- to 7-membered heterocyclyl,
alternatively to form 4- to 6-membered heterocyclyl, alternatively to form 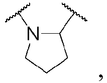
alternatively to form 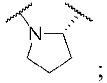
alternatively, R' and R are not taken together with the atoms to which they are attached to form a ring.

12. The compound of formula (I) of any one of claims 1-11, or an isotopic variant, a tautomer, a stereoisomer, a prodrug, a polymorph, a hydrate or a solvate thereof, or a pharmaceutically acceptable salt thereof, wherein, Rₐ, R_{b} and R_{c} are each independently selected from H, C₁₋₆ alkyl and C₁₋₆ haloalkyl, alternatively H;
alternatively, R_{b} and R_{c} are taken together with the atoms to which they are attached to form 3-to 7-membered heterocyclyl.

13. The compound of formula (I) of any one of claims 1-12, or an isotopic variant, a tautomer, a stereoisomer, a prodrug, a polymorph, a hydrate or a solvate thereof, or a pharmaceutically
acceptable salt thereof, wherein, R_{D} is 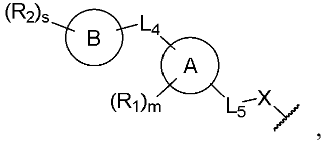 alternatively 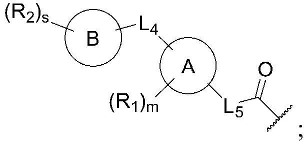
wherein,
ring A is selected from C₆₋₁₀ arylene, 5- to 10-membered heteroarylene, C₆₋₁₀ aryl fused C₅₋₁₀ cycloalkyl, C₆₋₁₀ aryl fused 5- to 10-membered heterocyclyl, 5- to 10-membered heteroaryl fused C₅₋₁₀ cycloalkyl and 5- to 10-membered heteroaryl fused 5- to 10-membered heterocyclyl; ring B is selected from C₃₋₁₀ cycloalkyl, 3- to 10-membered heterocyclyl, C₆₋₁₀ aryl and 5- to 10-membered heteroaryl, alternatively selected from C₆₋₁₀ aryl and 5- to 10-membered heteroaryl, or -L₄-ring B and R₂ are absent;
R₁ is independently selected from H, D, halogen, CN, -NO₂, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, -S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl, which is optionally substituted with 1, 2, or 3 independent R₁ₛ;
R₁ₛ is independently selected from H, D, halogen, CN, -ORₐ, -SRₐ, -NR_{b}R_{c}, -OC(O)Rₐ, - C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl and 3- to 10-membered heterocyclyl;
m=0, 1, 2, 3, 4 or 5;
R₂ is independently selected from H, D, halogen, CN, -NO₂, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, -S(O)Rₐ, -S(O)₂Rₐ, C₁₋₁₈ alkyl, C₁₋₁₈ haloalkyl, C₃₋₁₄ cycloalkyl, 3- to 14-membered heterocyclyl, C₆₋₁₄ aryl and 5- to 14-membered heteroaryl, which is optionally substituted with 1, 2, or 3 independent R₂ₛ;
R₂ₛ is independently selected from H, D, halogen, CN, -ORₐ, -SRₐ, -NR_{b}R_{c}, -OC(O)Rₐ, - C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl and 3- to 10-membered heterocyclyl;
s=0, 1, 2, 3, 4 or 5;
L₄ is selected from chemical bond, -CR₄R'₄-, -NR_{b}-, O, S, -C(O)-, -OC(O)-, -C(O)O-, - NR_{b}C(O)-, -C(O)NR_{b}-, -S(O)- and -S(O)₂-;
or, L₄ and the carbon atom on ring A to which L₄ is attached together form 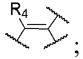 L₅ is selected from chemical bond, O, S, NR' and C₁₋₁₀ alkylene, which is optionally substituted with one or more independent R₃;
R₃ is independently selected from H, D, halogen, C₁₋₁₀ alkyl and C₁₋₁₀ haloalkyl;
R₄ and R'₄ are independently selected from H, D, halogen, C₁₋₆ alkyl and C₁₋₆ haloalkyl;
X is selected from -C(O)-, -S(O)- and -S(O)₂-, alternatively -C(O)-.

14. The compound of formula (I) of any one of claims 1-13, or an isotopic variant, a tautomer, a stereoisomer, a prodrug, a polymorph, a hydrate or a solvate thereof, or a pharmaceutically acceptable salt thereof, wherein, ring A is selected from C₆₋₁₀ arylene and 5- to 10-membered heteroarylene; alternatively selected from phenylene and 9- to 10-membered heteroarylene; alternatively selected from phenylene and indolylene; alternatively is phenylene; alternatively selected from 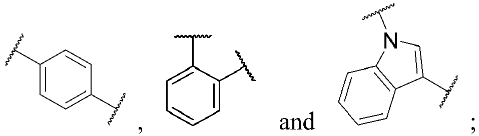 alternatively selected from 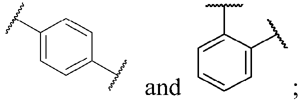 alternatively

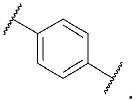

15. The compound of formula (I) of any one of claims 1-14, or an isotopic variant, a tautomer, a stereoisomer, a prodrug, a polymorph, a hydrate or a solvate thereof, or a pharmaceutically acceptable salt thereof, wherein, ring B is selected from C₃₋₇ cycloalkyl, 3- to 7-membered heterocyclyl, phenyl and 5- to 6-membered heteroaryl; alternatively selected from phenyl and 5- to 6-membered heteroaryl; alternatively is C₆₋₁₀ aryl, alternatively phenyl.

16. The compound of formula (I) of any one of claims 1-15, or an isotopic variant, a tautomer, a stereoisomer, a prodrug, a polymorph, a hydrate or a solvate thereof, or a pharmaceutically acceptable salt thereof, wherein, R₁ is independently selected from H, D, halogen, CN, -NO₂, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, -S(O)Rₐ, - S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, - SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, C₁₋₆ alkyl, C₁₋₆ haloalkyl, C₃₋₇ cycloalkyl and 3- to 7-membered heterocyclyl; alternatively selected from H, D, halogen, CN, -ORₐ, -SRₐ, - NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl; alternatively selected from H, D, halogen, CN, -ORₐ, -NR_{b}R_{c}, -OC(O)Rₐ, -C(O)ORₐ, C₁₋₆ alkyl and C₁₋₆ haloalkyl; alternatively selected from H, D, halogen, -ORₐ, -OC(O)Rₐ, C₁₋₆ alkyl and C₁₋₆ haloalkyl; alternatively selected from H, F, Me, -OMe and -OC(O)CH₃; alternatively, R₁ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl; alternatively H, D and halogen; alternatively is H or F; alternatively is halogen; alternatively is F.

17. The compound of formula (I) of any one of claims 1-16, or an isotopic variant, a tautomer, a stereoisomer, a prodrug, a polymorph, a hydrate or a solvate thereof, or a pharmaceutically acceptable salt thereof, wherein, R₂ is independently selected from H, D, halogen, CN, -NO₂, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, -S(O)Rₐ, - S(O)₂Rₐ, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, - SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, -ORₐ, -SRₐ, -NR_{b}R_{c}, -C(O)Rₐ, - OC(O)Rₐ, -C(O)ORₐ, -NR_{b}C(O)Rₐ, -C(O)NR_{b}R_{c}, C₁₋₆ alkyl, C₁₋₆ haloalkyl, C₃₋₇ cycloalkyl, 3-to 7-membered heterocyclyl, phenyl and 5- to 6-membered heteroaryl; alternatively selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, - C(O)NR_{b}R_{c}, C₁₋₆ alkyl and C₁₋₆ haloalkyl; alternatively selected from H, D, halogen, CN, C₁₋₆ alkyl and C₁₋₆ haloalkyl; alternatively selected from H, D, halogen, C₁₋₆ alkyl and C₁₋₆ haloalkyl; alternatively selected from H, Cl and Me; alternatively selected from H and Cl;
alternatively, R₂ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl, alternatively selected from H, D and halogen; yet alternatively is H or D.

18. The compound of formula (I) of any one of claims 1-17, or an isotopic variant, a tautomer, a stereoisomer, a prodrug, a polymorph, a hydrate or a solvate thereof, or a pharmaceutically acceptable salt thereof, wherein, R₁ₛ and R₂ₛ are each independently selected from H, D, halogen, CN, C₁₋₁₀ alkyl, C₁₋₁₀ haloalkyl, C₃₋₁₀ cycloalkyl and 3- to 10-membered heterocyclyl; alternatively selected from H, D, halogen, CN, C₁₋₆ alkyl, C₁₋₆ haloalkyl, C₃₋₇ cycloalkyl and 3- to 7-membered heterocyclyl; alternatively selected from H, D, halogen, C₁₋₆ alkyl and C₁₋₆ haloalkyl.

19. The compound of formula (I) of any one of claims 1-17, or an isotopic variant, a tautomer, a stereoisomer, a prodrug, a polymorph, a hydrate or a solvate thereof, or a pharmaceutically acceptable salt thereof, wherein, m=0, 1, 2, 3 or 4; alternatively m=0, 1 or 2; alternatively m=0 or 1; alternatively m=1.

20. The compound of formula (I) of any one of claims 1-19, or an isotopic variant, a tautomer, a stereoisomer, a prodrug, a polymorph, a hydrate or a solvate thereof, or a pharmaceutically acceptable salt thereof, wherein, s=0, 1 or 2; alternatively s=0.

21. The compound of formula (I) of any one of claims 1-20, or an isotopic variant, a tautomer, a stereoisomer, a prodrug, a polymorph, a hydrate or a solvate thereof, or a pharmaceutically acceptable salt thereof, wherein, L₄ is selected from chemical bond, -CR₄R'₄-, -NR_{b}-, O, S, - C(O)- and -S(O)-; alternatively selected from chemical bond, -CR₄R'₄-, -NR_{b}-, O and -C(O)-; alternatively selected from chemical bond, -NR_{b}- and -C(O)-; alternatively selected from chemical bond, -NH- and -C(O)-; alternatively selected from a chemical bond and -NH-; alternatively is a chemical bond .

22. The compound of formula (I) of any one of claims 1-21, or an isotopic variant, a tautomer, a stereoisomer, a prodrug, a polymorph, a hydrate or a solvate thereof, or a pharmaceutically acceptable salt thereof, wherein, L₅ is a chemical bond or C₁₋₁₀ alkylene, alternatively is a chemical bond or C₁₋₆ alkylene, alternatively is a chemical bond or C₁₋₄ alkylene, alternatively is a chemical bond or C₁₋₂ alkylene, alternatively is a chemical bond or methylene, alternatively -CH(CH₃)-;
alternatively, L₅ is optionally substituted with 1, 2, 3, 4, 5, or 6 independent R₃, alternatively optionally substituted with 1, 2, or 3 independent R₃, alternatively optionally substituted with 1 R₃.

23. The compound of formula (I) of any one of claims 1-21, or an isotopic variant, a tautomer, a stereoisomer, a prodrug, a polymorph, a hydrate or a solvate thereof, or a pharmaceutically acceptable salt thereof, wherein, R₃ is independently selected from H, D, halogen, C₁₋₆ alkyl and C₁₋₆ haloalkyl; alternatively selected from H, D, C₁₋₄ alkyl and C₁₋₄ haloalkyl; alternatively selected from H and Me; alternatively is Me.

24. The compound of formula (I) of any one of claims 1-23, or an isotopic variant, a tautomer, a stereoisomer, a prodrug, a polymorph, a hydrate or a solvate thereof, or a pharmaceutically acceptable salt thereof, wherein, R₄ and R'₄ are independently selected from H, D, C₁₋₆ alkyl and C₁₋₆ haloalkyl, alternatively H or D.

25. The compound of formula (I) of any one of claims 1-24, or an isotopic variant, a tautomer, a stereoisomer, a prodrug, a polymorph, a hydrate or a solvate thereof, or a pharmaceutically acceptable salt thereof, having the following structure: 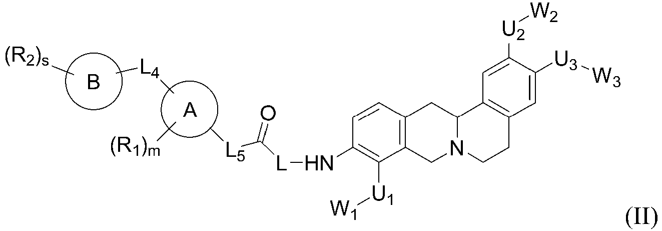 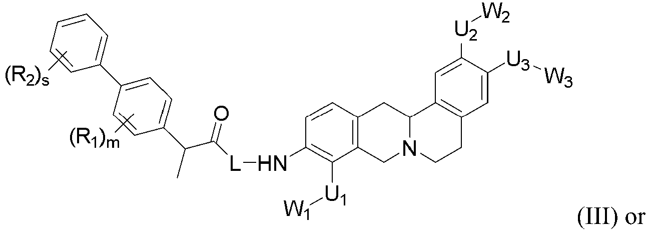 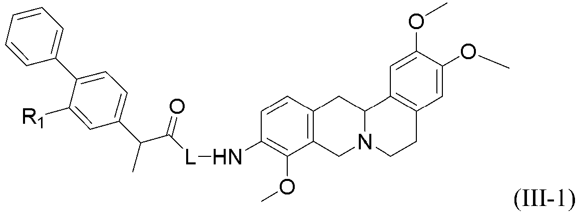 wherein, each variable is defined as in any one of claims 1-24.

26. The compound of formula (II) of claim 25, or an isotopic variant, a tautomer, a stereoisomer, a prodrug, a polymorph, a hydrate or a solvate thereof, or a pharmaceutically acceptable salt thereof, 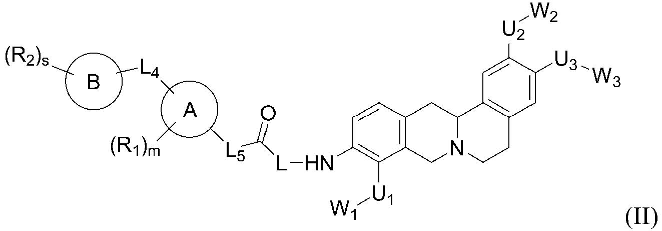 wherein,
ring A is selected from C₆₋₁₀ arylene and 5- to 10-membered heteroarylene;
ring B is selected from C₃₋₁₀ cycloalkyl, 3- to 10-membered heterocyclyl, C₆₋₁₀ aryl and 5- to 10-membered heteroaryl, alternatively selected from C₃₋₇ cycloalkyl, 3- to 7-membered heterocyclyl, phenyl and 5- to 6-membered heteroaryl, or -L₄-ring B and R₂ are absent;
R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, - C(O)NR_{b}R_{c}, C₁₋₆ alkyl, C₁₋₆ haloalkyl, C₃₋₇ cycloalkyl and 3- to 7-membered heterocyclyl, which is optionally substituted with 1, 2, or 3 independent R₁ₛ;
R₁ₛ is independently selected from H, D, halogen, CN, C₁₋₁₀ alkyl, C₁₋₁₀ haloalkyl, C₃₋₁₀ cycloalkyl and 3- to 10-membered heterocyclyl, alternatively selected from H, D, halogen, CN, C₁₋₆ alkyl, C₁₋₆ haloalkyl, C₃₋₇ cycloalkyl and 3- to 7-membered heterocyclyl;
m=0, 1, 2, 3, 4 or 5;
R₂ is selected from H, D, halogen, CN, -ORₐ, -SRₐ, -NR_{b}R_{c}, -C(O)Rₐ, -OC(O)Rₐ, -C(O)ORₐ, - NR_{b}C(O)Rₐ, -C(O)NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl, C₁₋₆ haloalkyl, C₃₋₇ cycloalkyl and 3- to 7-membered heterocyclyl, which is optionally substituted with 1, 2, or 3 independent R₂ₛ;
R₂ₛ is independently selected from H, D, halogen, CN, C₁₋₁₀ alkyl, C₁₋₁₀ haloalkyl, C₃₋₁₀ cycloalkyl and 3- to 10-membered heterocyclyl, alternatively selected from H, D, halogen, CN, C₁₋₆ alkyl, C₁₋₆ haloalkyl, C₃₋₇ cycloalkyl and 3- to 7-membered heterocyclyl;
s=0, 1, 2, 3, 4 or 5;
L₄ is selected from chemical bond, -CR₄R'₄-, -NR_{b}-, O, S, -C(O)- and -S(O)-;
L₅ is a chemical bond or C₁₋₆ alkylene, which is optionally substituted with 1, 2, 3, 4, 5, or 6 independent R₃;
R₃ is independently selected from H, D, halogen, C₁₋₆ alkyl and C₁₋₆ haloalkyl;
R₄ and R'₄ are independently selected from H, D, halogen, C₁₋₆ alkyl and C₁₋₆ haloalkyl;
U₁, U₂ and U₃ are each independently selected from O, S, -NH-, -C(O)-, -O-C(O)-, -NH-C(O)- and -O-CH₂-O-;
W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl;
L is a chemical bond or 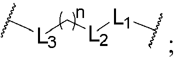
L₁ is selected from chemical bond, -C(O)-, -OC(O)- and -NHC(O)-;
L₂ is selected from chemical bond, C₃₋₇ cycloalkylene, 3- to 7-membered heterocyclylene, phenylene and 5- to 6-membered heteroarylene;
L₃ is selected from O, S and NR';
n is selected from 0, 1, 2, 3, 4, 5 and 6;
the methylene group in 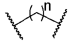 is optionally substituted with 1, 2, or 3 independent R;
R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ, alternatively selected from H, D, =O, C₁₋₆ alkyl, C₁₋₆ haloalkyl, -C₀₋₆ alkylene-C(O)ORₐ and - C₀₋₆ alkylene-OC(O)Rₐ;
R' is selected from H, C₁₋₆ alkyl, C₁₋₆ haloalkyl, C₃₋₇ cycloalkyl and 3- to 7-membered heterocyclyl, or R' and R are taken together with the atoms to which they are attached to form 3- to 7-membered heterocyclyl;
Rₐ, R_{b} and R_{c} are each independently selected from H, C₁₋₆ alkyl and C₁₋₆ haloalkyl, or R_{b} and R_{c} are taken together with the atoms to which they are attached to form 3- to 7-membered heterocyclyl;
wherein each of the above groups is optionally deuterated, up to full deuteration.

27. The compound of formula (II) of claim 26, or an isotopic variant, a tautomer, a stereoisomer, a prodrug, a polymorph, a hydrate or a solvate thereof, or a pharmaceutically acceptable salt thereof, wherein,
ring A is selected from C₆₋₁₀ arylene and 5- to 10-membered heteroarylene, alternatively selected from phenylene and 9- to 10-membered heteroarylene;
ring B is selected from C₆₋₁₀ aryl and 5- to 10-membered heteroaryl, alternatively selected from phenyl and 5- to 6-membered heteroaryl; alternatively is C₆₋₁₀ aryl, alternatively phenyl, or - L₄-ring B and R₂ are absent;
R₁ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl, alternatively selected from H, D, halogen, CN, -ORₐ, -NR_{b}R_{c}, -OC(O)Rₐ, -C(O)ORₐ, C₁₋₆ alkyl and C₁₋₆ haloalkyl,
alternatively selected from H, D, halogen, -ORₐ, -OC(O)Rₐ, C₁₋₆ alkyl and C₁₋₆ haloalkyl, alternatively H, D and halogen, which is optionally substituted with 1, 2, or 3 independent R₁ₛ; R₁ₛ is independently selected from H, D, halogen, C₁₋₆ alkyl and C₁₋₆ haloalkyl;
m=0, 1, 2, 3, 4 or 5, alternatively m=0, 1 or 2;
R₂ is independently selected from H, D, halogen, CN, -ORₐ, -SRₐ, -NR_{b}R_{c}, -C(O)Rₐ, -OC(O)Rₐ, -C(O)ORₐ, -NR_{b}C(O)Rₐ, -C(O)NR_{b}R_{c}, C₁₋₆ alkyl and C₁₋₆ haloalkyl, alternatively selected from H, D, halogen, CN, C₁₋₆ alkyl and C₁₋₆ haloalkyl, alternatively selected from H, D, halogen, C₁₋₆ alkyl and C₁₋₆ haloalkyl, alternatively H, D and halogen, which is optionally substituted with 1, 2, or 3 independent R₂ₛ;
R₂ₛ is independently selected from H, D, halogen, C₁₋₆ alkyl and C₁₋₆ haloalkyl;
s=0, 1, 2, 3, 4 or 5, alternatively s=0, 1 or 2;
L₄ is selected from chemical bond, -CR₄R'₄-, -NR_{b}-, O and -C(O)-, alternatively selected from chemical bond, -NR_{b}- and -C(O)-;
L₅ is a chemical bond or C₁₋₄ alkylene, alternatively is a chemical bond or C₁₋₂ alkylene, which is optionally substituted with 1, 2, or 3 independent R₃;
R₃ is independently selected from H, D, C₁₋₄ alkyl and C₁₋₄ haloalkyl;
R₄ and R'₄ are independently selected from H, D, C₁₋₆ alkyl and C₁₋₆ haloalkyl, alternatively selected from H and D;
U₁, U₂ and U₃ are each independently selected from O, S, -NH- and -C(O)-, alternatively O; W₁, W₂ and W₃ are each independently selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively C₁₋₆ alkyl;
L is a chemical bond or 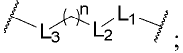
L₁ is selected from chemical bond, -C(O)- and -OC(O)-, alternatively selected from -C(O)- and -OC(O)-, alternatively -C(O)-;
L₂ is selected from chemical bond, phenylene and 5- to 6-membered heteroarylene, alternatively selected from a chemical bond and phenylene;
L₃ is selected from O and NR', alternatively NR'; alternatively is O;
n is selected from 0, 1, 2 and 3, alternatively selected from 1, 2 and 3, alternatively 1 or 2, alternatively 1;
the methylene group in 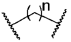 is optionally substituted with 1, 2, or 3 independent R, alternatively optionally substituted with 1 R;
R is independently selected from H, D, =O, C₁₋₆ alkyl, C₁₋₆ haloalkyl and -C₀₋₆ alkylene-C(O)ORₐ, alternatively selected from H, D, =O, C₁₋₃ alkyl, C₁₋₃ haloalkyl and -C₀₋₆ alkylene-C(O)ORₐ, alternatively selected from H, D, =O, C₁₋₃ alkyl and -C₀₋₆ alkylene-C(O)ORₐ, alternatively selected from H, D, =O, C₁₋₃ alkyl and -C₀₋₃ alkylene-C(O)ORₐ, alternatively selected from H, D, =O and -C₀₋₃ alkylene-C(O)OH;
R' is selected from H, C₁₋₆ alkyl and C₁₋₆ haloalkyl, alternatively H, or R' and R are taken together with the atoms to which they are attached to form 4- to 6-membered heterocyclyl; Alternatively, R' and R do not form a ring;
Rₐ, R_{b} and R_{c} are each independently selected from H, C₁₋₆ alkyl and C₁₋₆ haloalkyl.

28. The compound of formula (II) of claim 27, or an isotopic variant, a tautomer, a stereoisomer, a prodrug, a polymorph, a hydrate or a solvate thereof, or a pharmaceutically acceptable salt thereof, wherein,
ring A is selected from phenylene and indolylene, alternatively selected from 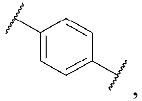 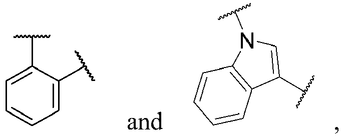 alternatively phenylene, alternatively selected from 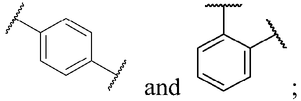
ring B is phenyl, or -L₄-ring B and R₂ are absent;
R₁ is independently selected from H, F, Me, -OMe and -OC(O)CH₃, alternatively H or F; m=0, 1 or 2;
R₂ is independently selected from H, Cl and Me, alternatively selected from H and Cl;
s=0, 1 or 2;
L₄ is selected from chemical bond, -NH- and -C(O)-; alternatively selected from a chemical bond and -NH-; alternatively is a chemical bond;
L₅ is a chemical bond or methylene, which is optionally substituted with 1 R₃; alternatively, L₅ is -CH(CH₃)-;
R₃ is independently selected from H and Me; alternatively is Me;
U₁, U₂ and U₃ is O;
W₁, W₂ and W₃ is methyl or CD₃, alternatively methyl;
L is a chemical bond or 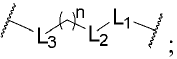
L₁ is selected from chemical bond, -C(O)- and -OC(O)-;
L₂ is selected from chemical bond, 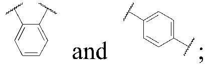
L₃ is selected from O and NR';
n is 0, 1, 2 and 3;
the methylene group in 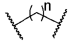 is optionally substituted with 1 R;
R is independently selected from H, =O, methyl, isobutyl, -C(O)OH and -(CH₂)₂C(O)OH, alternatively selected from H, =O, methyl, -C(O)OH and -(CH₂)₂C(O)OH, alternatively selected from H, =O, methyl and -(CH₂)₂C(O)OH, alternatively selected from H, =O and - (CH₂)₂C(O)OH;
R' is H, or R' and R are taken together with the atoms to which they are attached to form 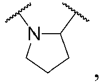 alternatively to form 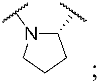
alternatively, 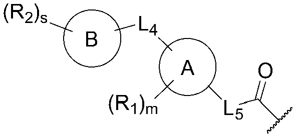 is selected from the following structures: 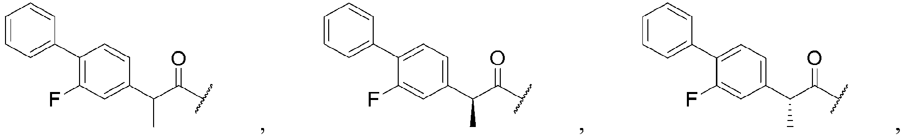 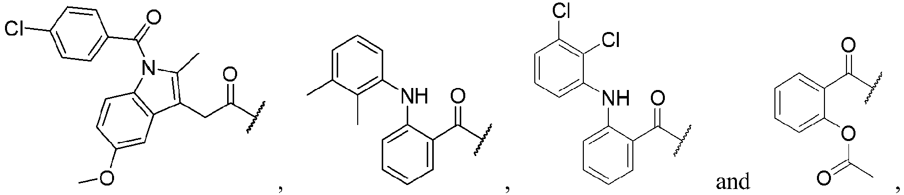
alternatively selected from 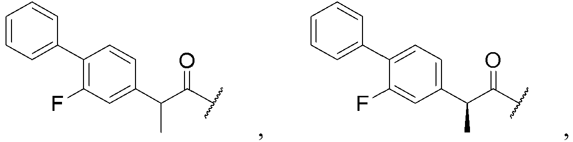 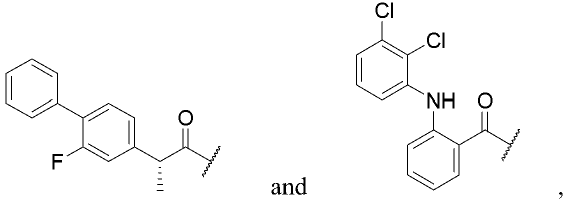 alternatively selected from

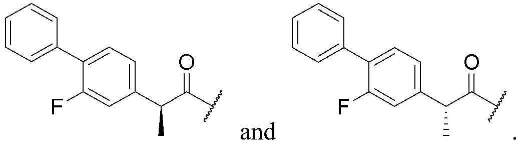

29. The compound of formula (II) of claim 25, or an isotopic variant, a tautomer, a stereoisomer, a prodrug, a polymorph, a hydrate or a solvate thereof, or a pharmaceutically acceptable salt thereof, 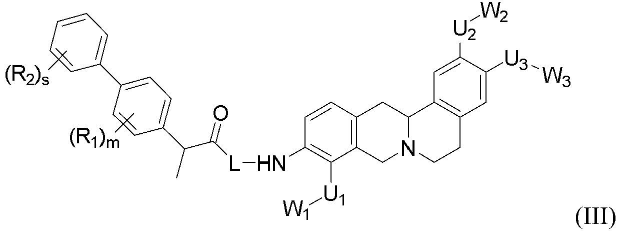 wherein,
R₁ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, which is optionally substituted with 1, 2, or 3 independent R₁ₛ;
R₁ₛ is independently selected from H, D, halogen, CN, C₁₋₁₀ alkyl, C₁₋₁₀ haloalkyl, C₃₋₁₀ cycloalkyl and 3- to 10-membered heterocyclyl;
m=0, 1, 2, 3 or 4;
R₂ is independently selected from H, D, halogen, CN, -ORₐ, -SRₐ, -NR_{b}R_{c}, C₁₋₁₀ alkyl, C₁₋₁₀ haloalkyl, C₃₋₁₀ cycloalkyl, 3- to 10-membered heterocyclyl, C₆₋₁₀ aryl and 5- to 10-membered heteroaryl, which is optionally substituted with 1, 2, or 3 independent R₂ₛ;
R₂ₛ is independently selected from H, D, halogen, CN, C₁₋₁₀ alkyl, C₁₋₁₀ haloalkyl, C₃₋₁₀ cycloalkyl and 3- to 10-membered heterocyclyl;
s=0, 1, 2, 3, 4 or 5;
U₁, U₂ and U₃ are each independently selected from O, S, -NH-, -C(O)-, -O-C(O)-, -NH-C(O)- and -O-CH₂-O-;
W₁, W₂ and W₃ are each independently selected from C₁₋₁₀ alkyl, C₁₋₁₀ deuterated alkyl, C₁₋₁₀ haloalkyl, C₃₋₁₀ cycloalkyl, 3- to 10-membered heterocyclyl, C₆₋₁₀ aryl and 5- to 10-membered heteroaryl, alternatively selected from C₁₋₁₀ alkyl, C₁₋₁₀ deuterated alkyl, C₁₋₁₀ haloalkyl, C₃₋₁₀ cycloalkyl and 3- to 10-membered heterocyclyl;
L is a chemical bond or 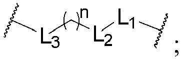
L₁ is selected from chemical bond, -C(O)-, -OC(O)- and -NHC(O)-;
L₂ is selected from chemical bond, C₃₋₇ cycloalkylene, 3- to 7-membered heterocyclylene, phenylene and 5- to 6-membered heteroarylene;
L₃ is selected from O, S and NR';
n is selected from 0, 1, 2, 3, 4, 5 and 6;
the methylene group in 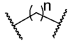 is optionally substituted with 1, 2, or 3 independent R;
R is independently selected from H, D, halogen, =O, C₁₋₆ alkyl, C₁₋₆ haloalkyl, -C₀₋₆ alkylene-C(O)ORₐ, -C₀₋₆ alkylene-OC(O)Rₐ, -C₀₋₆ alkylene-C(O)NR_{b}R_{c} and -C₀₋₆ alkylene-NR_{b}C(O)Rₐ, alternatively selected from H, D, =O, C₁₋₆ alkyl, C₁₋₆ haloalkyl, -C₀₋₆ alkylene-C(O)ORₐ and - C₀₋₆ alkylene-OC(O)Rₐ;
R' is selected from H, C₁₋₆ alkyl, C₁₋₆ haloalkyl, C₃₋₇ cycloalkyl and 3- to 7-membered heterocyclyl, or R' and R are taken together with the atoms to which they are attached to form 3- to 7-membered heterocyclyl;
Rₐ, R_{b} and R_{c} are each independently selected from H, C₁₋₆ alkyl and C₁₋₆ haloalkyl, or R_{b} and
R_{c} are taken together with the atoms to which they are attached to form 3- to 7-membered heterocyclyl;
wherein each of the above groups is optionally deuterated, up to full deuteration.

30. The compound of formula (III) of claim 29, or an isotopic variant, a tautomer, a stereoisomer, a prodrug, a polymorph, a hydrate or a solvate thereof, or a pharmaceutically acceptable salt thereof, wherein,
R₁ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl, alternatively H, D and halogen, which is optionally substituted with 1, 2, or 3 independent R₁ₛ;
R₁ₛ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl;
m=0, 1, 2, 3 or 4, alternatively m=0, 1 or 2;
R₂ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl, alternatively selected from H, D and halogen, yet alternatively is H or D, which is optionally substituted with 1, 2, or 3 independent R₂ₛ;
R₂ₛ is independently selected from H, D, halogen, CN, C₁₋₆ alkyl, C₁₋₆ haloalkyl, C₃₋₇ cycloalkyl and 3- to 7-membered heterocyclyl, alternatively selected from H, D, halogen, C₁₋₆ alkyl and C₁₋₆ haloalkyl;
s=0, 1, 2, 3, 4 or 5, alternatively s=0, 1 or 2;
U₁, U₂ and U₃ are each independently selected from O, S, -NH- and -C(O)-, alternatively O; W₁, W₂ and W₃ are each independently selected from C₁₋₁₀ alkyl, C₁₋₁₀ deuterated alkyl and C₁₋₁₀ haloalkyl, alternatively selected from C₁₋₆ alkyl, C₁₋₆ deuterated alkyl and C₁₋₆ haloalkyl, alternatively C₁₋₆ alkyl or C₁₋₆ deuterated alkyl, alternatively C₁₋₆ alkyl;
L is a chemical bond or 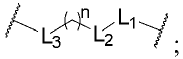
L₁ is selected from chemical bond, -C(O)- and -OC(O)-, alternatively selected from -C(O)- and -OC(O)-, alternatively -C(O)-;
L₂ is selected from chemical bond, phenylene and 5- to 6-membered heteroarylene, alternatively selected from a chemical bond and phenylene;
L₃ is selected from O and NR', alternatively NR'; alternatively is O;
n is selected from 0, 1, 2 and 3, alternatively selected from 1, 2 and 3, alternatively 1 or 2, alternatively 1;
the methylene group in 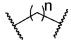 is optionally substituted with 1, 2, or 3 independent R, alternatively optionally substituted with 1 R;
R is independently selected from H, D, =O, C₁₋₆ alkyl, C₁₋₆ haloalkyl and -C₀₋₆ alkylene-C(O)ORₐ, alternatively selected from H, D, =O, C₁₋₃ alkyl, C₁₋₃ haloalkyl and -C₀₋₆ alkylene-C(O)ORₐ, alternatively selected from H, D, =O, C₁₋₃ alkyl and -C₀₋₆ alkylene-C(O)ORₐ, alternatively selected from H, D, =O, C₁₋₃ alkyl and -C₀₋₃ alkylene-C(O)ORₐ, alternatively selected from H, D, =O and -C₀₋₃ alkylene-C(O)OH;
R' is selected from H, C₁₋₆ alkyl and C₁₋₆ haloalkyl, alternatively H, or R' and R are taken together with the atoms to which they are attached to form 4- to 6-membered heterocyclyl; Alternatively, R' and R do not form a ring;
Rₐ is independently selected from H, C₁₋₆ alkyl and C₁₋₆ haloalkyl.

31. The compound of formula (III) of claim 30, or an isotopic variant, a tautomer, a stereoisomer, a prodrug, a polymorph, a hydrate or a solvate thereof, or a pharmaceutically acceptable salt thereof, wherein,
R₁ is H or F; m=1;
R₂ is H; s=0;
U₁, U₂ and U₃ is O;
W₁, W₂ and W₃ is methyl or CD₃, alternatively methyl;
L is a chemical bond or 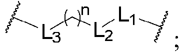
L₁ is selected from chemical bond, -C(O)- and -OC(O)-;
L₂ is selected from chemical bond, 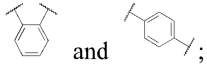
L₃ is selected from O and NR';
n is 0, 1, 2 and 3;
the methylene group in 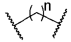 is optionally substituted with 1 R;
R is independently selected from H, =O, methyl, isobutyl, -C(O)OH and -(CH₂)₂C(O)OH, alternatively selected from H, =O, methyl, -C(O)OH and -(CH₂)₂C(O)OH, alternatively selected from H, =O, methyl and -(CH₂)₂C(O)OH, alternatively selected from H, =O and - (CH₂)₂C(O)OH;
R' is H, or R' and R are taken together with the atoms to which they are attached to form 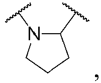 alternatively to form 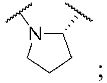
alternatively, L is selected from a chemical bond, 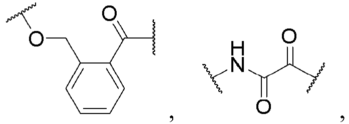 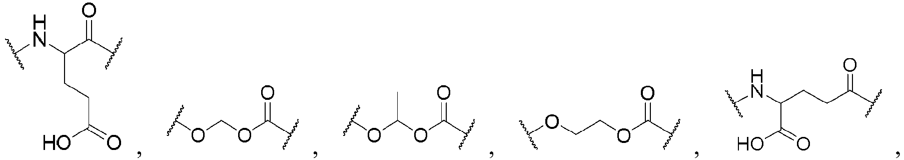 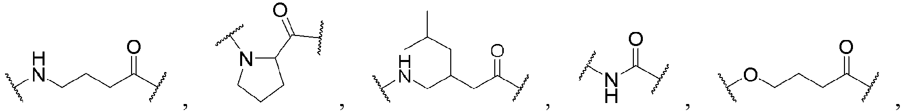 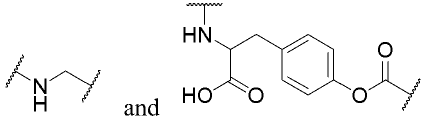 ; alternatively selected from: a chemical bond, 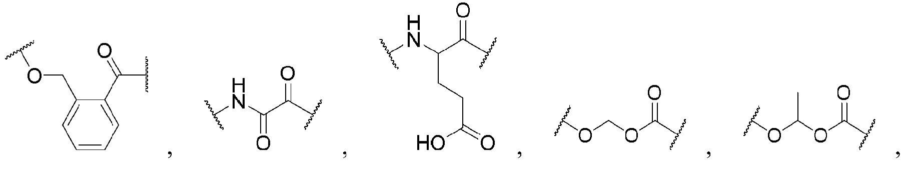 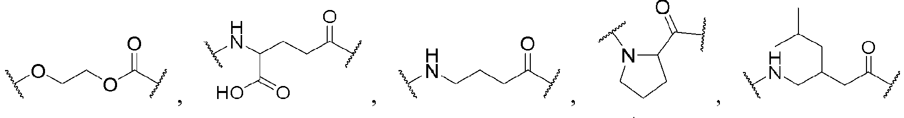 and 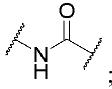 alternatively selected from: a chemical bond, 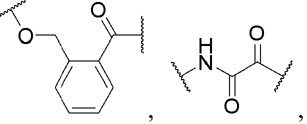 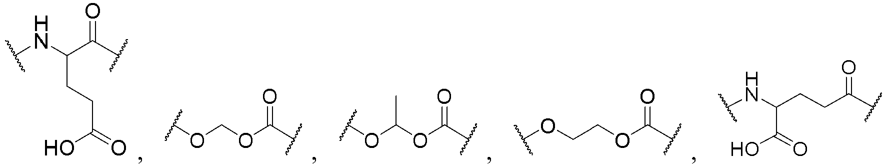 and 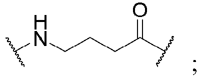 alternatively selected from: a chemical bond, 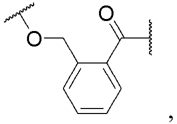 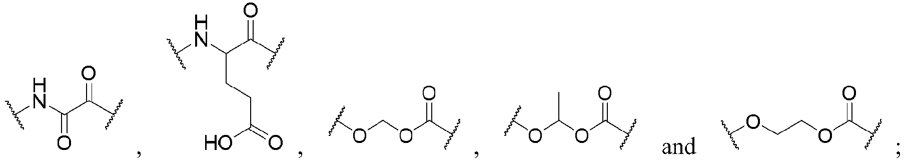
alternatively selected from: a chemical bond, 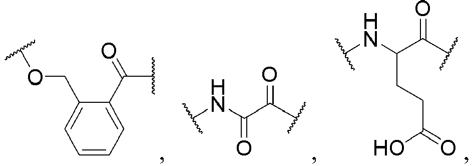 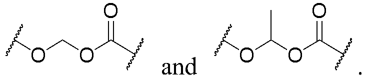
alternatively, 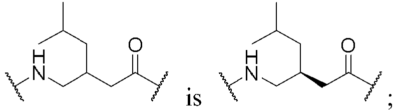
alternatively, 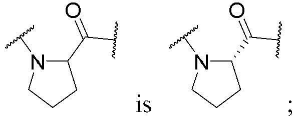
alternatively, 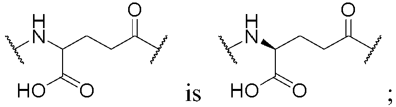
alternatively, 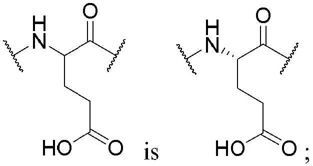
alternatively,

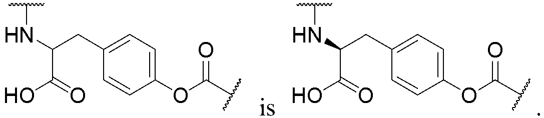

32. The compound of formula (III-1) of claim 25, or an isotopic variant, a tautomer, a stereoisomer, a prodrug, a polymorph, a hydrate or a solvate thereof, or a pharmaceutically acceptable salt thereof, 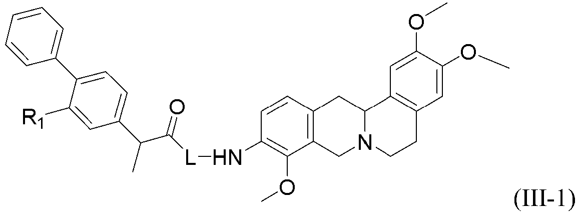 wherein, each variable is defined as in any one of claims 1-31;
alternatively, 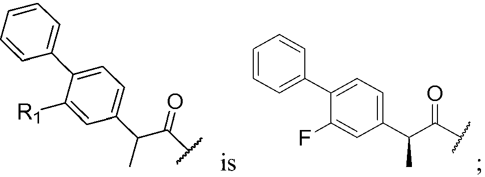
alternatively,

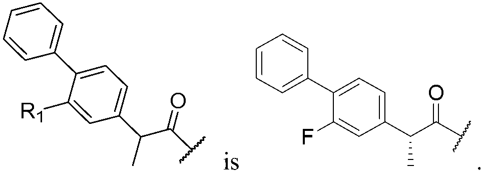

33. The compound of formula (II), formula (III), or formula (III-1) of claim 25, or an isotopic variant, a tautomer, a stereoisomer, a prodrug, a polymorph, a hydrate or a solvate thereof, or a pharmaceutically acceptable salt thereof, wherein,
L is 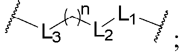
L₁ is selected from -C(O)-, -OC(O)- and -NHC(O)-; alternatively -OC(O)- and -NHC(O)-; alternatively -OC(O)-;
L₂ is a chemical bond;
L₃ is selected from O, S and NR'; alternatively O and S; alternatively is O;
n is selected from 1, 2 and 3, alternatively 1 or 2, alternatively 1;
the methylene group in 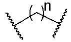 is optionally substituted with 1, 2 or 3 (alternatively 1) independent R;
R is independently selected from H, D, halogen, C₁₋₆ alkyl and C₁₋₆ haloalkyl; alternatively H, D, C₁₋₆ alkyl and C₁₋₆ haloalkyl; alternatively selected from H, D, C₁₋₃ alkyl and C₁₋₃ haloalkyl; alternatively selected from H and Me;
the remaining groups are defined as defined in any one of claims 1-32;
alternatively, L is

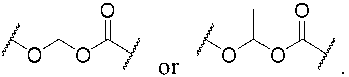

34. The compound of formula (I) of claim 1, or an isotopic variant, a tautomer, a stereoisomer, a prodrug, a polymorph, a hydrate or a solvate thereof, or a pharmaceutically acceptable salt thereof, wherein, the compound is selected from the following:

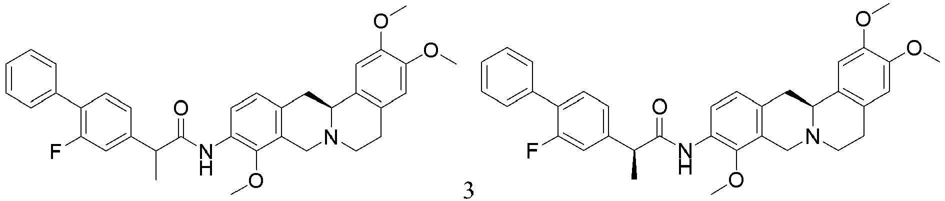

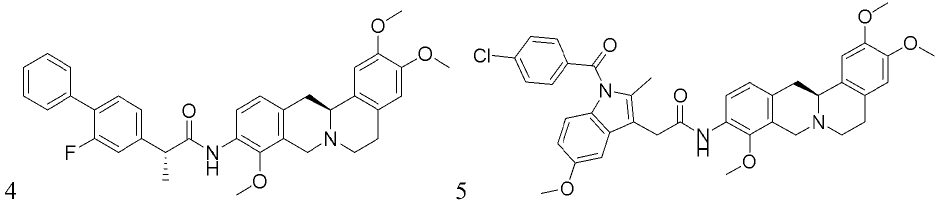

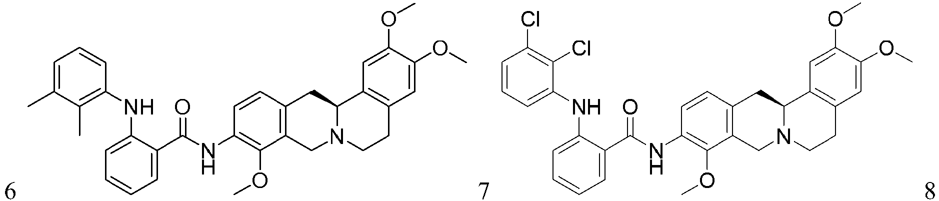

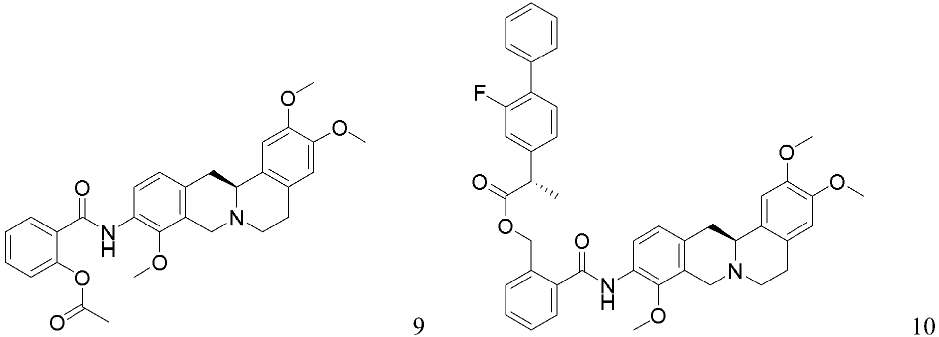

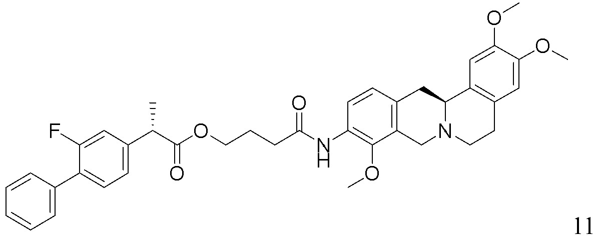

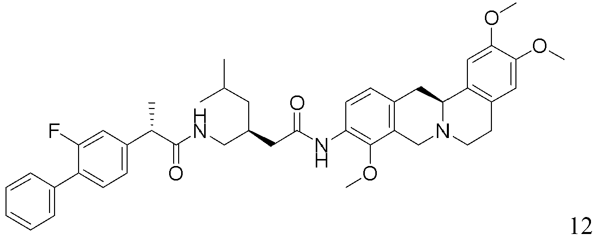

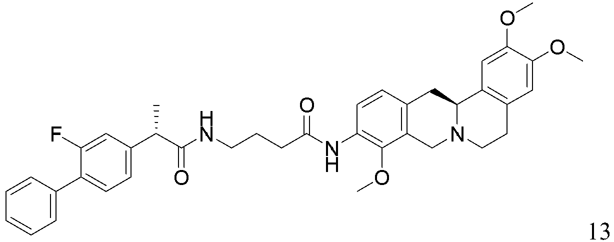

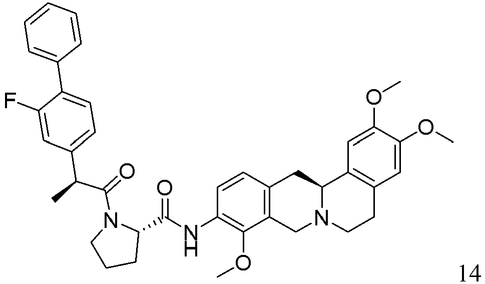

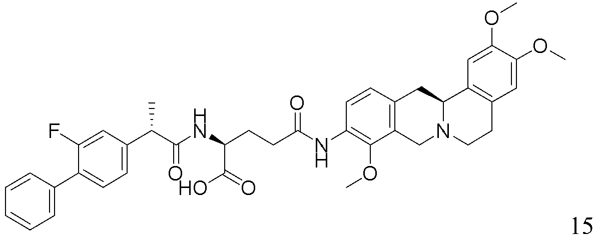

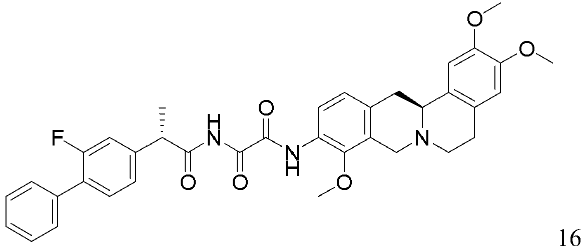

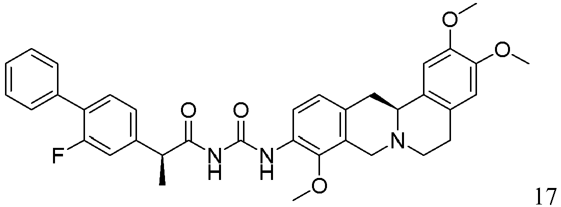

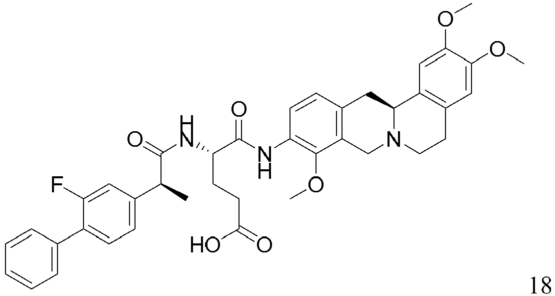

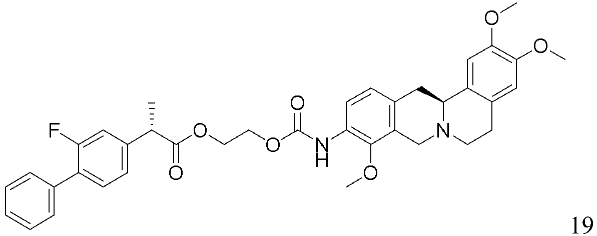

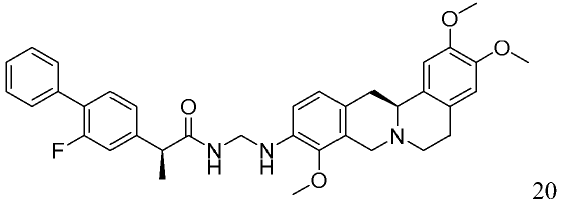

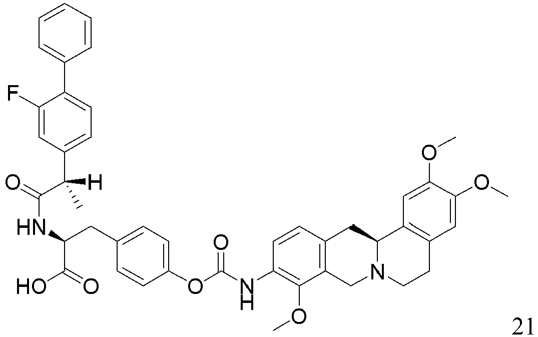

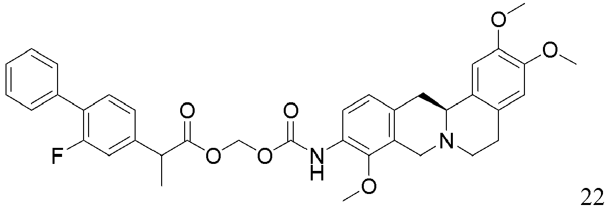

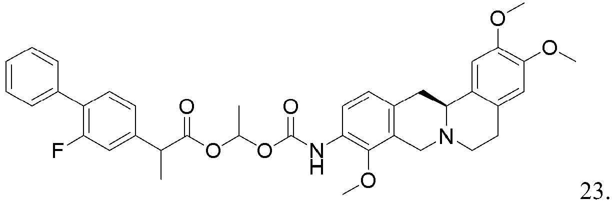

35. A pharmaceutical composition, comprising a compound of any one of claims 1-34, or an isotopic variant, a tautomer, a stereoisomer, a prodrug, a polymorph, a hydrate or a solvate thereof, or a pharmaceutically acceptable salt thereof, and a pharmaceutically acceptable carrier, adjuvant or vehicle, optionally other therapeutic agent.

36. Use of the compound of any one of claims 1-34, or an isotopic variant, a tautomer, a stereoisomer, a prodrug, a polymorph, a hydrate or a solvate thereof, or a pharmaceutically acceptable salt thereof, or the pharmaceutical composition of claim 35, in the manufacture of a medicament for the treatment or prevention of central nervous system related disease.

37. A method of treating or preventing a central nervous system related disease in a subject, comprising administering to the subject a compound of any one of claims 1-34, or an isotopic variant, a tautomer, a stereoisomer, a prodrug, a polymorph, a hydrate or a solvate thereof, or a pharmaceutically acceptable salt thereof, or the pharmaceutical composition of claim 35.

38. The compound of any one of claims 1-34, or an isotopic variant, a tautomer, a stereoisomer, a prodrug, a polymorph, a hydrate or a solvate thereof, or a pharmaceutically acceptable salt thereof, or the pharmaceutical composition of claim 35, for use in the treatment or prevention of a central nervous system related disease.

39. The use of claim 36, the method of claim 37, or the compound or pharmaceutical composition of claim 38, wherein, the disease is selected from pain, depression, and addiction.

40. The use, method, compound, or pharmaceutical composition of claim 39, wherein, the pain is selected from the group consisting of neuralgia, perioperative pain (including preoperative, intraoperative or post-operative pain, such as somatic or visceral pain caused by postoperative trauma or surgical incisions, pain caused by visceral injury, and other comprehensive types of pain), and cancer pain (such as pain caused by cancer).

41. The use, method, compound, or pharmaceutical composition of claim 40, wherein, the pain is acute pain or chronic pain (including acute/chronic pain before, during, or after surgery, acute neuralgia, or chronic neuralgia).

42. The use, method, compound, or pharmaceutical composition of claim 40, wherein, the neuralgia is central pain, such as spinal pain, thalamic pain, pontine pain, medullary pain, or cerebral cortical pain.

43. The use, method, compound, or pharmaceutical composition of claim 40, wherein, the postoperative pain is pain caused by surgery, such as pain resulting from abdominal surgery, orthopedic surgery, cesarean section, or brain surgery.

44. The use of claim 36, the method of claim 37, or the compound or pharmaceutical composition of claim 38, wherein, the compound or pharmaceutical composition is used for sedation or sleep aid.
